(19) Europäisches Patentamt
European Patent Office
Office européen des brevets

(11) EP 4 327 867 A2

(12) EUROPEAN PATENT APPLICATION

(43) Date of publication:
**28.02.2024 Bulletin 2024/09**

(21) Application number: **23215194.4**

(22) Date of filing: **11.02.2010**

(51) International Patent Classification (IPC):
***A61P 3/08*** *(2006.01)*

(52) Cooperative Patent Classification (CPC):
**A61K 31/431; A61K 9/0019; A61K 9/1694; A61K 9/2018; A61K 9/2095; A61K 9/4858; A61K 31/7004; A61P 3/00; A61P 3/04; A61P 3/06; A61P 3/08; A61P 3/10**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**BA RS**

(30) Priority: **13.02.2009 US 152317 P**
**22.10.2009 US 254033 P**

(62) Document number(s) of the earlier application(s) in accordance with Art. 76 EPC:
**10703652.7 / 2 395 968**

(27) Previously filed application:
**11.02.2010 EP 10703652**

(71) Applicant: **Boehringer Ingelheim International GmbH**
**55216 Ingelheim am Rhein (DE)**

(72) Inventors:
- **EISENREICH, Wolfram**
**55216 Ingelheim am Rhein (DE)**
- **LADYZHYNSKY, Nadia S.**
**Ridgefield, CT 06877-0368 (US)**
- **LI, Danping**
**Ridgefield, CT 06877-0368 (US)**
- **SCHULTZ, Leon**
**Ridgefield, CT 06877-0368 (US)**
- **WANG, Zeren**
**Ridgefield, CT 06877-0368 (US)**
- **MACHA, Sreeraj**
**Ridgefield, CT 06877-0368 (US)**
- **BARTA, Albert**
**55216 Ingelheim am Rhein (DE)**

(74) Representative: **Lutze, Oliver et al**
**Boehringer Ingelheim International GmbH**
**Binger Straße 173**
**55216 Ingelheim am Rhein (DE)**

Remarks:
This application was filed on 08.12.2023 as a divisional application to the application mentioned under INID code 62.

# (54) PHARMACEUTICAL COMPOSITION COMPRISING GLUCOPYRANOSYL DIPHENYLMETHANE DERIVATIVES, PHARMACEUTICAL DOSAGE FORM THEREOF, PROCESS FOR THEIR PREPARATION AND USES THEREOF FOR IMPROVED GLYCEMIC CONTROL IN A PATIENT

(57) The present invention relates to pharmaceutical compositions comprising a SGLT-2 inhibitor, pharmaceutical dosage forms, their preparation, their use and methods for treating metabolic disorders.

EP 4 327 867 A2

## Description

### Technical Field of the Invention

The present invention relates to pharmaceutical compositions comprising a SGLT-2 inhibitor as active pharmaceutical ingredient. Furthermore the present invention relates to a pharmaceutical dosage form comprising such a pharmaceutical composition. In addition the invention relates to a process for the preparation of such a pharmaceutical dosage form. In addition the invention relates to the use of the pharmaceutical composition and of the pharmaceutical dosage form in the treatment and/or prevention of selected diseases and medical conditions, in particular of one or more conditions selected from type 1 diabetes mellitus, type 2 diabetes mellitus, impaired glucose tolerance, impaired fasting blood glucose and hyperglycemia inter alia. Furthermore the present invention relates to methods of treating and/or preventing of such diseases and medical conditions wherein a pharmaceutical composition or pharmaceutical dosage form according to the invention is administered to a patient in need thereof.

### Background of the Invention

Type 2 diabetes is an increasingly prevalent disease that due to a high frequency of complications leads to a significant reduction of life expectancy. Because of diabetes-associated microvascular complications, type 2 diabetes is currently the most frequent cause of adult-onset loss of vision, renal failure, and amputations in the industrialized world. In addition, the presence of type 2 diabetes is associated with a two to five fold increase in cardiovascular disease risk.

After long duration of disease, most patients with type 2 diabetes will eventually fail on oral therapy and become insulin dependent with the necessity for daily injections and multiple daily glucose measurements.

The UKPDS (United Kingdom Prospective Diabetes Study) demonstrated that intensive treatment with metformin, sulfonylureas or insulin resulted in only a limited improvement of glycemic control (difference in HbA1c -0.9%). In addition, even in patients within the intensive treatment arm glycemic control deteriorated significantly over time and this was attributed to deterioration of β-cell function. Importantly, intensive treatment was not associated with a significant reduction in macrovascular complications, i.e. cardiovascular events. Therefore many patients with type 2 diabetes remain inadequately treated, partly because of limitations in long term efficacy, tolerability and dosing inconvenience of existing antihyperglycemic therapies.

Oral antidiabetic drugs conventionally used in therapy (such as e.g. first- or second-line, and/or mono- or (initial or add-on) combination therapy) include, without being restricted thereto, metformin, sulphonylureas, thiazolidinediones, glinides and α-glucosidase inhibitors.

The high incidence of therapeutic failure is a major contributor to the high rate of long-term hyperglycemia-associated complications or chronic damages (including micro- and macrovascular complications such as e.g. diabetic nephrophathy, retinopathy or neuropathy, or cardiovascular complications) in patients with type 2 diabetes.

Therefore, there is an unmet medical need for methods, medicaments and pharmaceutical compositions with a good efficacy with regard to glycemic control, with regard to disease-modifying properties and with regard to reduction of cardiovascular morbidity and mortality while at the same time showing an improved safety profile.

SGLT2 inhibitors inhibitors represent a novel class of agents that are being developed for the treatment or improvement in glycemic control in patients with type 2 diabetes. Glucopyranosyl-substituted benzene derivative are described in the prior art as SGLT2 inhibitors, for example in WO 01/27128, WO 03/099836, WO 2005/092877, WO 2006/034489, WO 2006/064033, WO 2006/117359, WO 2006/117360, WO 2007/025943, WO 2007/028814, WO 2007/031548, WO 2007/093610, WO 2007/128749, WO 2008/049923, WO 2008/055870, WO 2008/055940. The glucopyranosyl-substituted benzene derivatives are proposed as inducers of urinary sugar excretion and as medicaments in the treatment of diabetes.

Renal filtration and reuptake of glucose contributes, among other mechanisms, to the steady state plasma glucose concentration and can therefore serve as an antidiabetic target. Reuptake of filtered glucose across epithelial cells of the kidney proceeds via sodium-dependent glucose cotransporters (SGLTs) located in the brush-border membranes in the tubuli along the sodium gradient. There are at least 3 SGLT isoforms that differ in their expression pattern as well as in their physico-chemical properties. SGLT2 is exclusively expressed in the kidney, whereas SGLT1 is expressed additionally in other tissues like intestine, colon, skeletal and cardiac muscle. SGLT3 has been found to be a glucose sensor in interstitial cells of the intestine without any transport function. Potentially, other related, but not yet characterized genes, may contribute further to renal glucose reuptake. Under normoglycemia, glucose is completely reabsorbed by SGLTs in the kidney, whereas the reuptake capacity of the kidney is saturated at glucose concentrations higher than 10mM, resulting in glucosuria ("diabetes mellitus"). This threshold concentration can be decreased by SGLT2-inhibition. It has been shown in experiments with the SGLT inhibitor phlorizin that SGLT-inhibition will partially inhibit the reuptake of glucose from the glomerular filtrate into the blood leading to a decrease in blood glucose concentration and

to glucosuria.

## Aim of the present invention

The aim of the present invention is to provide a pharmaceutical composition comprising a SGLT-2 inhibitor which avoids or reduces sticking during the production process of the composition.

Another aim of the present invention is to provide a pharmaceutical composition comprising a SGLT-2 inhibitor which avoids or reduce filming during the production process of the composition.

Another aim of the invention is to provide a pharmaceutical dosage form comprising a SGLT-2 inhibitor which has a short disintegration time, which has good dissolution properties and/or which enables a high bioavailability of the SGLT-2 inhibitor in a patient.

Another aim of the invention is to provide a pharmaceutical composition comprising a SGLT2 inhibitor which has high content uniformity and/or which allows an effective production with regard to time and costs of pharmaceutical dosage forms.

Another aim of the invention it to provide a pharmaceutical composition and a pharmaceutical dosage form, each comprising a SGLT2 inhibitor, and a method for preventing, slowing progression of, delaying or treating a metabolic disorder, in particular of type 2 diabetes mellitus.

A further aim of the present invention is to provide a pharmaceutical composition and a pharmaceutical dosage form, each comprising a SGLT2 inhibitor, and a method for improving glycemic control in a patient in need thereof, in particular in patients with type 2 diabetes mellitus.

Another aim of the present invention is to provide a pharmaceutical composition and a pharmaceutical dosage form, each comprising a SGLT2 inhibitor, and a method for improving glycemic control in a patient with insufficient glycemic control.

Another aim of the present invention is to provide a pharmaceutical composition and a pharmaceutical dosage form, each comprising a SGLT2 inhibitor, and a method for preventing, slowing or delaying progression from impaired glucose tolerance (IGT), impaired fasting blood glucose (IFG), insulin resistance and/or metabolic syndrome to type 2 diabetes mellitus.

Yet another aim of the present invention is to provide a pharmaceutical composition and a pharmaceutical dosage form, each comprising a SGLT2 inhibitor, and a method for preventing, slowing progression of, delaying or treating of a condition or disorder from the group consisting of complications of diabetes mellitus.

A further aim of the present invention is to provide a pharmaceutical composition and a pharmaceutical dosage form, each comprising a SGLT2 inhibitor, and a method for reducing the weight or preventing an increase of the weight in a patient in need thereof.

Another aim of the present invention is to provide a pharmaceutical composition and a pharmaceutical dosage form, each comprising a SGLT2 inhibitor, with a high efficacy for the treatment of metabolic disorders, in particular of diabetes mellitus, impaired glucose tolerance (IGT), impaired fasting blood glucose (IFG), and/or hyperglycemia, which has good to very good pharmacological and/or pharmacokinetic and/or physicochemical properties.

Another aim of the present invention is to provide a process for the preparation of a pharmaceutical dosage form according to the invention which is effective in costs and/or time.

Further aims of the present invention become apparent to the one skilled in the art by description hereinbefore and in the following and by the examples.

## Summary of the Invention

In one aspect the present invention provides a pharmaceutical composition comprising a SGLT-2 inhibitor as a active pharmaceutical ingredient and one or more excipients, in particular one or more diluents, and/or one or more disintegrants. In a further aspect, the pharmaceutical composition further comprises one or more binders. In one aspect, a pharmaceutical compositions according to the invention is a solid pharmaceutical composition, for example a solid pharmaceutical composition for oral administration.

In one embodiment, the active ingredient represents 25% or less of the weight of the pharmaceutical composition. Preferably, the active ingredient represents 0.5% to 25% of the weight of the pharmaceutical composition. More preferably, the active ingredient represents 1.0% to 20% of the weight of the pharmaceutical composition. Even more preferably, the active ingredient represents 2.0% to 15% of the weight of the pharmaceutical composition.

Within the scope of the present invention it has been found that a pharmaceutical composition comprising a SGLT-2 inhibitor as an active pharmaceutical ingredient with a particle size distribution of $X90 < 200\ \mu m$, in particular with a particle size distribution of $1\ \mu m < X90 < 200\ \mu m$, shows an advantageous dissolution profile and/or good bioavailability and allows a high content uniformity and an effective production with regard to time and costs of pharmaceutical dosage forms.

Therefore in another aspect the present invention provides a pharmaceutical composition comprising a SGLT-2 inhibitor as an active pharmaceutical ingredient and one or more excipients, wherein the active ingredient has a particle size distribution of X90 < 200 μm, in particular a particle size distribution of 1μm < X90 < 200 μm, preferably determined by volume by laser-diffraction method.

In one embodiment, the ratio of disintegrant(s) to binder(s) in a composition of the present invention is between 1.5:3.5 and 1:1. In one embodiment, a disintegrant in the pharmaceutical composition is croscarmellose sodium In one embodiment, a binder in the pharmaceutical composition is hydroxypropyl cellulose. In one embodiment, a diluent in the pharmaceutical composition is lactose monohydrate or microcrystalline cellulose. In one embodiment, the pharmaceutical composition comprises lactose monohydrate and microcrystalline cellulose. In one embodiment, the pharmaceutical composition further comprises a glidant, for example colloidal silicon dioxide or talc. In one embodiment, the pharmaceutical composition further comprises a lubricant.

In one embodiment, a binder in a composition according to the present invention is a binder with fine particle size. In one embodiment, at least 99% of the particles of the binder (by weight) are 250μm or smaller. In one embodiment, at least 99.5% of the particles of the binder (by weight) are 250μm or smaller. In one embodiment, 99.9% of the particles of the binder (by weight) pass through a sieve with the screen size of 60 mesh, i.e. are 250 μm or smaller.

In another embodiment, the present invention provides a dosage form, for example a tablet, comprising a pharmaceutical composition according to the present invention.

In one embodiment, the dosage form, for example tablet, comprises:

| | Amount (% by weight) |
|---|---|
| Active ingredient | 0.5 - 25 |
| One or more Diluent(s) | 65 - 90 |
| One or more Binders | 1 5 |
| One or more Disintegrants | 1 - 3 |
| Optionally Additional additives | ad 100 % |

In another embodiment, the dosage form, for example tablet, comprises:

| | Amount (% by weight) |
|---|---|
| Active ingredient | 0.5 - 25 |
| One or more Diluent(s) | 65 - 93 |
| One or more Binders | 1 - 5 |
| One or more Disintegrants | 1 - 4 |
| Optionally Additional additives | ad 100 % |

In one embodiment, the dosage form, for example tablet, comprises per mg of dosage form:

| | Amount (% by weight) |
|---|---|
| Active ingredient | 0.5 - 25 |
| Lactose monohydrate | 35 - 90 |
| Microcrystalline cellulose | 0 - 30 |
| Hydroxypropyl cellulose | 1 - 5 |
| Croscarmellose sodium | 1 - 3 |
| Optionally Additional additives | ad 100 % |

In another embodiment, the dosage form, for example tablet, comprises per mg of dosage form:

| | Amount (% by weight) |
|---|---|
| Active ingredient | 0.5 - 25 |
| Lactose monohydrate | 28 - 70 |
| Microcrystalline cellulose | 20 - 50 |
| Hydroxypropyl cellulose | 1 - 5 |
| Croscarmellose sodium | 1 - 4 |
| Optionally Additional additives | ad 100 % |

In one embodiment, the dosage form, for example tablet, further comprises a lubricant, for example magnesium stearate, for example in a concentration of 0.25 - 2 %.

In one embodiment, the dosage form, for example tablet, further comprises a glidant, for example colloidal silicon dioxide, for example in a concentration of 0.25 - 2 %.

A dosage form, for example tablet, according to the invention may be film-coated. Typically a film coat represents 2-5% by weight of the total composition and comprises preferably a film-forming agent, a plasticizer, a glidant and optionally one or more pigments. An exemplary coat composition may comprise hydroxypropyl methylcellulose (HPMC), polyethylene glycol (PEG), talc, titanium dioxide and optionally iron oxide, including iron oxide red and/or yellow.

In another aspect, the present invention provides a wet granulation process for making a pharmaceutical composition, wherein said process comprises the steps of:

(1) Premixing the active ingredient and the main portion of the excipients including the binder in a mixer to obtain a pre-mixture;
(2) granulating the pre-mixture of step (1) by adding the granulation liquid, preferably purified water;
(3) drying the granules of step (2) in a fluidized bed dryer or a drying oven;
(4) optionally dry sieving of the dried granules of step (3);
(5) mixing the dried granules of step (4) with the remaining excipients like glidant and lubricant in a mixer to obtain the final mixture;
(6) tableting the final mixture of step (5) by compressing it on a suitable tablet press to produce tablets cores;
(7) optionally film-coating of the tablet cores of step (6) with a non-functional coat.

In another aspect, the present invention provides a pharmaceutical composition obtainable by the above process.

In another aspect, the present invention provides a direct compression process for making a pharmaceutical composition, wherein said process comprises the steps of:

(1) Premixing the active ingredient and the main portion of the excipients in a mixer to obtain a pre-mixture;
(2) optionally dry screening the pre-mixture through a screen in order to segregate cohesive particles and to improve content uniformity;
(3) mixing the pre-mixture of step (1) or (2) in a mixer, optionally by adding remaining excipients to the mixture and continuing mixing;
(4) tableting the final mixture of step (3) by compressing it on a suitable tablet press to produce the tablet cores;
(5) optionally film-coating of the tablet cores of step (4) with a non-functional coat.

In another aspect, the present invention provides a pharmaceutical composition obtainable by the above process.

In another aspect, the present invention provides a dry granulation process for making a pharmaceutical composition, wherein said process comprises the steps of:

(1) mixing the active ingredient with either all or a portion of the excipients in a mixer;
(2) compaction of the mixture of step (1) on a suitable roller compactor;
(3) reducing the ribbons obtained during step (2) to granules, preferably small granules, by suitable milling or sieving steps;
(4) optionally mixing the granules of step (3) with the remaining excipients in a mixer to obtain the final mixture;
(5) tabletting the granules of step (3) or the final mixture of step (4) by compressing it on a suitable tablet press to produce the tablet cores;

(6) optionally film-coating of the tablet cores of step (5) with a non-functional coat.

In another aspect, the present invention provides a pharmaceutical composition obtainable by the above process.

In one embodiment, a pharmaceutical composition according to the present invention is obtained by high shear wet granulation.

Preferably the SGLT2 inhibitor is selected from a glucopyranosyl-substituted benzene derivative of the formula (I)

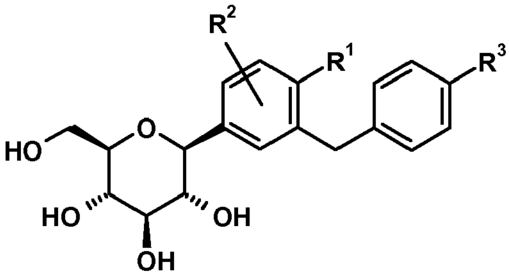

I

wherein $R^1$ denotes Cl, methyl or cyano; $R^2$ denotes H, methyl, methoxy or hydroxy and $R^3$ denotes (*R*)-tetrahydrofuran-3-yloxy or (*S*)-tetrahydrofuran-3-yloxy; or a prodrug of one of the beforementioned SGLT2 inhibitors.

In the above glucopyranosyl-substituted benzene derivatives of the formula (I) the following definitions of the substituents are preferred.

Preferably $R^1$ denotes chloro or cyano; in particular chloro.
Preferably $R^2$ denotes H.
Preferably $R^3$ denotes (*R*)-tetrahydrofuran-3-yloxy or (*S*)-tetrahydrofuran-3-yloxy.

Preferred glucopyranosyl-substituted benzene derivatives of the formula (I) are selected from the group of compounds (I.8) to (I.11):

| | |
|---|---|
| (I.8) | 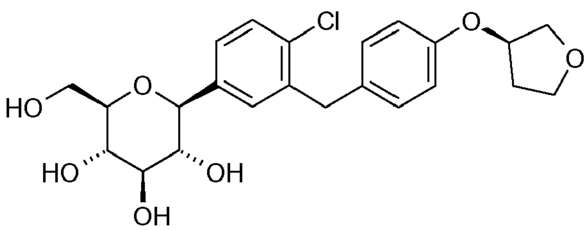 1-chloro-4-(β-D-glucopyranos-1-yl)-2-[4-(*(R)*-tetrahydrofuran-3-yloxy)-benzyl]-benzene, |
| (I.9) | 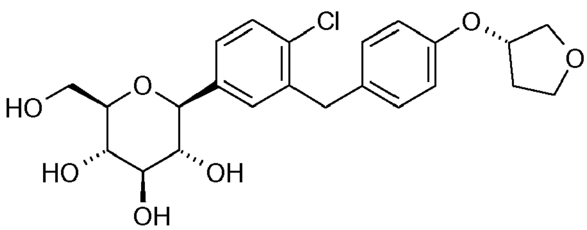 1-chloro-4-(β-D-glucopyranos-1-yl)-2-[4-(*(S)*-tetrahydrofuran-3-yloxy)-benzyl]-benzene, |
| (I. 10) | 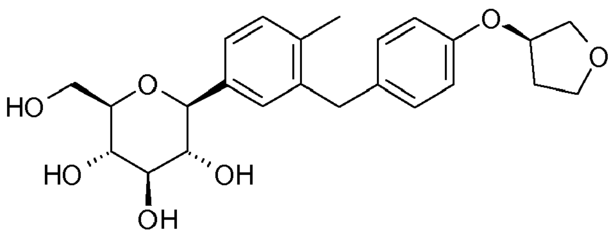 1-methyl-2-[4-(*(R)*-tetrahydrofuran-3-yloxy)-benzyl]-4-(β-D-glucopyranos-1-yl)-benzene, |

(continued)

| | |
|---|---|
| (I. 11) | 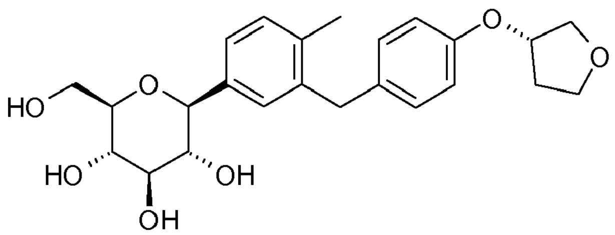 1-methyl-2-[4-((*S*)-tetrahydrofuran-3-yloxy)-benzyl]-4-(β-D-glucopyranos-1-yl)-benzene. |

Even more preferred glucopyranosyl-substituted benzene derivatives of the formula (I) are selected from the compounds (1.8), (1.9) and (1.11).

Even more preferred glucopyranosyl-substituted benzene derivatives of the formula (I) are selected from the compounds (I.8) and (I.9), or a crystalline form (I.9X) of compound (I.9).

The pharmaceutical compositions according to the invention allow a high content uniformity and an effective production with regard to time and costs of pharmaceutical dosage forms, such as tablets and capsules. Furthermore, in one embodiment, these pharmaceutical dosage forms are in particular tablets.

Therefore in another aspect the present invention provides a pharmaceutical dosage form comprising a pharmaceutical composition according to the invention. In one aspect, the pharmaceutical dosage forms according to the invention is a solid pharmaceutical dosage form, for example a solid pharmaceutical dosage form for oral administration.

In another aspect, the present invention provides a process for the preparation of a pharmaceutical dosage form according to the invention comprising one or more granulation processes wherein the active pharmaceutical ingredient together with one or more excipients is granulated.

In another aspect, a pharmaceutical composition or dosage form according to the present invention exhibits a distinctive pharmacokinetic profile after administration to a subject, in particular after administration to a human, as for example described hereinbelow.

It can be found that a pharmaceutical composition comprising a SGLT2 inhibitor as defined hereinafter can advantageously be used for preventing, slowing progression of, delaying or treating a metabolic disorder, in particular for improving glycemic control in patients. This opens up new therapeutic possibilities in the treatment and prevention of type 2 diabetes mellitus, overweight, obesity, complications of diabetes mellitus and of neighboring disease states

Therefore, in a first aspect the present invention provides a method for preventing, slowing the progression of, delaying or treating a metabolic disorder selected from the group consisting of type 1 diabetes mellitus, type 2 diabetes mellitus, impaired glucose tolerance (IGT), impaired fasting blood glucose (IFG), hyperglycemia, postprandial hyperglycemia, overweight, obesity and metabolic syndrome in a patient in need thereof characterized in that a pharmaceutical composition or a pharmaceutical dosage form of the present invention is administered to the patient.

According to another aspect of the invention, there is provided a method for improving glycemic control and/or for reducing of fasting plasma glucose, of postprandial plasma glucose and/or of glycosylated hemoglobin HbA1c in a patient in need thereof characterized in that a pharmaceutical composition or a pharmaceutical dosage form of the present invention is administered to the patient.

The pharmaceutical composition according to this invention may also have valuable disease-modifying properties with respect to diseases or conditions related to impaired glucose tolerance (IGT), impaired fasting blood glucose (IFG), insulin resistance and/or metabolic syndrome.

According to another aspect of the invention, there is provided a method for preventing, slowing, delaying or reversing progression from impaired glucose tolerance (IGT), impaired fasting blood glucose (IFG), insulin resistance and/or from metabolic syndrome to type 2 diabetes mellitus in a patient in need thereof characterized in that a pharmaceutical composition or a pharmaceutical dosage form of the present invention is administered to the patient.

As by the use of a pharmaceutical composition according to this invention, an improvement of the glycemic control in patients in need thereof is obtainable, also those conditions and/or diseases related to or caused by an increased blood glucose level may be treated.

According to another aspect of the invention, there is provided a method for preventing, slowing the progression of, delaying or treating of a condition or disorder selected from the group consisting of complications of diabetes mellitus such as cataracts and micro- and macrovascular diseases, such as nephropathy, retinopathy, neuropathy, tissue ischaemia, diabetic foot, arteriosclerosis, myocardial infarction, accute coronary syndrome, unstable angina pectoris, stable angina pectoris, stroke, peripheral arterial occlusive disease, cardiomyopathy, heart failure, heart rhythm disorders and vascular restenosis, in a patient in need thereof characterized in that a pharmaceutical composition or a pharmaceutical dosage form of the present invention is administered to the patient. In particular one or more aspects of diabetic

nephropathy such as hyperperfusion, proteinuria and albuminuria may be treated, their progression slowed or their onset delayed or prevented. The term "tissue ischaemia" particularly comprises diabetic macroangiopathy, diabetic microangiopathy, impaired wound healing and diabetic ulcer. The terms "micro- and macrovascular diseases" and "micro- and macrovascular complications" are used interchangeably in this application.

By the administration of a pharmaceutical composition according to this invention and due to the activity of the SGLT2 inhibitor excessive blood glucose levels are not converted to insoluble storage forms, like fat, but excreted through the urine of the patient. Therefore, no gain in weight or even a reduction in body weight is the result.

According to another aspect of the invention, there is provided a method for reducing body weight or preventing an increase in body weight or facilitating a reduction in body weight in a patient in need thereof characterized in that a pharmaceutical composition or a pharmaceutical dosage form of the present invention is administered to the patient.

The pharmacological effect of the SGLT2 inhibitor in the pharmaceutical composition according to this invention is independent of insulin. Therefore, an improvement of the glycemic control is possible without an additional strain on the pancreatic beta cells. By an administration of a pharmaceutical composition according to this invention a beta-cell degeneration and a decline of beta-cell functionality such as for example apoptosis or necrosis of pancreatic beta cells can be delayed or prevented. Furthermore, the functionality of pancreatic cells can be improved or restored, and the number and size of pancreatic beta cells increased. It may be shown that the differentiation status and hyperplasia of pancreatic beta-cells disturbed by hyperglycemia can be normalized by treatment with a pharmaceutical composition according to this invention.

According to another aspect of the invention, there is provided a method for preventing, slowing, delaying or treating the degeneration of pancreatic beta cells and/or the decline of the functionality of pancreatic beta cells and/or for improving and/or restoring the functionality of pancreatic beta cells and/or restoring the functionality of pancreatic insulin secretion in a patient in need thereof characterized in that a pharmaceutical composition or a pharmaceutical dosage form of the present invention is administered to the patient.

By the administration of a pharmaceutical composition according to the present invention, an abnormal accumulation of fat in the liver may be reduced or inhibited. Therefore, according to another aspect of the present invention, there is provided a method for preventing, slowing, delaying or treating diseases or conditions attributed to an abnormal accumulation of liver fat in a patient in need thereof characterized in that an SGLT2 inhibitor as defined hereinbefore and hereinafter is administered to the patient. Diseases or conditions which are attributed to an abnormal accumulation of liver fat are particularly selected from the group consisting of general fatty liver, non-alcoholic fatty liver (NAFL), non-alcoholic steatohepatitis (NASH), hyperalimentation-induced fatty liver, diabetic fatty liver, alcoholic-induced fatty liver or toxic fatty liver.

As a result thereof, another aspect of the invention provides a method for maintaining and/or improving the insulin sensitivity and/or for treating or preventing hyperinsulinemia and/or insulin resistance in a patient in need thereof characterized in that a pharmaceutical composition or a pharmaceutical dosage form of the present invention is administered to the patient.

According to another aspect of the invention there is provided the use of a pharmaceutical composition or a pharmaceutical dosage form of the present invention for the manufacture of a medicament for

- preventing, slowing the progression of, delaying or treating a metabolic disorder selected from the group consisting of type 1 diabetes mellitus, type 2 diabetes mellitus, impaired glucose tolerance (IGT), impaired fasting blood glucose (IFG), hyperglycemia, postprandial hyperglycemia, overweight, obesity and metabolic syndrome; or
- improving glycemic control and/or for reducing of fasting plasma glucose, of postprandial plasma glucose and/or of glycosylated hemoglobin HbA1c; or
- preventing, slowing, delaying or reversing progression from impaired glucose tolerance (IGT), impaired fasting blood glucose (IFG), insulin resistance and/or from metabolic syndrome to type 2 diabetes mellitus; or
- preventing, slowing the progression of, delaying or treating of a condition or disorder selected from the group consisting of complications of diabetes mellitus such as cataracts and micro- and macrovascular diseases, such as nephropathy, retinopathy, neuropathy, tissue ischaemia, diabetic foot, arteriosclerosis, myocardial infarction, accute coronary syndrome, unstable angina pectoris, stable angina pectoris, stroke, peripheral arterial occlusive disease, cardiomyopathy, heart failure, heart rhythm disorders and vascular restenosis,; or
- reducing body weight or preventing an increase in body weight or facilitating a reduction in body weight; or
- preventing, slowing, delaying or treating the degeneration of pancreatic beta cells and/or the decline of the functionality of pancreatic beta cells and/or for improving and/or restoring the functionality of pancreatic beta cells and/or restoring the functionality of pancreatic insulin secretion; or
- preventing, slowing, delaying or treating diseases or conditions attributed to an abnormal accumulation of liver fat; or
- maintaining and/or improving the insulin sensitivity and/or for treating or preventing hyperinsulinemia and/or insulin resistance;

in a patient in need thereof characterized in that the SGLT2 inhibitor is administered, as defined hereinbefore and hereinafter.

According to another aspect of the invention, there is provided the use of a pharmaceutical composition or a pharmaceutical dosage form of the present invention according to the present invention for the manufacture of a medicament for a therapeutic and preventive method as described hereinbefore and hereinafter.

Accordingly, the present invention provides:

A pharmaceutical composition comprising a compound of the formula (I.9),

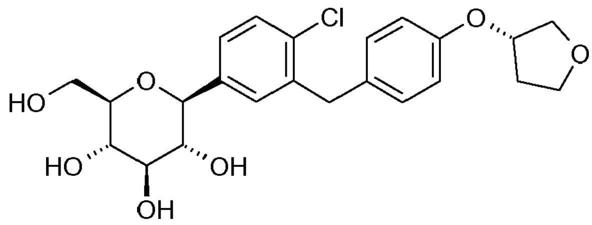

(I.9)

which when administered to a fasting human:

a. at a dose of 2.5 mg exhibits:

i. a $C_{max}$ of 40.3 to 96.3 nmol/L; and
ii. a AUC of 283 to 677 nmol*h/L; and/or

b. at a dose of 5.0 mg exhibits:

i. a $C_{max}$ of 123 to 230 nmol/L; and
ii. a AUC of 1,000 to 1,310 nmol*h/L; and/or

c. at a dose of 10.0 mg exhibits:

i. a $C_{max}$ of 143 to 796 nmol/L; and
ii. a AUC of 1,170 to 3,190 nmol*h/L; and/or

d. at a dose of 25.0 mg exhibits:

i. a $C_{max}$ of 334 to 1,030 nmol/L; and
ii. a AUC of 2,660 to 7,640 nmol*h/L; and/or

e. at a dose of 50.0 mg exhibits:

i. a $C_{max}$ of 722 to 2,020 nmol/L; and
ii. a AUC of 6,450 to 14,100 nmol*h/L.

A pharmaceutical composition comprising a compound of the formula (I.9),

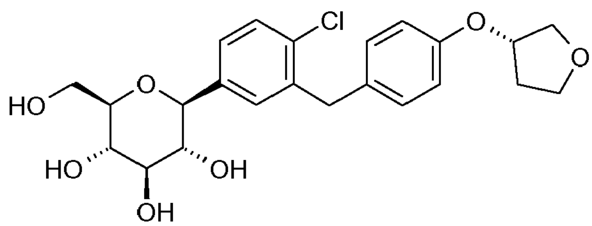

(I.9)

which when administered to a fasting human:

a. at a dose of 2.5 mg exhibits:

i. a geometric mean $C_{max}$ of 52.9 to 66.6 nmol/L; and
ii. a geometric mean AUC of 394 to 468 nmol*h/L; and/or

b. at a dose of 10.0 mg exhibits:

i. a geometric mean $C_{max}$ of 221 to 372 nmol/L; and
ii. a geometric mean AUC of 1,690 to 2,660 nmol*h/L; and/or

c. at a dose of 25.0 mg exhibits:

i. a geometric mean $C_{max}$ of 490 to 709 nmol/L; and
ii. a geometric mean AUC of 3,750 to 6,130 nmol*h/L; and/or

d. at a dose of 50.0 mg exhibits:

i. a geometric mean $C_{max}$ of 1,080 to 1,140 nmol/L; and
ii. a geometric mean AUC of 8,310 to 8,460 nmol*h/L.

A pharmaceutical composition comprising a compound of the formula (I.9), which when administered to a fasting human as:

a. a single dose of 2.5 mg exhibits:

i. a $C_{max}$ of 42.8 to 81.2 nmol/L; and
ii. a $AUC_{0\text{-}inf}$ of 326 to 631 nmol*h/L; and/or

b. a single dose of 5.0 mg exhibits:

i. a $C_{max}$ of 123 to 230 nmol/L; and
ii. a $AUC_{0\text{-}inf}$ of 1,000 to 1,310 nmol*h/L; and/or

c. a single dose of 10.0 mg exhibits:

i. a $C_{max}$ of 143 to 796 nmol/L; and
ii. a $AUC_{0\text{-}inf}$ of 1,170 to 3,190 nmol*h/L; and/or

d. a single dose of 25.0 mg exhibits:

i. a $C_{max}$ of 334 to 1,030 nmol/L; and
ii. a $AUC_{0\text{-}inf}$ of 2,660 to 7,170 nmol*h/L; and/or

e. a single dose of 50.0 mg exhibits:

i. a $C_{max}$ of 722 to 2,020 nmol/L; and
ii. a $AUC_{0\text{-}inf}$ of 6,450 to 14,100 nmol*h/L.

A pharmaceutical composition comprising a compound of the formula (I.9), which when administered to a fasting human as:

a. a single dose of 2.5 mg exhibits:

i. a geometric mean $C_{max}$ of 52.9 to 61.3 nmol/L; and
ii. a geometric mean $AUC_{0\text{-}inf}$ of 394 to 468 nmol*h/L; and/or

b. a single dose of 10.0 mg exhibits:

i. a geometric mean $C_{max}$ of 221 to 372 nmol/L; and
ii. a geometric mean $AUC_{0\text{-}inf}$ of 1,690 to 2,660 nmol*h/L; and/or

c. a single dose of 25.0 mg exhibits:

i. a geometric mean $C_{max}$ of 490 to 709 nmol/L; and
ii. a geometric mean $AUC_{0\text{-}inf}$ of 3,750 to 6,130 nmol*h/L; and/or

d. a single dose of 50.0 mg exhibits:

i. a geometric mean $C_{max}$ of 1,080 to 1,140 nmol/L; and
ii. a geometric mean $AUC_{0\text{-}inf}$ of 8,310 to 8,460 nmol*h/L.

A pharmaceutical composition comprising a compound of the formula (I.9), which when administered to a fasting human:

a. in multiple doses of 2.5 mg exhibits:

i. a $C_{max,ss}$ of 40.3 to 96.3 nmol/L; and
ii. a $AUC_{\tau,ss}$ of 283 to 677 nmol*h/L; and/or

b. in multiple doses of 10.0 mg exhibits:

i. a $C_{max,ss}$ of 166 to 479 nmol/L; and
ii. a $AUC_{\tau,ss}$ of 1,350 to 2,600 nmol*h/L; and/or

c. in multiple doses of 25.0 mg exhibits:

i. a $C_{max,ss}$ of 443 to 907 nmol/L; and
ii. a $AUC_{\tau,ss}$ of 2,790 to 7,640 nmol*h/L.

A pharmaceutical composition comprising a compound of the formula (I.9), which when administered to a fasting human:

a. in multiple doses of 10.0 mg exhibits:

i. a geometric mean $C_{max,ss}$ of 252 to 272 nmol/L; and
ii. a geometric mean $AUC_{\tau,ss}$ of 1,850 to 2,000 nmol*h/L; and/or

b. in multiple doses of 25.0 mg exhibits:

i. a geometric mean $C_{max,ss}$ of 622 to 676 nmol/L; and
ii. a geometric mean $AUC_{\tau,ss}$ of 4,640 to 4,890 nmol*h/L.

A pharmaceutical composition comprising a compound of the formula (I.9),

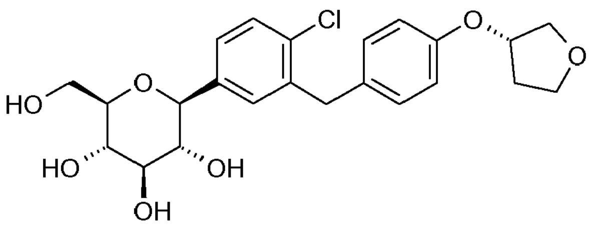

(I.9)

which when administered to a fasting human exhibits a dose-normalized $C_{max,\ norm}$ of 13 to 80 nmol/L/mg; and a dose-normalized $AUC_{0\text{-}inf,\ norm}$ of 106 to 306 nmol*h/L/mg. In one embodiment, said pharmaceutical composition exhibits said dose-normalized $C_{max,\ norm}$ and said dose-normalized $AUC_{0\text{-}inf,\ norm}$ over a dose range of 2.5 mg to 50 mg of said compound of the formula (I.9).

A pharmaceutical composition comprising a compound of the formula (I.9),

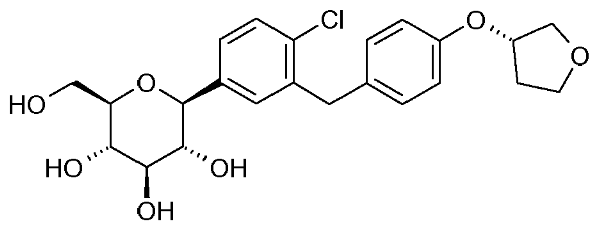

(I.9)

which when administered to a fasting human exhibits a dose-normalized $C_{max}$, $_{norm}$ of 13 to 80 nmol/L/mg; and a dose-normalized $AUC_{0-inf}$, $_{norm}$ of 106 to 306 nmol*h/L/mg over a dose range of 5 mg to 25 mg of said compound of the formula (I.9).

A pharmaceutical composition comprising a compound of the formula (I.9),

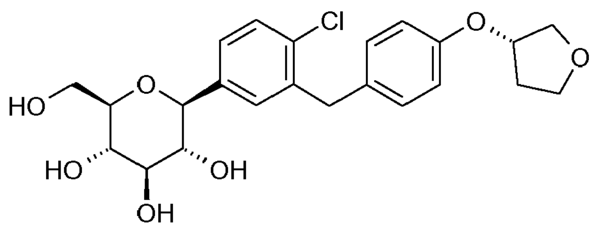

(I.9)

which when administered to a fasting human exhibits a dose-normalized geometric mean $C_{max}$, $_{norm}$ of 20 to 37 nmol/L/mg; and a dose-normalized geometric mean $AUC_{0-inf}$, $_{norm}$ of 150 to 266 nmol*h/L/mg. In one embodiment, said pharmaceutical composition exhibits said dose-normalized geometric mean $C_{max}$, $_{norm}$ and said dose-normalized geometric mean $AUC_{0-inf}$, $_{norm}$ over a dose range of 2.5 mg to 50 mg of said compound of the formula (I.9).

A pharmaceutical composition comprising a compound of the formula (I.9),

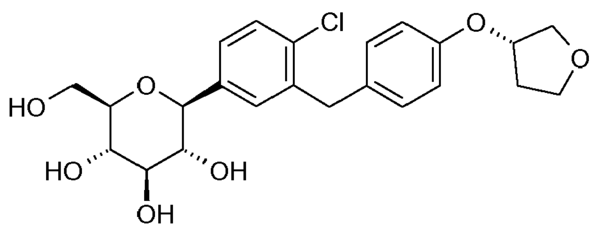

(I.9)

which when administered to a fasting human exhibits a dose-normalized geometric mean $C_{max}$, $_{norm}$ of 20 to 37 nmol/L/mg; and a dose-normalized geometric mean $AUC_{0-inf, norm}$ of 150 to 266 nmol*h/L/mg over a dose range of 5 mg to 25 mg of said compound of the formula (I.9).

A pharmaceutical composition comprising a compound of the formula (I.9),

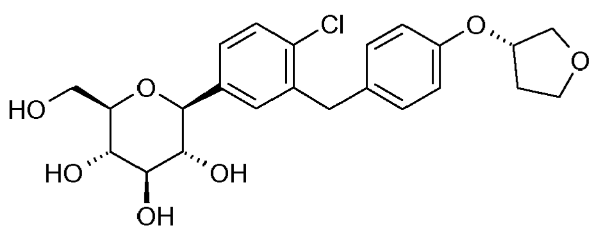

(I.9)

which when administered to a fasting human as a single dose exhibits a dose-normalized $C_{max}$, $_{norm}$ of 13 to 80 nmol/L/mg; and a dose-normalized $AUC_{0-inf, norm}$ of 106 to 287 nmol*h/L/mg. In one embodiment, said pharmaceutical composition exhibits said dose-normalized $C_{max}$, $_{norm}$ and said dose-normalized $AUC_{0-inf, norm}$ over a dose range of 2.5 mg to 50 mg of said compound of the formula (I.9) when administered to a fasting human as a single dose.

A pharmaceutical composition comprising a compound of the formula (I.9),

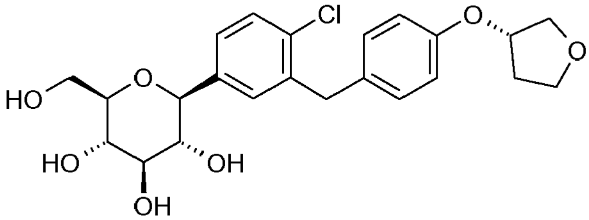

(I.9)

which when administered to a fasting human as a single dose exhibits a dose-normalized $C_{max, norm}$ of 13 to 80 nmol/L/mg; and a dose-normalized $AUC_{0-inf, norm}$ of 106 to 287 nmol*h/L/mg over a dose range of 5 mg to 25 mg of said compound of the formula (I.9).

A pharmaceutical composition comprising a compound of the formula (I.9),

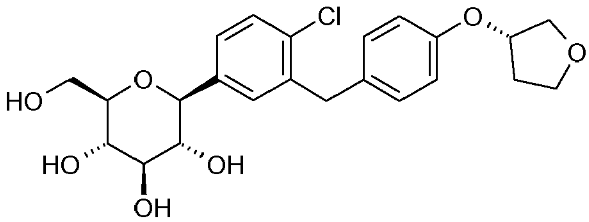

(I.9)

which when administered to a fasting human as a single dose exhibits a dose-normalized geometric mean $C_{max, norm}$ of 20 to 37 nmol/L/mg; and a dose-normalized geometric mean $AUC_{0-inf, norm}$ of 150 to 266 nmol*h/L/mg. In one embodiment, said pharmaceutical composition exhibits said dose-normalized geometric mean $C_{max, norm}$ and said dose-normalized geometric mean $AUC_{0-inf, norm}$ over a dose range of 2.5 mg to 50 mg of said compound of the formula (I.9) when administered to a fasting human as a single dose.

A pharmaceutical composition comprising a compound of the formula (I.9),

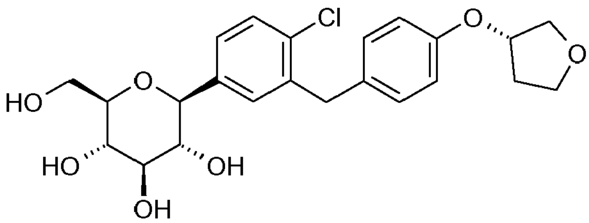

(I.9)

which when administered to a fasting human as a single dose exhibits a dose-normalized geometric mean $C_{max, norm}$ of 20 to 37 nmol/L/mg; and a dose-normalized geometric mean $AUC_{0-inf, norm}$ of 150 to 266 nmol*h/L/mg over a dose range of 5 mg to 25 mg of said compound of the formula (I.9).

A pharmaceutical composition comprising a compound of the formula (I.9),

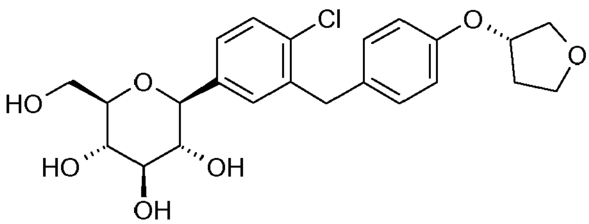

(I.9)

which when administered to a fasting human in multiple doses exhibits a dose-normalized $C_{max,ss, norm}$ of 16 to 48 nmol/L/mg; and a dose-normalized $AUC_{\tau,ss, norm}$ of 112 to 306 nmol*h/L/mg. In one embodiment, said pharmaceutical composition exhibits said dose-normalized $C_{max,ss, norm}$ and said dose-normalized $AUC_{\tau,ss, norm}$ over a dose range of 2.5 mg to 25 mg of said compound of the formula (I.9) when administered to a fasting human in multiple doses.

A pharmaceutical composition comprising a compound of the formula (I.9),

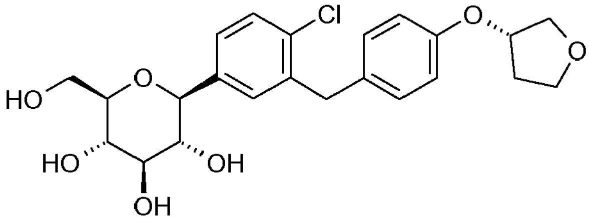

(I.9)

which when administered to a fasting human in multiple doses exhibits a dose-normalized geometric mean $C_{max,ss, norm}$ of 25 to 27 nmol/L/mg; and a dose-normalized geometic mean $AUC_{\tau,ss, norm}$ of 184 to 200 nmol*h/L/mg. In one embodiment, said pharmaceutical composition exhibits said dose-normalized geometric mean $C_{max,ss, norm}$ and said dose-normalized geometric mean $AUC_{\tau,ss, norm}$ over a dose range of 2.5 mg to 25 mg of said compound of the formula (I.9) when administered to a fasting human in multiple doses.

A pharmaceutical composition as provided above, wherein the particle size distribution in said composition is X90 < 200 µm.

A pharmaceutical composition as provided above, wherein said compound of the formula (I.9) represents 25% or less of the weight of said composition.

A pharmaceutical composition comprising a compound of the formula (I.9),

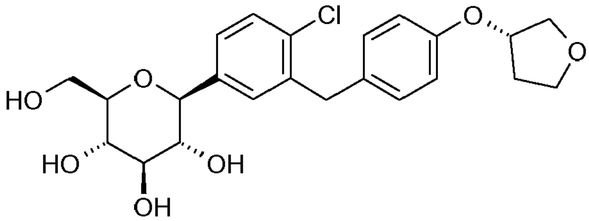

(I.9)

wherein the particle size distribution in said composition is X90 < 200 µm, wherein said compound of the formula (I.9) represents 25% or less of the weight of said composition.

A pharmaceutical composition as provided above, wherein said composition comprises crystalline form (L9X) of said compound of the formula (I.9).

A pharmaceutical composition as provided above, wherein the particle size distribution in said composition is X90 ≤ 150 µm, X90 ≤ 100 µm or X90 ≤ 90 µm.

A pharmaceutical composition as provided above, wherein said compound of the formula (I.9) represents 20% or less of the weight of said composition or 15% or less of the weight of said composition.

A pharmaceutical composition as provided above, wherein the particle size distribution in said composition is X90 < 100 µm, wherein said compound of the formula (I.9) represents 20% or less of the weight of said composition.

A pharmaceutical composition as provided above, wherein the particle size distribution in said composition is X90 < 90 µm, wherein said compound of the formula (I.9) represents 15% or less of the weight of said composition.

A pharmaceutical composition as provided above, wherein said composition comprises a disintegrant and a binder, wherein the ratio of said disintegrant to said binder is between 1.5 : 3.5 and 1 : 1 (weight/weight).

A pharmaceutical composition as provided above, wherein at least 99% of the particles of said binder (by weight) are 250 µm or smaller.

A pharmaceutical composition as provided above, wherein said binder is hydroxypropyl cellulose.

A pharmaceutical composition as provided above, wherein said composition is obtained by high shear wet granulation, wherein said composition further comprising a diluent, wherein 5 - 20% (by weight) of said diluent is added to said composition as a dry add after said wet granulation.

A pharmaceutical composition as provided above, wherein said diluent is microcrystalline cellulose.

A pharmaceutical composition as provided above, wherein said high shear wet granulation comprises the steps of:

(1) Premixing the compound of the formula (I.9) and the main portion of the excipients including the binder in a mixer to obtain a pre-mixture;
(2) granulating the pre-mixture of step (1) by adding the granulation liquid, preferably water;
(3) drying the granules of step (2) in a fluidized bed dryer or a drying oven;
(4) optionally dry sieving of the dried granules of step (3);
(5) mixing the dried granules of step (4) with the remaining excipients like glidant and lubricant in a mixer to obtain

the final mixture;
(6) tableting the final mixture of step (5) by compressing it on a suitable tablet press to produce tablets cores;
(7) optionally film-coating of the tablet cores of step (6) with a film coat.

A pharmaceutical composition as provided above, wherein said composition comprises:

| | Amount (% by weight) |
|---|---|
| the compound of the formula (I.9) | 0.5 - 25 |
| one or more diluents | 65 - 93 |
| one or more binders | 1 - 5 |
| one or more disintegrants | 1 - 4 |
| optionally one or more additional additives | ad 100 % |

A pharmaceutical composition as provided above, wherein said composition comprises:

| | Amount (% by weight) |
|---|---|
| the compound of the formula (I.9) | 0.5 - 25 |
| Lactose monohydrate | 28 - 70 |
| Microcrystalline cellulose | 20 - 50 |
| Hydroxypropyl cellulose | 1 - 5 |
| Croscarmellose sodium | 1 - 4 |
| Additional additives | ad 100 % |

A pharmaceutical composition as provided above, further comprising one or more lubricants.
A pharmaceutical composition as provided above, wherein said lubricant is magnesium stearate.
A pharmaceutical composition as provided above, further comprising one or more glidants.
A pharmaceutical composition as provided above, wherein said glidant is colloidal silicon dioxide.
A pharmaceutical composition as provided above, further comprising one or more film coats.
A pharmaceutical composition as provided above, wherein said film coat is applied in a concentration of 1 - 5 % and comprises hypromellose, polyethylene glycol, talc, titanium dioxide, iron oxides and optionally further colorants.
A pharmaceutical dosage form comprising any one of the pharmaceutical compositions provided above. For example the pharmaceutical dosage form is a tablet.
A method of treating disease(s), as set forth herein comprising administering to a patient any one of the pharmaceutical compositions or a pharmaceutical dosage forms provided above.
A wet granulation process for making a pharmaceutical dosage form comprising a compound of the formula (I.9) and one or more excipients, wherein said process comprises the steps of:

(1) Premixing said compound of the formula (I.9) and the main portion of the excipients including a binder in a mixer to obtain a pre-mixture;
(2) granulating the pre-mixture of step (1) by adding a granulation liquid, preferably water;
(3) drying the granules of step (2) in a fluidized bed dryer or a drying oven;
(4) optionally dry sieving of the dried granules of step (3);
(5) mixing the dried granules of step (4) with the remaining excipients in a mixer to obtain the final mixture;
(6) tableting the final mixture of step (5) by compressing it on a suitable tablet press to produce tablets cores;
(7) optionally film-coating of the tablet cores of step (6) with a film coat.

In one embodiment, at least 99% of the particles of said binder (by weight) are 250 μm or smaller. In one embodiment, the excipients in said step (1) also include a diluant, wherein 80 - 95% (by weight) of the diluent is premixed with the compound of the formula (I.9) is step (1) and 5 - 20% (by weight) of the diluent is added to said composition as a dry add in step (5).
A pharmaceutical composition obtainable by a process provided above.

A direct compression process for making a pharmaceutical composition comprising a compound of the formula (I.9) and one or more excipients, wherein said process comprises the steps of:

(1) Premixing said compound of the formula (I.9) and the main portion of the excipients in a mixer to obtain a pre-mixture;
(2) optionally dry screening the pre-mixture through a screen in order to segregate cohesive particles and to improve content uniformity;
(3) mixing the pre-mixture of step (1) or (2) in a mixer, optionally by adding remaining excipients to the mixture and continuing mixing;
(4) tableting the final mixture of step (3) by compressing it on a suitable tablet press to produce the tablet cores;
(5) optionally film-coating of the tablet cores of step (4) with a film coat.

A pharmaceutical composition obtainable by the process provided above.
A dry granulation process for making a pharmaceutical composition comprising a compound of the formula (I.9) and one or more excipients, wherein said process comprises the steps of:

(1) mixing said compound of the formula (I.9) with either all or a portion of the excipients in a mixer;
(2) compaction of the mixture of step (1) on a suitable roller compactor;
(3) reducing the ribbons obtained during step (2) to granules by suitable milling or sieving steps;
(4) optionally mixing the granules of step (3) with the remaining excipients in a mixer to obtain the final mixture;
(5) tabletting the granules of step (3) or the final mixture of step (4) by compressing it on a suitable tablet press to produce the tablet cores;
(6) optionally film-coating of the tablet cores of step (5) with a fim coat.

A pharmaceutical composition obtainable by the process above.

**Definitions**

The term **"active ingredient"** of a pharmaceutical composition according to the present invention means the SGLT2 inhibitor according to the present invention. An "active ingredient is also sometimes referred to herein as an "active substance".

The term **"body mass index"** or **"BMI"** of a human patient is defined as the weight in kilograms divided by the square of the height in meters, such that BMI has units of kg/m$^2$.

The term **"overweight"** is defined as the condition wherein the individual has a BMI greater than or 25 kg/m$^2$ and less than 30 kg/m$^2$. The terms "overweight" and "pre-obese" are used interchangeably.

The term **"obesity"** is defined as the condition wherein the individual has a BMI equal to or greater than 30 kg/m$^2$. According to a WHO definition the term obesity may be categorized as follows: the term "class I obesity" is the condition wherein the BMI is equal to or greater than 30 kg/m$^2$ but lower than 35 kg/m$^2$; the term "class II obesity" is the condition wherein the BMI is equal to or greater than 35 kg/m$^2$ but lower than 40 kg/m$^2$; the term "class III obesity" is the condition wherein the BMI is equal to or greater than 40 kg/m$^2$.

The term **"visceral obesity"** is defined as the condition wherein a waist-to-hip ratio of greater than or equal to 1.0 in men and 0.8 in women is measured. It defines the risk for insulin resistance and the development of pre-diabetes.

The term **"abdominal obesity"** is usually defined as the condition wherein the waist circumference is > 40 inches or 102 cm in men, and is > 35 inches or 94 cm in women. With regard to a Japanese ethnicity or Japanese patients abdominal obesity may be defined as waist circumference ≥ 85 cm in men and ≥ 90 cm in women (see e.g. investigating committee for the diagnosis of metabolic syndrome in Japan).

The term "**euglycemia**" is defined as the condition in which a subject has a fasting blood glucose concentration within the normal range, greater than 70 mg/dL (3.89 mmol/L) and less than 110 mg/dL (6.11 mmol/L). The word "fasting" has the usual meaning as a medical term.

The term "**hyperglycemia**" is defined as the condition in which a subject has a fasting blood glucose concentration above the normal range, greater than 110 mg/dL (6.11 mmol/L). The word "fasting" has the usual meaning as a medical term.

The term "**hypoglycemia**" is defined as the condition in which a subject has a blood glucose concentration below the normal range of 60 to 115 mg/dL (3.3 to 6.3 mmol/L).

The term **"postprandial hyperglycemia"** is defined as the condition in which a subject has a 2 hour postprandial blood glucose or serum glucose concentration greater than 200 mg/dL (11.11 mmol/L).

The term "**impaired fasting blood glucose**" or **"IFG"** is defined as the condition in which a subject has a fasting blood glucose concentration or fasting serum glucose concentration in a range from 100 to 125 mg/dl (i.e. from

5.6 to 6.9 mmol/l), in particular greater than 110 mg/dL and less than 126 mg/dl (7.00 mmol/L). A subject with "normal fasting glucose" has a fasting glucose concentration smaller than 100 mg/dl, i.e. smaller than 5.6 mmol/l.

The term "**impaired glucose tolerance**" or **"IGT"** is defined as the condition in which a subject has a 2 hour postprandial blood glucose or serum glucose concentration greater than 140 mg/dl (7.78 mmol/L) and less than 200 mg/dL (11.11 mmol/L). The abnormal glucose tolerance, i.e. the 2 hour postprandial blood glucose or serum glucose concentration can be measured as the blood sugar level in mg of glucose per dL of plasma 2 hours after taking 75 g of glucose after a fast. A subject with "normal glucose tolerance" has a 2 hour postprandial blood glucose or serum glucose concentration smaller than 140 mg/dl (7.78 mmol/L).

The term "**hyperinsulinemia**" is defined as the condition in which a subject with insulin resistance, with or without euglycemia, has fasting or postprandial serum or plasma insulin concentration elevated above that of normal, lean individuals without insulin resistance, having a waist-to-hip ratio < 1.0 (for men) or < 0.8 (for women).

The terms "insulin-sensitizing", "insulin resistance-improving" or "insulin resistance-lowering" are synonymous and used interchangeably.

The term "**insulin resistance**" is defined as a state in which circulating insulin levels in excess of the normal response to a glucose load are required to maintain the euglycemic state (Ford ES, et al. JAMA. (2002) 287:356-9). A method of determining insulin resistance is the euglycaemic-hyperinsulinaemic clamp test. The ratio of insulin to glucose is determined within the scope of a combined insulin-glucose infusion technique. There is found to be insulin resistance if the glucose absorption is below the 25th percentile of the background population investigated (WHO definition). Rather less laborious than the clamp test are so called minimal models in which, during an intravenous glucose tolerance test, the insulin and glucose concentrations in the blood are measured at fixed time intervals and from these the insulin resistance is calculated. With this method, it is not possible to distinguish between hepatic and peripheral insulin resistance.

Furthermore, insulin resistance, the response of a patient with insulin resistance to therapy, insulin sensitivity and hyperinsulinemia may be quantified by assessing the "homeostasis model assessment to insulin resistance (HOMA-IR)" score, a reliable indicator of insulin resistance (Katsuki A, et al. Diabetes Care 2001; 24: 362-5). Further reference is made to methods for the determination of the HOMA-index for insulin sensitivity (Matthews et al., Diabetologia 1985, 28: 412-19), of the ratio of intact proinsulin to insulin (Forst et al., Diabetes 2003, 52(Suppl.1): A459) and to an euglycemic clamp study. In addition, plasma adiponectin levels can be monitored as a potential surrogate of insulin sensitivity. The estimate of insulin resistance by the homeostasis assessment model (HOMA)-IR score is calculated with the formula (Galvin P, et al. Diabet Med 1992;9:921-8):

$$\text{HOMA-IR} = [\text{fasting serum insulin } (\mu U/mL)] \times [\text{fasting plasma glucose}(mmol/L)/22.5]$$

As a rule, other parameters are used in everyday clinical practice to assess insulin resistance. Preferably, the patient's triglyceride concentration is used, for example, as increased triglyceride levels correlate significantly with the presence of insulin resistance.

Patients with a predisposition for the development of IGT or IFG or type 2 diabetes are those having euglycemia with hyperinsulinemia and are by definition, insulin resistant. A typical patient with insulin resistance is usually overweight or obese. If insulin resistance can be detected, this is a particularly strong indication of the presence of pre-diabetes. Thus, it may be that in order to maintain glucose homoeostasis a person needs 2-3 times as much insulin as a healthy person, without this resulting in any clinical symptoms.

The methods to investigate the **function of pancreatic beta-cells** are similar to the above methods with regard to insulin sensitivity, hyperinsulinemia or insulin resistance: An improvement of beta-cell function can be measured for example by determining a HOMA-index for beta-cell function (Matthews et al., Diabetologia 1985, 28: 412-19), the ratio of intact proinsulin to insulin (Forst et al., Diabetes 2003, 52(Suppl.1): A459), the insulin/C-peptide secretion after an oral glucose tolerance test or a meal tolerance test, or by employing a hyperglycemic clamp study and/or minimal modeling after a frequently sampled intravenous glucose tolerance test (Stumvoll et al., Eur J Clin Invest 2001, 31: 380-81).

The term "**pre-diabetes**" is the condition wherein an individual is pre-disposed to the development of type 2 diabetes. Pre-diabetes extends the definition of impaired glucose tolerance to include individuals with a fasting blood glucose within the high normal range ≥ 100 mg/dL (J. B. Meigs, et al. Diabetes 2003; 52:1475-1484) and fasting hyperinsulinemia (elevated plasma insulin concentration). The scientific and medical basis for identifying pre-diabetes as a serious health threat is laid out in a Position Statement entitled "The Prevention or Delay of Type 2 Diabetes" issued jointly by the American Diabetes Association and the National Institute of Diabetes and Digestive and Kidney Diseases (Diabetes Care 2002; 25:742-749).

Individuals likely to have insulin resistance are those who have two or more of the following attributes: 1) overweight or obese, 2) high blood pressure, 3) hyperlipidemia, 4) one or more 1$^{st}$ degree relative with a diagnosis of IGT or IFG or type 2 diabetes. Insulin resistance can be confirmed in these individuals by calculating the HOMA-IR

score. For the purpose of this invention, insulin resistance is defined as the clinical condition in which an individual has a HOMA-IR score > 4.0 or a HOMA-IR score above the upper limit of normal as defined for the laboratory performing the glucose and insulin assays.

The term "**type 2 diabetes**" is defined as the condition in which a subject has a fasting blood glucose or serum glucose concentration greater than 125 mg/dL (6.94 mmol/L). The measurement of blood glucose values is a standard procedure in routine medical analysis. If a glucose tolerance test is carried out, the blood sugar level of a diabetic will be in excess of 200 mg of glucose per dL (11.1 mmol/l) of plasma 2 hours after 75 g of glucose have been taken on an empty stomach. In a glucose tolerance test 75 g of glucose are administered orally to the patient being tested after 10-12 hours of fasting and the blood sugar level is recorded immediately before taking the glucose and 1 and 2 hours after taking it. In a healthy subject, the blood sugar level before taking the glucose will be between 60 and 110 mg per dL of plasma, less than 200 mg per dL 1 hour after taking the glucose and less than 140 mg per dL after 2 hours. If after 2 hours the value is between 140 and 200 mg, this is regarded as abnormal glucose tolerance.

The term **"late stage type 2 diabetes mellitus"** includes patients with a secondary drug failure, indication for insulin therapy and progression to micro- and macrovascular complications e.g. diabetic nephropathy, or coronary heart disease (CHD).

The term "**HbA1c**" refers to the product of a non-enzymatic glycation of the haemoglobin B chain. Its determination is well known to one skilled in the art. In monitoring the treatment of diabetes mellitus the HbA1c value is of exceptional importance. As its production depends essentially on the blood sugar level and the life of the erythrocytes, the HbA1c in the sense of a "blood sugar memory" reflects the average blood sugar levels of the preceding 4-6 weeks. Diabetic patients whose HbA1c value is consistently well adjusted by intensive diabetes treatment (i.e. < 6.5 % of the total haemoglobin in the sample), are significantly better protected against diabetic microangiopathy. For example, metformin on its own achieves an average improvement in the HbA1c value in the diabetic of the order of 1.0 - 1.5 %. This reduction of the HbA1C value is not sufficient in all diabetics to achieve the desired target range of < 6.5 % and preferably < 6 % HbA1c.

The term **"insufficient glycemic control"** or "inadequate glycemic control" in the scope of the present invention means a condition wherein patients show HbA1c values above 6.5 %, in particular above 7.0 %, even more preferably above 7.5 %, especially above 8 %.

The "**metabolic syndrome**", also called "syndrome X" (when used in the context of a metabolic disorder), also called the "dysmetabolic syndrome" is a syndrome complex with the cardinal feature being insulin resistance (Laaksonen DE, et al. Am J Epidemiol 2002;156:1070-7). According to the ATP III/NCEP guidelines (Executive Summary of the Third Report of the National Cholesterol Education Program (NCEP) Expert Panel on Detection, Evaluation, and Treatment of High Blood Cholesterol in Adults (Adult Treatment Panel III) JAMA: Journal of the American Medical Association (2001) 285:2486-2497), diagnosis of the metabolic syndrome is made when three or more of the following risk factors are present:

1. Abdominal obesity, defined as waist circumference > 40 inches or 102 cm in men, and > 35 inches or 94 cm in women; or with regard to a Japanese ethnicity or Japanese patients defined as waist circumference ≥ 85 cm in men and ≥ 90 cm in women;
2. Triglycerides: ≥ 150 mg/dL
3. HDL-cholesterol < 40 mg/dL in men
4. Blood pressure ≥ 130/85 mm Hg (SBP ≥ 130 or DBP ≥ 85)
5. Fasting blood glucose ≥ 110 mg/dL

The NCEP definitions have been validated (Laaksonen DE, et al. Am J Epidemiol. (2002) 156:1070-7). Triglycerides and HDL cholesterol in the blood can also be determined by standard methods in medical analysis and are described for example in Thomas L (Editor): "Labor und Diagnose", TH-Books Verlagsgesellschaft mbH, Frankfurt/Main, 2000.

According to a commonly used definition, **hypertension** is diagnosed if the systolic blood pressure (SBP) exceeds a value of 140 mm Hg and diastolic blood pressure (DBP) exceeds a value of 90 mm Hg. If a patient is suffering from manifest diabetes it is currently recommended that the systolic blood pressure be reduced to a level below 130 mm Hg and the diastolic blood pressure be lowered to below 80 mm Hg.

The term **"SGLT2 inhibitor"** in the scope of the present invention relates to compounds, in particular to glucopyranosyl-derivatives, i.e. compounds having a glucopyranosyl-moiety, which show an inhibitory effect on the sodium-glucose transporter 2 (SGLT2), in particular the human SGLT2. The inhibitory effect on hSGLT2 measured as IC50 is prerably below 1000 nM, even more preferably below 100 nM, most preferably below 50 nM. The inhibitory effect on hSGLT2 can be determined by methods known in the literature, in particular as described in the application WO 2005/092877 or WO 2007/093610 (pages 23/24), which are incorporated herein by reference in its entirety. The term "SGLT2 inhibitor" also comprises any pharmaceutically acceptable salts thereof, hydrates and solvates thereof, including

the respective crystalline forms.

The terms **"treatment"** and "treating" comprise therapeutic treatment of patients having already developed said condition, in particular in manifest form. Therapeutic treatment may be symptomatic treatment in order to relieve the symptoms of the specific indication or causal treatment in order to reverse or partially reverse the conditions of the indication or to stop or slow down progression of the disease. Thus the compositions and methods of the present invention may be used for instance as therapeutic treatment over a period of time as well as for chronic therapy.

The terms **"prophylactically treating",** "preventivally treating" and "preventing" are used interchangeably and comprise a treatment of patients at risk to develop a condition mentioned hereinbefore, thus reducing said risk.

The term **"tablet"** comprises tablets without a coating and tablets with one or more coatings. Furthermore the "term" tablet comprises tablets having one, two, three or even more layers and press-coated tablets, wherein each of the beforementioned types of tablets may be without or with one or more coatings. The term "tablet" also comprises mini, melt, chewable, effervescent and orally disintegrating tablets.

The terms **"pharmacopoe"** and **"pharmacopoeias"** refer to standard pharmacopoeias such as the "USP 31-NF 26 through Second Supplement" (United States Pharmacopeial Convention) or the "European Pharmacopoeia 6.3" (European Directorate for the Quality of Medicines and Health Care, 2000-2009).

**Brief Description of the Figures**

Figure 1 shows an X-ray powder diffractogram of the crystalline form (I.9X) of the compound (I.9).
Figure 2 shows the thermoanalysis and determination of the melting point via DSC of the crystalline form (I.9X) of the compound (I.9).
Figure 3 shows the results of the administration of a compound of the invention to ZDF rats.

**Detailed Description**

The aspects according to the present invention, in particular the pharmaceutical compositions, methods and uses, refer to SGLT2 inhibitors as defined hereinbefore and hereinafter.

Preferably the SGLT2 inhibitor is selected from a glucopyranosyl-substituted benzene derivative of the formula (I)

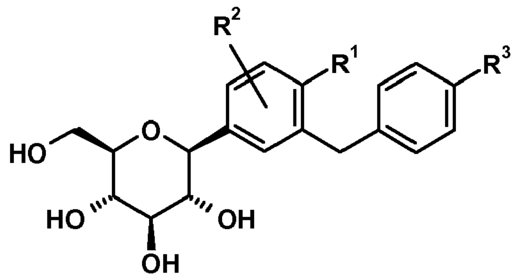

I

wherein $R^1$ denotes Cl, methyl or cyano; $R^2$ denotes H, methyl, methoxy or hydroxy and $R^3$ denotes (*R*)-tetrahydrofuran-3-yloxy or (*S*)-tetrahydrofuran-3-yloxy; or a prodrug of one of the beforementioned SGLT2 inhibitors.

Compounds of the formula (I) and methods of their synthesis are described for example in the following patent applications: WO 2005/092877, WO 2006/117360, WO 2006/117359, WO 2006/120208, WO 2006/064033, WO 2007/031548, WO 2007/093610, WO 2008/020011, WO 2008/055870.

In the above glucopyranosyl-substituted benzene derivatives of the formula (I) the following definitions of the substituents are preferred.

Preferably $R^1$ denotes chloro or cyano; in particular chloro.
Preferably $R^2$ denotes H.
Preferably $R^3$ denotes (*R*)-tetrahydrofuran-3-yloxy or (*S*)-tetrahydrofuran-3-yloxy.

Preferred glucopyranosyl-substituted benzene derivatives of the formula (I) are selected from the group of compounds (I.8) to (i.11):

| | |
|---|---|
| (I.8) | 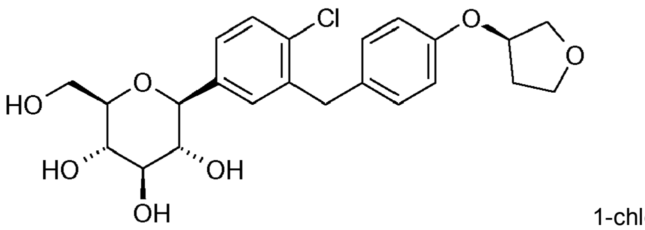 1-chloro-4-(β-D-glucopyranos-1-yl)-2-[4-(*(R)*-tetrahydrofuran-3-yloxy)-benzyl]-benzene, |
| (I.9) | 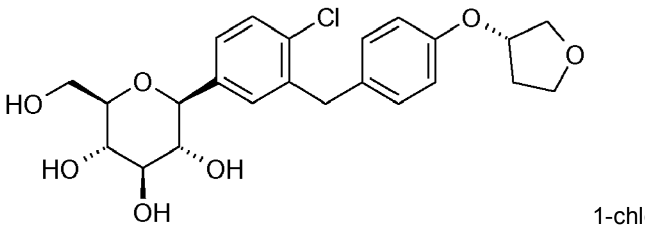 1-chloro-4-(β-D-glucopyranos-1-yl)-2-[4-(*(S)*-tetrahydrofuran-3-yloxy)-benzyl]-benzene, |
| (I. 10) | 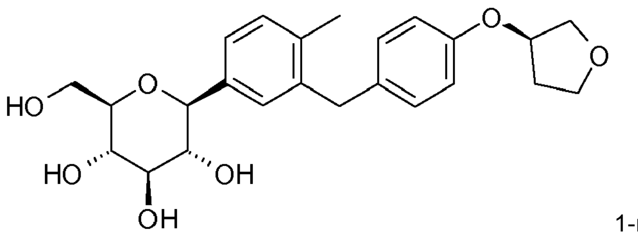 1-methyl-2-[4-(*(R)*-tetrahydrofuran-3-yloxy)-benzyl]-4-(β-D-glucopyranos-1-yl)-benzene, |
| (I. 11) | 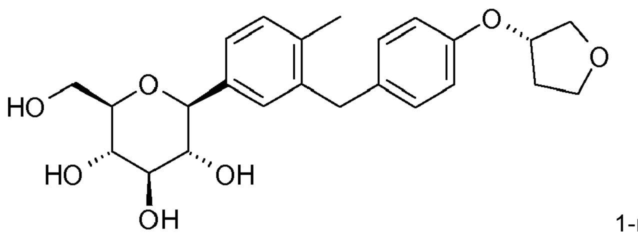 1-methyl-2-[4-(*(S)*-tetrahydrofuran-3-yloxy)-benzyl]-4-(β-D-glucopyranos-1-yl)-benzene. |

Even more preferred glucopyranosyl-substituted benzene derivatives of the formula (I) are selected from the compounds (I.8), (I.9) and (1.11).

Even more preferred glucopyranosyl-substituted benzene derivatives of the formula (I) are selected from the compounds (I.8) and (I.9).

According to this invention, it is to be understood that the definitions of the above listed glucopyranosyl-substituted benzene derivatives of the formula (I) also comprise their hydrates, solvates and polymorphic forms thereof, and prodrugs thereof. With regard to the preferred compound (I.8), an advantageous crystalline form is described in the international patent application WO 2006/117360 which hereby is incorporated herein in its entirety. With regard to the preferred compound (I.9) an advantageous crystalline form is described in the international patent applciation WO 2006/117359 which hereby is incorporated herein in its entirety. With regard to the preferred compound (i.11) an advantageous crystalline form is described in the international patent applciation WO 2008/049923 which hereby is incorporated herein in its entirety. These crystalline forms possess good solubility properties which enable a good bioavailability of the SGLT2 inhibitor. Furthermore, the crystalline forms are physico-chemically stable and thus provide a good shelf-life stability of the pharmaceutical composition.

For avoidance of any doubt, the disclosure of each of the foregoing documents cited above in connection with the specified SGLT2 inhibitors is specifically incorporated herein by reference in its entirety.

A preferred crystalline form (L9X) of the compound (I.9) can be characterized by an X-ray powder diffraction pattern that comprises peaks at 18.84, 20.36 and 25.21 degrees 2Θ (±0.1 degrees 2Θ), wherein said X-ray powder diffraction pattern (XRPD) is made using $CuK_{\alpha 1}$ radiation.

In particular said X-ray powder diffraction pattern comprises peaks at 14.69, 18.84, 19.16, 19.50, 20.36 and 25.21 degrees 2Θ (±0.1 degrees 2Θ), wherein said X-ray powder diffraction pattern is made using $CuK_{\alpha 1}$ radiation.

In particular said X-ray powder diffraction pattern comprises peaks at 14.69, 17.95, 18.43, 18.84, 19.16, 19.50, 20.36, 22.71, 23.44, 24.81, 25.21 and 25.65 degrees 2Θ (±0.1 degrees 2Θ), wherein said X-ray powder diffraction pattern is made using $CuK_{\alpha 1}$ radiation.

More specifically, the crystalline form (L9X) is characterised by an X-ray powder diffraction pattern, made using $CuK_{\alpha 1}$ radiation, which comprises peaks at degrees 2Θ (±0.1 degrees 2Θ) as contained in Table 1. Particularly characteristic are peaks with a relative intensity $I/I_0$ above 20.

**Table 1**: X-ray powder diffraction pattern of the crystalline form (L9X) (only peaks up to 30° in 2 Θ are listed):

| 2 Θ [°] | d-value [Å] | Intensity $I/I_0$ [%] |
|---|---|---|
| 4.46 | 19.80 | 8 |
| 9.83 | 8.99 | 4 |
| 11.68 | 7.57 | 4 |
| 13.35 | 6.63 | 14 |
| 14.69 | 6.03 | 42 |
| 15.73 | 5.63 | 16 |
| 16.20 | 5.47 | 8 |
| 17.95 | 4.94 | 30 |
| 18.31 | 4.84 | 22 |
| 18.43 | 4.81 | 23 |
| 18.84 | 4.71 | 100 |
| 19.16 | 4.63 | 42 |
| 19.50 | 4.55 | 31 |
| 20.36 | 4.36 | 74 |
| 20.55 | 4.32 | 13 |
| 21.18 | 4.19 | 11 |
| 21.46 | 4.14 | 13 |
| 22.09 | 4.02 | 19 |
| 22.22 | 4.00 | 4 |
| 22.71 | 3.91 | 28 |
| 23.44 | 3.79 | 27 |
| 23.72 | 3.75 | 3 |
| 24.09 | 3.69 | 3 |
| 24.33 | 3.66 | 7 |
| 24.81 | 3.59 | 24 |
| 25.21 | 3.53 | 46 |
| 25.65 | 3.47 | 23 |
| 26.40 | 3.37 | 2 |
| 26.85 | 3.32 | 8 |
| 27.26 | 3.27 | 17 |
| 27.89 | 3.20 | 2 |
| 28.24 | 3.16 | 3 |

(continued)

| 2 Θ [°] | d-value [Å] | Intensity $I/I_0$ [%] |
|---|---|---|
| 29.01 | 3.08 | 4 |
| 29.41 | 3.03 | 18 |

Even more specifically, the crystalline form (L9X) is characterised by an X-ray powder diffraction pattern, made using $CuK_{\alpha 1}$ radiation, which comprises peaks at degrees 2Θ (±0.1 degrees 2Θ) as shown in Figure 1.

Furthermore the crystalline form (L9X) is characterised by a melting point of about 149°C ± 5°C (determined via DSC; evaluated as onset-temperature; heating rate 10 K/min).The obtained DSC curve is shown in Figure 2.

The X-ray powder diffraction patterns are recorded, within the scope of the present invention, using a STOE - STADI P-diffractometer in transmission mode fitted with a location-sensitive detector (OED) and a Cu-anode as X-ray source (CuKα1 radiation, λ = 1,54056 Å, 40kV, 40mA). In the Table 1 above the values "2Θ [°]" denote the angle of diffraction in degrees and the values "d [Å]" denote the specified distances in Å between the lattice planes. The intensity shown in the Figure 1 is given in units of cps (counts per second).

In order to allow for experimental error, the above described 2Θ values should be considered accurate to ± 0.1 degrees 2Θ, in particular ± 0.05 degrees 2Θ. That is to say, when assessing whether a given sample of crystals of the compound (I.9) is the crystalline form in accordance with the invention, a 2Θ value which is experimentally observed for the sample should be considered identical with a characteristic value described above if it falls within ± 0.1 degrees 2Θ of the characteristic value, in particular if it falls within ± 0.05 degrees 2Θ of the characteristic value.

The melting point is determined by DSC (Differential Scanning Calorimetry) using a DSC 821 (Mettler Toledo).

In one embodiment, a pharmaceutical composition or dosage form according to the present invention comprises the compound (I.9), wherein at least 50 % by weight of the compound (I.9) is in the form of its crystalline form (I.9X) as defined hereinbefore. Preferably in said composition or dosage form at least 80 % by weight, more preferably at least 90 % by weight of the compound (I.9) is in the form of its crystalline form (I.9X) as defined hereinbefore.

Regarding the active pharmaceutical ingredients it can be found that the dissolution properties of the pharmaceutical composition and dosage form is affected *inter alia* by the particle size and particle size distribution of the respective active pharmaceutical ingredient. In the pharmaceutical composition and pharmaceutical dosage form according to the invention the active pharmaceutical ingredients preferably have a particle size distribution such that at least 90 % of the respective active pharmaceutical ingredient particles, with regard to the distribution by volume, has a particle size smaller than 200 μm, i.e. X90 < 200 μm.

In particular, with regard to the glucopyranosyl-substituted benzene derivative of the formula (I), in particular the compound (I.9) or its crystalline form (I.9X), it was found that the particle size, in particular the particle size and the particle size distribution, influence the manufacturability, in particular that too small particles, especially too many small particles, (for example so called "fines", i.e. particles which are smaller than 63 μm) influence the manufacturability by sticking or filming during tabletting. On the other hand too large particles negatively affect the dissolution properties of the pharmaceutical composition and dosage form and thus the bioavailability. In the following preferred ranges of the particle size distribution are described.

Therefore, in one aspect, in the pharmaceutical composition and pharmaceutical dosage form according to the invention the glucopyranosyl-substituted benzene derivative of the formula (I), in particular the compound (I.9), preferably its crystalline form (I.9X), preferably has a particle size distribution (by volume) such that at least 90 % of the respective active pharmaceutical ingredient has a particle size smaller than 200 μm, i.e. X90 < 200 μm, preferably X90 ≤ 150 μm. More preferably the particle size distribution is such that X90 ≤ 100 μm, even more preferably X90 ≤ 90 μm. In addition the particle size distribution is preferably such that X90 ≥ 1 μm, more preferably X90 ≥ 5 μm, even more preferably X90 ≥ 10 μm. Therefore preferred particle size distributions are such that 1 μm ≤ X90 < 200 μm, particularly 1 μm ≤ X90 ≤ 150 μm, more preferably 5 μm ≤ X90 ≤ 150 μm, even more preferably 5 μm ≤ X90 ≤ 100 μm, even more preferably 10 μm ≤ X90 ≤ 100 μm. A preferred example X90 ≤ 75 μm. Another preferred example is 20 μm ≤ X90 ≤ 50 μm. Another particle size according to the present invention is 10 μm ≤ X90 ≤ 75 μm. Another particle size according to the present invention is 60 μm ≤ X90 ≤ 150 μm.

Furthermore in the pharmaceutical composition and pharmaceutical dosage form according to the invention the glucopyranosyl-substituted benzene derivative of the formula (I), in particular the compound (I.9), preferably its crystalline form (I.9X), preferably has a particle size distribution (by volume) such that X50 ≤ 90 μm, more preferably X50 ≤ 75 μm, even more preferably X50 ≤ 50 μm, most preferably X50 ≤ 40 μm. In addition the particle size distribution is preferably such that X50 ≥ 1 μm, more preferably X50 ≥ 5 μm, even more preferably X50 ≥ 8 μm. Therefore preferred particle size distributions are such that 1 μm < X50 ≤ 90 μm, particularly 1 μm ≤ X50 ≤ 75 μm, more preferably 5 μm ≤ X50 ≤ 75 μm, even more preferably 5 μm ≤ X50 ≤ 50 μm. A preferred example is 8 μm ≤ X50 ≤ 40 μm.

Furthermore in the pharmaceutical composition and pharmaceutical dosage form according to the invention the glucopyranosyl-substituted benzene derivative of the formula (I), in particular the compound (I.9), preferably its crystalline form (I.9X), preferably has a particle size distribution (by volume) such that X10 ≥ 0.1 μm, more preferably X10 ≥ 0.5 μm, even more preferably X10 ≥ 1 μm, in particular X10 ≥ 2 μm. In addition the particle size distribution is preferably such that X10 ≤ 10 μm, more preferably X10 ≤ 5 μm. Therefore preferred particle size distributions are such that 0.5 μm ≤ X10 ≤ 10 μm, particularly 1 μm ≤ X10 ≤ 5 μm.

Therefore a pharmaceutical composition or pharmaceutical dosage form according to this invention may preferably be characterized by the above specified particle size distributions X90, X50 and/or X10 or one of the following embodiments:

| Embodiment | Glucopyranosyl-substituted benzene derivative, in particular of the compound (I.9) |
|---|---|
| 1 | X90 < 200 μm |
| 2 | 1 μm ≤ X90 ≤ 150 μm |
| 3 | 5 μm ≤ X90 ≤ 150 μm |
| 4 | 10 μm ≤ X90 ≤ 100 μm |
| 5 | X90 ≤ 150 μm<br>1 μm ≤ X50 ≤ 75 μm |
| 6 | X90 ≤ 150 μm<br>5 μm ≤ X50 ≤ 50 μm |
| 7 | X90 ≤ 150 μm<br>1 μm ≤ X50 ≤ 75 μm<br>X10 ≥ 0.1 μm |
| 8 | X90 ≤ 150 μm<br>5 μm ≤ X50 ≤ 50 μm<br>X10 ≥ 0.5 μm |

The value X90 refers to the 90% value of the volume distribution measured using a laser diffractometer. In other words, for the purposes of the present invention, the X90 value denotes the particle size below which 90% of the quantity of particles is found based on the volume distribution. Analogously the value X50 refers to the 50% value (median) of the volume distribution measured using a laser diffractometer. In other words, for the purposes of the present invention, the X50 value denotes the particle size below which 50% of the quantity of particles is found based on the volume distribution. Analogously the value X10 refers to the 10% value of the volume distribution measured using a laser diffractometer. In other words, for the purposes of the present invention, the X10 value denotes the particle size below which 10% of the quantity of particles is found based on the volume distribution.

Preferably all X90, X50, X10 values hereinbefore and hereinafter are by volume and determined by laser-diffraction method, in particular low angle laser light scattering, i.e. Fraunhofer diffraction. A preferred test is described in the experimental section. The laser diffraction method is sensitive to the volume of a particle and provides a volume-average particle size, which is equivalent to the weight-average particle size if the density is constant. The skilled artesian knows that the results of the particle size distribution determination by one technique can be correlated with that from another technique, for example on an empirical basis by routine experimentation. Alternatively the particle size distribution in the pharmaceutical composition or dosage form can be determined by microscopy, in particular electron microscopy or scanning electron microscopy.

In the following the suitable excipients and carriers in the pharmaceutical compositions according to the invention are described in further detail.

A pharmaceutical composition according to the invention typically comprises one or more diluents, one or more disintegrants and optionally one or more binders. Some of the excipients may have two or more functions at the same time, e.g. act as a filler and a binder.

Suitable diluents (also referred to as fillers) according to the invention are for example, lactose, in particular lactose monohydrate, cellulose and derivatives, such as powdered cellulose, microcrystalline or silicified microcrystalline cellulose, cellulose acetate, starches and derivatives such as pregelatinized starch, corn starch, wheat starch, rice starch, potato starch, sterilizable maize, sodium chloride, calcium carbonate, calcium phosphate, particularly dibasic calcium phosphate, calcium sulphate, dicalcium or tricalcium phosphate, magnesium carbonate, magnesium oxide, sugars and derivatives such as confectioner's sugar, fructose, sucrose, dextrates, dextrin, D-sorbitol sulfobutylether β-cyclodextrin,

dextrose, polydextrose, trehalose, maltose, maltitol, mannitol, maltodextrin, sorbitol, inulin, xylitol, erythritol, isomalt, kaolin and lactitol. Preferred diluents are lactose monohydrate and microcrystalline cellulose.

Suitable disintegrants according to the invention are for example powdered cellulose, crospovidone, croscarmellose sodium, docusate sodium, low-substituted hydroxypropyl cellulose, magnesium aluminum silicate, microcrystalline cellulose, polacrilin potassium, sodium starch glycolate, starch, particularly pregelatinized starch and corn starch. A preferred disintegrant is croscarmellose sodium.

Any binder usually employed in pharmaceutical compositions may be used in the context of the instant invention. Binders are for example naturally occurring or partially or totally synthetic polymers selected from acacia, agar, alginic acid, carbomers, carmellose sodium, carrageenan, cellulose acetate phthalate, ceratonia, chitosan, confectionar's sugar, copovidone, povidone, cottonseed oil, dextrate, dextrin, dextrose, polydextrose, maltodextrin, maltose, cellulose and derivatives thereof such as microcrystalline cellulose, methylcellulose, ethylcellulose, hydroxyethyl cellulose, hydroxyethyl methylcellulose, hydroxypropyl celluloses, carboxymethylcelluloses, hypromelloses (cellulose hydroxypropyl methyl ether), starch and derivatives thereof, such as pregelatinized starch, hydroxypropylstarch, corn starch, gelatin, glyceryl behenate, tragacanth, guar gum, hydrogenated vegetable oils, inulin, poloxamer, polycarbophils, polyethylene oxide, polyvinylpyrrolidone, copolymers of N-vinylpyrrolidone and vinyl acetate, polymethacrylates, polyethylene glycols, alginates such as sodium alginate, gelatin, sucrose, sunflower oil, zein as well as derivatives and mixtures thereof. Preferred binders are microcrystalline cellulose and hydroxypropyl cellulose.

In one aspect, the amount of small particles is reduced by using a binder with fine particle size for the preparation of the pharmaceutical composition or dosage form. Accordingly, in one embodiment, a binder in a composition according to the present invention is a binder with fine particle size and the present invention provides a pharmaceutical composition comprising a glucopyranosyl-substituted benzene derivative of the formula (I), in particular the compound (I.9) or its crystalline form (I.9X), and a binder with fine particle size. In one embodiment, at least 99% of the particles of the binder (by weight) are 250 μm or smaller. In one embodiment, at least 99.5% of the particles of the binder are 250 μm or smaller. For example, the binder in a composition according to the present invention is hydroxypropyl cellulose Klucel EXF. Another example of binder with small particle size is Copovidone Kollidon VA 64 fine.

In one aspect, hydroxyproply cellulose with low viscosity is used in the present invention. Several grades of hydroxyproply cellulose with different moleculare weight values are available, e.g. 80,000, 95,000, 140,000, 370,000, 850,000 and 1,150,000. Hydroxyproply cellulose with low molecular weight has low viscosity, high molecular weight hydroxyproply cellulose results in high viscosity. For a pharmaceutical composition or dosage form of the present invention, low viscosity values for hydroxyproply cellulose are preferred. Accordingly, in one embodiment, hydroxyproply cellulose grades with a molecular weight of not more than 370,000 are used in a pharmaceutical composition or dosage form of the present invention. In another embodiment, hydroxyproply cellulose grades with a molecular weight of not more than 140,000 are used in a pharmaceutical composition or dosage form of the present invention. In another embodiment, hydroxyproply cellulose grades with molecular weight values of 80,000 or 95,000 are used in a pharmaceutical composition or dosage form of the present invention.

The pharmaceutical composition according to the present invention may also comprise one or more lubricants. Suitable lubricants according to the invention are stearic acid as well as salts thereof including talc, sodium stearate, calcium stearate, zinc stearate, magnesium stearate, sodium stearyl fumarate, glyceryl monostearate, particularly magnesium stearate, polyethylene glycols, in particular polyethylene glycol with a molecular weight in a range from about 4400 to about 9000, hydrogenated castor oil, fatty acid, for example fumaric acid, and salts of fatty acids, in particular the calcium, magnesium, sodium or pottasium salts thereof, for example calcium behenate, calcium stearate, sodium stearyl fumarate or magnesium stearate (for example (e.g. HyQual®, Mallinckrodt), glycerides such as glyceryl behenate (Compritol® 888), Dynasan® 118 or Boeson® VP.

The pharmaceutical composition according to the present invention may also comprise one or more glidants. Suitable glidants according to the invention are silicon dioxide, particularly colloidal silicon dioxide (e.g. Aerosil®, Cab-O-Sil®), stearic acid as well as salts thereof including sodium stearate, calcium stearate, zinc stearate, magnesium stearate, magnesium silicate, calcium silicate, magnesium trisilicate and talc. Preferred glidants are colloidal silicon dioxide and talc.

In another embodiment, a pharmaceutical composition according to the instant invention comprises

| | Amount (% by weight) |
|---|---|
| Active ingredient | 0.5 - 25 |
| One or more diluents | 65 - 93 |
| One or more binders | 1 - 5 |
| One or more disintegrants | 1 - 4 |

(continued)

| | Amount (% by weight) |
|---|---|
| Optionally additional additives | ad 100 % |

In one aspect, the active ingredient is a compound of the formula (I), for example of the formula (I.9) or its crystalline form (L9X).

In another embodiment, a pharmaceutical composition according to the instant invention comprises

| | Amount (% by weight) |
|---|---|
| Active ingredient | 0.5 - 25 |
| One or more diluents | 65 - 90 |
| One or more binders | 1 - 5 |
| One or more disintegrants | 1 - 3 |
| Optionally additional additives | ad 100 % |

In one aspect, the active ingredient is a compound of the formula (I), for example of the formula (I.9) or its crystalline form (L9X).

In another embodiment, a pharmaceutical composition according to the instant invention comprises

| | Amount (% by weight) |
|---|---|
| Active ingredient | 0.5 - 17 |
| One or more diluents | 70 - 90 |
| One or more binders | 1 - 5 |
| One or more disintegrants | 1 - 4 |
| Optionally additional additives | ad 100 % |

The active ingredient is a compound of the formula (I), for example of the formula (I.9) or its crystalline form (L9X).

In another embodiment, a pharmaceutical composition according to the instant invention comprises

| | Amount (% by weight) |
|---|---|
| Active ingredient | 1 - 25 |
| One or more diluents | 69 - 93 |
| One or more binders | 1 - 3 |
| One or more disintegrants | 1 - 3 |
| Optionally additional additives | ad 100 % |

In one aspect, the active ingredient is a compound of the formula (I), for example of the formula (I.9) or its crystalline form (L9X).

In another embodiment, a pharmaceutical composition according to the instant invention comprises

| | Amount (% by weight) |
|---|---|
| Active ingredient | 0.5 - 25 |
| Lactose monohydrate | 28 - 70 |
| Microcrystalline cellulose | 20 - 50 |
| Hydroxypropyl cellulose | 1 - 5 |

(continued)

| | Amount (% by weight) |
|---|---|
| Croscarmellose sodium | 1 - 4 |
| Optionally additional additives | ad 100 % |

In one aspect, the active ingredient is a compound of the formula (I), for example of the formula (I.9) or its crystalline form (L9X).

In another embodiment, a pharmaceutical composition according to the instant invention comprises

| | Amount (% by weight) |
|---|---|
| Active ingredient | 0.5 - 25 |
| Lactose monohydrate | 35 - 90 |
| Microcrystalline cellulose | 0 - 30 |
| Hydroxypropyl cellulose | 1 - 5 |
| Croscarmellose sodium | 1 - 3 |
| Optionally additional additives | ad 100 % |

In one aspect, the active ingredient is a compound of the formula (I), for example of the formula (I.9) or its crystalline form (L9X).

In another embodiment, a pharmaceutical composition according to the instant invention comprises

| | Amount (% by weight) |
|---|---|
| Active ingredient | 0.5 - 25 |
| Lactose monohydrate | 35 - 70 |
| Microcrystalline cellulose | 20 - 40 |
| Hydroxypropyl cellulose | 1 - 5 |
| Croscarmellose sodium | 1 - 3 |
| Optionally additional additives | ad 100 % |

In one aspect, the active ingredient is a compound of the formula (I), for example of the formula (I.9) or its crystalline form (L9X).

In another embodiment, a pharmaceutical composition according to the instant invention comprises

| | Amount (% by weight) |
|---|---|
| Active ingredient | 0.5 - 17 |
| Lactose monohydrate | 28 - 60 |
| Microcrystalline cellulose | 30 - 50 |
| Hydroxypropyl cellulose | 1 - 5 |
| Croscarmellose sodium | 1 - 4 |
| Optionally additional additives | ad 100 % |

In one aspect, the active ingredient is a compound of the formula (I), for example of the formula (I.9) or its crystalline form (L9X).

In another embodiment, a pharmaceutical composition according to the instant invention comprises

| | Amount (% by weight) |
|---|---|
| Active ingredient | 1 - 25 |
| Lactose monohydrate | 39 - 63 |
| Microcrystalline cellulose | 20 - 40 |
| Hydroxypropyl cellulose | 1 - 5 |
| Croscarmellose sodium | 1 - 3 |
| Optionally additional additives | ad 100 % |

In one aspect, the active ingredient is a compound of the formula (I), for example of the formula (I.9) or its crystalline form (L9X).

In one embodiment, the ratio of said disintegrant to said binder in a pharmaceutical composition of the present invention is between 1.5:3.5 and 1:1 (weight/weight).

In one aspect, with regard to the glucopyranosyl-substituted benzene derivative of the formula (I), in particular the compound (I.9) or its crystalline form (I.9X), it was found that the amount of active ingredient influences the manufacturability of the pharmaceutical composition or dosage form, in particular that a high concentration of the active ingredient influences the manufacturability by sticking or filming during tabletting. Accordingly, in one embodiment, the active ingredient represents 25% or less of the weight of the pharmaceutical composition. In another embodiment, the active ingredient represents 20% or less, preferably 15% or less, of the weight of the pharmaceutical composition. Preferably, the active ingredient represents 0.5% to 25% of the weight of the pharmaceutical composition. More preferably, the active ingredient represents 1.0% to 20% of the weight of the pharmaceutical composition. Even more preferably, the active ingredient represents 2.0% to 15% of the weight of the pharmaceutical composition.

In the following, preferred ranges of the amount of the glucopyranosyl-substituted benzene derivative to be employed in the pharmaceutical dosage form according to this invention are described. These ranges refer to the amounts to be administered per day with respect to an adult patient, in particular to a human being, for example of approximately 70 kg body weight, and can be adapted accordingly with regard to an administration 2, 3, 4 or more times daily and with regard to other routes of administration and with regard to the age of the patient. The ranges of the dosage and amounts are calculated for the active ingredient.

A preferred amount of the glucopyranosyl-substituted benzene derivative, in particular the compound (I.9) or its crystalline form (I.9X) is in a range from 0.5 to 100 mg, preferably from 0.5 to 50 mg, even more preferably from 1 to 25 mg, even more preferably 5 to 25 mg. Preferred dosages of the glucopyranosyl-substituted benzene derivative are for example 1 mg, 2 mg, 2.5 mg, 5 mg, 7.5 mg, 10 mg, 12.5 mg, 15 mg, 20 mg, 25 mg and 50 mg.

A pharmaceutical composition according to the present invention may be comprised in a tablet, a capsule or a film-coated tablet,

In one embodiment, a tablet comprising a pharmaceutical composition according to the present invention comprises a lubricant, such as magnesium stearate. Such lubricant may be present in a concentration of 0.25 - 2 % in said tablet.

In one embodiment, a tablet comprising a pharmaceutical composition according to the present invention comprises a glidant, such as colloidal silicon dioxide. Such glidant may be present in a concentration of 0.25 - 2 % in said tablet.

A tablet according to the invention may be film-coated. Typically a film coat represents 2-5% by weight of the total composition and comprises preferably a film-forming agent, a plasticizer, an anti-tacking agent and optionally one or more pigments. An exemplary coat composition may comprise hydroxypropyl methylcellulose (HPMC), polyethylene glycol (PEG), talc, titanium dioxide and optionally iron oxide, including iron oxide red and/or yellow. For example, a film coat according ot the present invention comprises 50% hypromellose, 5% macrogol, 24.75% titanium oxide, 20% talc and 0.25% iron oxide yellow (Opadry yellow 02B38190).

In one aspect, a film coat according to the present invention comprises

| | Amount (% by weight) |
|---|---|
| Film-forming agent | 30 - 70 |
| One or more plasticizers | 1-10 |
| One or more anti-tacking agents | 5-30 |

(continued)

| | Amount (% by weight) |
|---|---|
| One or more colorants | 0-30 |
| Optionally additional additives | ad 100 % |

In one embodiment, the pharmaceutical dosage form according to the invention has dissolution properties such that after 45 minutes at least 75 %, preferably at least 80 %, preferably at least 90 % by weight of the pharmaceutical active ingredient is dissolved. In another embodiment after 30 minutes at least 75 %, preferably at least 80 %, preferably at least 90 % by weight of the pharmaceutical active ingredient is dissolved. In another embodiment after 15 minutes at least 65 %, preferably at least 75 %, preferably at least 80 %, preferably at least 90 % by weight of the pharmaceutical active ingredient is dissolved. The dissolution properties can be determined in a standard dissolution test, for example as described in pharmacopoeias, such as the USP31-NF26 S2, chapter 711 (dissolution).

In one embodiment, the pharmaceutical dosage form according to the invention has disintegration properties such that within 40 minutes, alternatively within 30 minutes, preferably within 20 minutes, more preferably within 15 minutes, even more preferably within 10 minutes, the pharmaceutical dosage form is disintegrated. The disintegration properties can be determined in a standard disintegration test, for example as described in pharmacopoeias, such as the USP31-NF26 S2, chapter 701 (disintegration).

In one embodiment, the pharmaceutical dosage form according to the invention has a high content uniformity, preferably within a range from 85 to 115 %, more preferably from 90 to 110 %, even more preferably from 95 to 105 % by weight with regard to the pharmaceutical ingredient. The content uniformity can be determined in a standard test using for example randomly 10 selected pharmaceutical dosage forms, for example as described in pharmacopoeias.

A dosage form according to this invention, such as a tablet, capsule or film-coated tablet, may be prepared by methods well-known to the one skilled in the art.

Suitable methods of manufacturing a tablet include compression of the pharmaceutical composition in the form of a powder, i.e. direct compression, or compression of the pharmaceutical composition in the form of granules, and if needed with additional excipients.

Granules of the pharmaceutical composition according to the invention may be prepared by methods well-known to the one skilled in the art. Preferred methods for the granulation of the active ingredients together with the excipients include wet granulation, for example high shear wet granulation and fluidized bed wet granulation, dry granulation, also called roller compaction.

In the wet granulation process the granulation liquid are the solvent alone or a preparation of one or more binders in a solvent or mixture of solvents. Suitable binders are described hereinbefore. Examples are hypromellose, hydroxypropyl cellulose, povidone and copovidone. Suitable solvents are for example purified water, ethanol, methanol, isopropanol, acetone, preferably purified water, including mixtures thereof. The solvent is a volatile component, which does not remain in the final product. The one or more active ingredients and the other excipients, in particular the one or more diluents and the one or more disintegrants, usually with exception of the lubricant, are premixed and granulated with the granulation liquid, for example using a high shear granulator. The wet granulation step is usually followed by one or more drying and sieving steps. For example a drying oven or a fluid bed dryer can then be used for drying.

The dried granules are sieved through an appropriate sieve. After optional addition of the other excipients, in particular disintegrant, binder, filler and/or glidant, with exception of the lubricant the mixture is blended in a suitable blender, for example a free fall blender, followed by addition of the one or more lubricants, for example magnesium stearate, and final blending in the blender.

An exemplary wet granulation process for making a pharmaceutical composition according to the instant invention comprises the steps of:

(1) Premixing the active ingredient and the main portion of the excipients including the binder in a mixer to obtain a pre-mixture;
(2) granulating the pre-mixture of step (1) by adding the granulation liquid, preferably purified water;
(3) drying the granules of step (2) in a fluidized bed dryer or a drying oven;
(4) optionally dry sieving of the dried granules of step (3);
(5) mixing the dried granules of step (4) with the remaining excipients like filler (also referred to as diluents), binder, disintegrant and/or glidant in a mixer to obtain the main mixture;
(6) mixing the main mixture of step (5) with the lubricant in a mixer to obtain the final mixture;
(7) tableting the final mixture of step (6) by compressing it on a suitable tablet press to produce tablets cores;
(8) optionally film-coating of the tablet cores of step (7) with a non-functional coat.

In one aspect, it was found that providing a portion of a diluent after wet granulation, for example as a dry add, reduces the sticking and/or filming during the manufacture of the pharmaceutical composition or dosage form. Adding additional diluents after wet granulation can also improve the physical stability of the dosage form (tablet hardness). Accordingly, in one embodiment, in a wet granulation process according to the present invention a diluent is added after wet granulation, for example as a dry add, such as for example in step (5) above. In one embodiment, the amount of diluent added after wet granulation, for example as a dry add, such as for example in step (5) above, is 1% to 20% of the weight of a tablet (without film coating), preferably 2.5% to 10% of the weight of a tablet (without film coating). Such diluent is for example microcrystalline cellulose. Such diluent may be added in step (1) and in step (5) above.
In one aspect, a pharmaceutical composition according to the present invention is produced by high shear wet granulation.
The present invention also provides a pharmaceutical composition obtainable by a process above.
An exemplary direct compression process according to the present invention for making a pharmaceutical composition comprises the steps of:

(1) Premixing the active ingredient and the main portion of the excipients in a mixer to obtain a pre-mixture;
(2) optionally dry screening the pre-mixture through a screen in order to segregate cohesive particles and to improve content uniformity;
(3) mixing the pre-mixture of step (1) or (2) in a mixer, optionally by adding remaining excipients to the mixture and continuing mixing;
(4) tableting the final mixture of step (3) by compressing it on a suitable tablet press to produce the tablet cores;
(5) optionally film-coating of the tablet cores of step (4) with a non-functional coat.

The present invention also provides a pharmaceutical composition obtainable by the above process.
An exemplary dry granulation process according to the present invention for making a pharmaceutical composition comprises the steps of:

(1) mixing the active ingredient with either all or a portion of the excipients in a mixer;
(2) compaction of the mixture of step (1) on a suitable roller compactor;
(3) reducing the ribbons obtained during step (2) to granules, preferably small granules, by suitable milling or sieving steps;
(4) optionally mixing the granules of step (3) with the remaining excipients in a mixer to obtain the final mixture;
(5) tabletting the granules of step (3) or the final mixture of step (4) by compressing it on a suitable tablet press to produce the tablet cores;
(6) optionally film-coating of the tablet cores of step (5) with a non-functional coat.

In one embodiment, the size of the granules according to the present invention is in the range from 25 to 800 μm, for example from 40 μm to 500 μm. The size of the granules may be measured via sieve analysis, for example with a sonic sifter. In one embodiment, at least 80 %, at least 90 %, or at least 95 % by weight of the granules is in the given range.
In one embodiment, a pharmaceutical composition or dosage form according to the present invention exhibits a distinctive pharmacokinetic profile after administration to a subject, in particular after administration to a human, as for example described hereinbelow.
Accordingly, in one embodiment, a pharmaceutical composition according to the present invention when administered to a fasting human at a dose of 2.5 mg exhibits a $C_{max}$ of 40.3 to 96.3 nmol/L; and a AUC of 283 to 677 nmol*h/L.
In one embodiment, a pharmaceutical composition according to the present invention when administered to a fasting human at a dose of 2.5 mg exhibits a geometric mean $C_{max}$ of 52.9 to 66.6 nmol/L; and a geometric mean AUC of 394 to 468 nmol*h/L.
In one embodiment, a pharmaceutical composition according to the present invention when administered to a fasting human at a dose of 5.0 mg exhibits a $C_{max}$ of 123 to 230 nmol/L; and a $AUC_{0\text{-}inf}$ of 1,000 to 1,310 nmol*h/L.
In one embodiment, a pharmaceutical composition according to the present invention when administered to a fasting human at a dose of 10.0 mg exhibits a $C_{max}$ of 143 to 796 nmol/L; and a AUC of 1,170 to 3,190 nmol*h/L.
In one embodiment, a pharmaceutical composition according to the present invention when administered to a fasting human at a dose of 10.0 mg exhibits a geometric mean $C_{max}$ of 221 to 372 nmol/L; and a geometric mean AUC of 1,690 to 2,660 nmol*h/L.
In one embodiment, a pharmaceutical composition according to the present invention when administered to a fasting human at a dose of 25.0 mg exhibits a $C_{max}$ of 334 to 1,030 nmol/L; and a AUC of 2,660 to 7,640 nmol*h/L.
In one embodiment, a pharmaceutical composition according to the present invention when administered to a fasting human at a dose of 25.0 mg exhibits a geometric mean $C_{max}$ of 490 to 709 nmol/L; and a geometric meanAUC of 3,750 to 6,130 nmol*h/L.

In one embodiment, a pharmaceutical composition according to the present invention when administered to a fasting human at a dose of 50.0 mg exhibits a $C_{max}$ of 722 to 2,020 nmol/L; and a AUC of 6,450 to 14,100 nmol*h/L.

In one embodiment, a pharmaceutical composition according to the present invention when administered to a fasting human at a dose of 50.0 mg exhibits a geometric mean $C_{max}$ of 1,080 to 1,140 nmol/L; and a geometric mean $AUC_{0\text{-}inf}$ of 8,310 to 8,460 nmol*h/L.

In one embodiment, a pharmaceutical composition according to the present invention when administered to a fasting human:

a) at a dose of 2.5 mg exhibits:

i. $C_{max}$ of 40.3 to 96.3 nmol/L; and
ii. AUC of 283 to 677 nmol*h/L; and/or

b) at a dose of 5.0 mg exhibits:

i. $C_{max}$ of 123 to 230 nmol/L; and
ii. AUC of 1,000 to 1,310 nmol*h/L; and/or

c) at a dose of 10.0 mg exhibits:

i. $C_{max}$ of 143 to 796 nmol/L; and
ii. AUCof 1,170 to 3,190 nmol*h/L; and/or

d) at a dose of 25.0 mg exhibits:

i. $C_{max}$ of 334 to 1,030 nmol/L; and
ii. AUC of 2,660 to 7,640 nmol*h/L; and/or

e) at a dose of 50.0 mg exhibits:

i. $C_{max}$ of 722 to 2,020 nmol/L; and
ii. AUCof 6,450 to 14,100 nmol*h/L.

In one embodiment, a pharmaceutical composition according to the present invention when administered to a fasting human:

a. at a dose of 2.5 mg exhibits:

iii. a geometric mean $C_{max}$ of 52.9 to 66.6 nmol/L; and
iv. a geometric mean AUC of 394 to 468 nmol*h/L; and/or

b. at a dose of 10.0 mg exhibits:

i. a geometric mean $C_{max}$ of 221 to 372 nmol/L; and
ii. a geometric mean $AUC_f$ of 1,690 to 2,660 nmol*h/L; and/or

c. at a dose of 25.0 mg exhibits:

i. a geometric mean $C_{max}$ of 490 to 709 nmol/L; and
ii. a geometric mean AUC of 3,750 to 6,130 nmol*h/L; and/or

d. at a dose of 50.0 mg exhibits:

i. a geometric mean $C_{max}$ of 1,080 to 1,140 nmol/L; and
ii. a geometric mean AUC of 8,310 to 8,460 nmol*h/L.

In one embodiment, a pharmaceutical composition according to the present invention when administered to a fasting human as a single dose of 2.5 mg exhibits a $C_{max}$ of 42.8 to 81.2 nmol/L; and a $AUC_{0\text{-}inf}$ of 326 to 631 nmol*h/L.

In one embodiment, a pharmaceutical composition according to the present invention when administered to a fasting human as a single dose of 2.5 mg exhibits a geometric mean $C_{max}$ of 52.9 to 61.3 nmol/L; and a geometric mean $AUC_{0-inf}$ of 394 to 468 nmol*h/L.

In one embodiment, a pharmaceutical composition according to the present invention when administered to a fasting human as a single dose of 5.0 mg exhibits a $C_{max}$ of 123 to 230 nmol/L; and a $AUC_{0-inf}$ of 1,000 to 1,310 nmol*h/L.

In one embodiment, a pharmaceutical composition according to the present invention when administered to a fasting human as a single dose of 10.0 mg exhibits a $C_{max}$ of 143 to 796 nmol/L; and a $AUC_{0-inf}$ of 1,170 to 3,190 nmol*h/L.

In one embodiment, a pharmaceutical composition according to the present invention when administered to a fasting human as a single dose of 10.0 mg exhibits a geometric mean $C_{max}$ of 221 to 372 nmol/L; and a geometric mean $AUC_{0-inf}$ of 1,690 to 2,660 nmol*h/L.

In one embodiment, a pharmaceutical composition according to the present invention when administered to a fasting human as a single dose of 25.0 mg exhibits a $C_{max}$ of 334 to 1,030 nmol/L; and a $AUC_{0-inf}$ of 2,660 to 7,170 nmol*h/L.

In one embodiment, a pharmaceutical composition according to the present invention when administered to a fasting human as a single dose of 25.0 mg exhibits a geometric mean $C_{max}$ of 490 to 709 nmol/L; and a geometric mean $AUC_{0-inf}$ of 3,750 to 6,130 nmol*h/L.

In one embodiment, a pharmaceutical composition according to the present invention when administered to a fasting human as a single dose of 50.0 mg exhibits a $C_{max}$ of 722 to 2,020 nmol/L; and a $AUC_{0-inf}$ of 6,450 to 14,100 nmol*h/L.

In one embodiment, a pharmaceutical composition according to the present invention when administered to a fasting human as a single dose of 50.0 mg exhibits a geometric mean $C_{max}$ of 1,080 to 1,140 nmol/L; and a geometric mean $AUC_{0-inf}$ of 8,310 to 8,460 nmol*h/L.

In one embodiment, a pharmaceutical composition according to the present invention when administered to a fasting human as:

a) a single dose of 2.5 mg exhibits:

i. $C_{max}$ of 42.8 to 81.2 nmol/L; and
ii. $AUC_{0-inf}$ of 326 to 631 nmol*h/L; and/or

b) a single dose of 5.0 mg exhibits:

i. $C_{max}$ of 123 to 230 nmol/L; and
ii. $AUC_{0-inf}$ of 1,000 to 1,310 nmol*h/L; or

c) a single dose of 10.0 mg exhibits:

i. $C_{max}$ of 143 to 796 nmol/L; and
ii. $AUC_{0-inf}$ of 1,170 to 3,190 nmol*h/L; and/or

d) a single dose of 25.0 mg exhibits:

i. $C_{max}$ of 334 to 1,030 nmol/L; and
ii. $AUC_{0-inf}$ of 2,660 to 7,170 nmol*h/L; and/or

e) a single dose of 50.0 mg exhibits:

i. $C_{max}$ of 722 to 2,020 nmol/L; and
ii. $AUC_{0-inf}$ of 6,450 to 14,100 nmol*h/L.

In one embodiment, a pharmaceutical composition according to the present invention when administered to a fasting human as:

a. a single dose of 2.5 mg exhibits:

j. a geometric mean $C_{max}$ of 52.9 to 61.3 nmol/L; and
ii. a geometric mean $AUC_{0-inf}$ of 394 to 468 nmol*h/L; and/or
b. a single dose of 10.0 mg exhibits:

i. a geometric mean $C_{max}$ of 221 to 372 nmol/L; and
ii. a geometric mean $AUC_{0-inf}$ of 1,690 to 2,660 nmol*h/L; and/or

c. a single dose of 25.0 mg exhibits:

i. a geometric mean $C_{max}$ of 490 to 709 nmol/L; and
ii. a geometric mean $AUC_{0-inf}$ of 3,750 to 6,130 nmol*h/L; and/or

d. a single dose of 50.0 mg exhibits:

i. a geometric mean $C_{max}$ of 1,080 to 1,140 nmol/L; and
ii. a geometric mean $AUC_{0-inf}$ of 8,310 to 8,460 nmol*h/L.

In one embodiment, a pharmaceutical composition according to the present invention when administered to a fasting human in multiple doses of 2.5 mg exhibits a $C_{max,ss}$ of 40.3 to 96.3 nmol/L; and a $AUC_{\tau,ss}$ of 283 to 677 nmol*h/L.

In one embodiment, a pharmaceutical composition according to the present invention when administered to a fasting human in multiple doses of 10.0 mg exhibits a $C_{max,ss}$ of 166 to 479 nmol/L; and a $AUC_{\tau,ss}$ of 1,350 to 2,600 nmol*h/L.

In one embodiment, a pharmaceutical composition according to the present invention when administered to a fasting human in multiple doses of 10.0 mg exhibits a geometric mean $C_{max,ss}$ of 252 to 272 nmol/L; and a geometric mean $AUC_{\tau,ss}$ of 1,850 to 2,000 nmol*h/L.

In one embodiment, a pharmaceutical composition according to the present invention when administered to a fasting human in multiple doses of 25.0 mg exhibits a $C_{max,ss}$ of 443 to 907 nmol/L; and a $AUC_{\tau,ss\ f}$ of 2,790 to 7,640 nmol*h/L.

In one embodiment, a pharmaceutical composition according to the present invention when administered to a fasting human in multiple doses of 25.0 mg exhibits a geometric mean $C_{max,ss}$ of 622 to 676 nmol/L; and a geometric mean $AUC_{\tau,ss}$ of 4,640 to 4,890 nmol*h/L.

In one embodiment, a pharmaceutical composition according to the present invention when administered to a fasting human:

a) in multiple doses of 2.5 mg exhibits:

i. $C_{max,ss}$ of 40.3 to 96.3 nmol/L; and
ii. $AUC_{\tau,ss}$ of 283 to 677 nmol*h/L; and/or

b) in multiple doses of 10.0 mg exhibits:

i. $C_{max,ss}$ of 166 to 479 nmol/L; and
ii. $AUC_{\tau,ss}$ of 1,350 to 2,600 nmol*h/L; and/or

c) in multiple doses of 25.0 mg exhibits:

i. $C_{max,ss}$ of 443to 907 nmol/L; and
ii. $AUC_{\tau,ss}$ of 2,790 to 7,640 nmol*h/L.

In one embodiment, a pharmaceutical composition according to the present invention when administered to a fasting human:

a. in multiple doses of 10.0 mg exhibits:

i. a geometric mean $C_{max,ss}$ of 252 to 272 nmol/L; and
ii. a geometric mean $AUC_{\tau,ss}$ of 1,850 to 2,000 nmol*h/L; and/or

b. in multiple doses of 25.0 mg exhibits:

i. a geometric mean $C_{max,ss}$ of 622 to 676 nmol/L; and
ii, a geometric mean $AUC_{\tau,ss}$ of 4,640 to 4,890 nmol*h/L.

In another embodiment, a pharmaceutical composition according to the present invention when administered to a fasting human exhibits a dose-normalized $C_{max, norm}$ of 13 to 80 nmol/L/mg; and a dose-normalized $AUC_{0-inf, norm}$ of 106 to 306 nmol*h/L/mg. In one embodiment, said pharmaceutical composition exhibits said dose-normalized $C_{max, norm}$ and said dose-normalized $AUC_{0-inf, norm}$ over a dose range of 2.5 mg to 50 mg of active ingredient.

In one embodiment, a pharmaceutical composition according to the present invention when administered to a fasting human exhibits a dose-normalized $C_{max, norm}$ of 13 to 80 nmol/L/mg; and a dose-normalized $AUC_{0-inf, norm}$ of 106 to 306 nmol*h/L/mg over a dose range of 5 mg to 25 mg of active ingredient.

In one embodiment, a pharmaceutical composition according to the present invention when administered to a fasting human exhibits a dose-normalized geometric mean $C_{max, norm}$ of 20 to 37 nmol/L/mg; and a dose-normalized geometric mean $AUC_{0-inf, norm}$ of 150 to 266 nmol*h/L/mg. In one embodiment, said pharmaceutical composition exhibits said dose-normalized geometric mean $C_{max, norm}$ and said dose-normalized geometric mean $AUC_{0-inf, norm}$ over a dose range of 2.5 mg to 50 mg of active ingredient.

In one embodiment, a pharmaceutical composition according to the present invention when administered to a fasting human exhibits a dose-normalized geometric mean $C_{max, norm}$ of 20 to 37 nmol/L/mg; and a dose-normalized geometric mean $AUC_{0-inf, norm}$ of 150 to 266 nmol*h/L/mg over a dose range of 5 mg to 25 mg of active ingredient.

In one embodiment, a pharmaceutical composition according to the present invention when administered to a fasting human as a single dose exhibits a dose-normalized $C_{max, norm}$ of 13 to 80 nmol/L/mg; and a dose-normalized $AUC_{0-inf, norm}$ of 106 to 287 nmol*h/L/mg. In one embodiment, said pharmaceutical composition exhibits said dose-normalized $C_{max, norm}$ and said dose-normalized $AUC_{0-inf, norm}$ over a dose range of 2.5 mg to 50 mg of active ingredient when administered to a fasting human as a single dose.

In one embodiment, a pharmaceutical composition according to the present invention when administered to a fasting human as a single dose exhibits a dose-normalized $C_{max, norm}$ of 13 to 80 nmol/L/mg; and a dose-normalized $AUC_{0-inf, norm}$ of 106 to 287 nmol*h/L/mg over a dose range of 5 mg to 25 mg of active ingredient.

In one embodiment, a pharmaceutical composition according to the present invention when administered to a fasting human as a single dose exhibits a dose-normalized geometric mean $C_{max, norm}$ of 20 to 37 nmol/L/mg; and a dose-normalized geometric mean $AUC_{0-inf, norm}$ of 150 to 266 nmol*h/L/mg. In one embodiment, said pharmaceutical composition exhibits said dose-normalized geometric mean $C_{max, norm}$ and said dose-normalized geometric mean $AUC_{0-inf, norm}$ over a dose range of 2.5 mg to 50 mg of active ingredient when administered to a fasting human as a single dose.

In one embodiment, a pharmaceutical composition according to the present invention when administered to a fasting human as a single dose exhibits a dose-normalized geometric mean $C_{max, norm}$ of 20 to 37 nmol/L/mg; and a dose-normalized geometric mean $AUC_{0-inf, norm}$ of 150 to 266 nmol*h/L/mg over a dose range of 5 mg to 25 mg of active ingredient.

In one embodiment, a pharmaceutical composition according to the present invention when administered to a fasting human in multiple doses exhibits a dose-normalized $C_{max,ss, norm}$ of 16 to 48 nmol/L/mg; and a dose-normalized $AUC_{\tau,ss, norm}$ of 112 to 306 nmol*h/L/mg. In one embodiment, said pharmaceutical composition exhibits said dose-normalized $C_{max,ss, norm}$ and said dose-normalized $AUC_{\tau,ss, norm}$ over a dose range of 2.5 mg to 25 mg of active ingredient when administered to a fasting human in multiple doses.

In one embodiment, a pharmaceutical composition according to the present invention when administered to a fasting human in multiple doses exhibits a dose-normalized geometric mean $C_{max,ss, norm}$ of 25 to 27 nmol/L/mg; and a dose-normalized geometic mean $AUC_{\tau,ss, norm}$ of 184 to 200 nmol*h/L/mg. In one embodiment, said pharmaceutical composition exhibits said dose-normalized geometric mean $C_{max,ss, norm}$ and said dose-normalized geometric mean $AUC_{\tau,ss, norm}$ over a dose range of 2.5 mg to 25 mg of active ingredient when administered to a fasting human in multiple doses.

When this invention refers to patients requiring treatment or prevention, it relates primarily to treatment and prevention in humans, but the pharmaceutical composition may also be used accordingly in veterinary medicine in mammals. In the scope of this invention adult patients are preferably humans of the age of 18 years or older.

As described hereinbefore by the administration of the pharmaceutical composition according to this invention and in particular in view of the high SGLT2 inhibitory activity of the SGLT2 inhibitors therein, excessive blood glucose is excreted through the urine of the patient, so that no gain in weight or even a reduction in body weight may result. Therefore, a treatment or prophylaxis according to this invention is advantageously suitable in those patients in need of such treatment or prophylaxis who are diagnosed of one or more of the conditions selected from the group consisting of overweight and obesity, in particular class I obesity, class II obesity, class III obesity, visceral obesity and abdominal obesity. In addition a treatment or prophylaxis according to this invention is advantageously suitable in those patients in which a weight increase is contraindicated. The pharmaceutical composition as well as the methods according to the present invention allow a reduction of the HbA1c value to a desired target range, for example < 7 % and preferably < 6.5 %, for a higher number of patients and for a longer time of therapeutic treatment compared with a corresponding monotherapy or a therapy using only two of the combination partners.

The pharmaceutical composition according to this invention and in particular the SGLT2 inhibitor therein exhibits a very good efficacy with regard to glycemic control, in particular in view of a reduction of fasting plasma glucose, postprandial plasma glucose and/or glycosylated hemoglobin (HbA1c). By administering a pharmaceutical composition according to this invention, a reduction of HbA1c equal to or greater than preferably 0.5 %, even more preferably equal to or greater than 1.0 % can be achieved and the reduction is particularly in the range from 1.0 % to 2.0 %.

Furthermore, the method and/or use according to this invention is advantageously applicable in those patients who show one, two or more of the following conditions:

(a) a fasting blood glucose or serum glucose concentration greater than 110 mg/dL, in particular greater than 125 mg/dL;
(b) a postprandial plasma glucose equal to or greater than 140 mg/dL;
(c) an HbA1c value equal to or greater than 6.5 %, in particular equal to or greater than 7.0 %, especially equal to or greater than 7.5 %, even more particularly equal to or greater than 8.0 %.

The present invention also discloses the use of the pharmaceutical composition for improving glycemic control in patients having type 2 diabetes or showing first signs of pre-diabetes. Thus, the invention also includes diabetes prevention. If therefore a pharmaceutical composition according to this invention is used to improve the glycemic control as soon as one of the above-mentioned signs of pre-diabetes is present, the onset of manifest type 2 diabetes mellitus can be delayed or prevented.

Furthermore, the pharmaceutical composition according to this invention is particularly suitable in the treatment of patients with insulin dependency, i.e. in patients who are treated or otherwise would be treated or need treatment with an insulin or a derivative of insulin or a substitute of insulin or a formulation comprising an insulin or a derivative or substitute thereof. These patients include patients with diabetes type 2 and patients with diabetes type 1.

Therefore, according to a preferred embodiment of the present invention, there is provided a method for improving glycemic control and/or for reducing of fasting plasma glucose, of postprandial plasma glucose and/or of glycosylated hemoglobin HbA1c in a patient in need thereof who is diagnosed with impaired glucose tolerance (IGT), impaired fasting blood glucose (IFG) with insulin resistance, with metabolic syndrome and/or with type 2 or type 1 diabetes mellitus characterized in that an SGLT2 inhibitor as defined hereinbefore and hereinafter is administered to the patient.

According to another preferred embodiment of the present invention, there is provided a method for improving gycemic control in patients, in particular in adult patients, with type 2 diabetes mellitus as an adjunct to diet and exercise.

It can be found that by using a pharmaceutical composition according to this invention, an improvement of the glycemic control can be achieved even in those patients who have insufficient glycemic control in particular despite treatment with an antidiabetic drug, for example despite maximal recommended or tolerated dose of oral monotherapy with metformin. A maximal recommended dose with regard to metformin is for example 2000 mg per day or 850 mg three times a day or any equivalent thereof.

Therefore, the method and/or use according to this invention is advantageously applicable in those patients who show one, two or more of the following conditions:

(a) insufficient glycemic control with diet and exercise alone;
(b) insufficient glycemic control despite oral monotherapy with metformin, in particular despite oral monotherapy at a maximal tolerated dose of metformin;
(c) insufficient glycemic control despite oral monotherapy with another antidiabetic agent, in particular despite oral monotherapy at a maximal tolerated dose of the other antidiabetic agent.

The lowering of the blood glucose level by the administration of an SGLT2 inhibitor according to this invention is insulin-independent. Therefore, a pharmaceutical composition according to this invention is particularly suitable in the treatment of patients who are diagnosed having one or more of the following conditions

- insulin resistance,
- hyperinsulinemia,
- pre-diabetes,
- type 2 diabetes mellitus, particular having a late stage type 2 diabetes mellitus,
- type 1 diabetes mellitus.

Furthermore, a pharmaceutical composition according to this invention is particularly suitable in the treatment of patients who are diagnosed having one or more of the following conditions

(a) obesity (including class I, II and/or III obesity), visceral obesity and/or abdominal obesity,

(b) triglyceride blood level ≥ 150 mg/dL,
(c) HDL-cholesterol blood level < 40 mg/dL in female patients and < 50 mg/dL in male patients,
(d) a systolic blood pressure ≥ 130 mm Hg and a diastolic blood pressure ≥ 85 mm Hg,
(e) a fasting blood glucose level ≥ 110 mg/dL.

It is assumed that patients diagnosed with impaired glucose tolerance (IGT), impaired fasting blood glucose (IFG), with insulin resistance and/or with metabolic syndrome suffer from an increased risk of developing a cardiovascular disease, such as for example myocardial infarction, coronary heart disease, heart insufficiency, thromboembolic events. A glycemic control according to this invention may result in a reduction of the cardiovascular risks.

A pharmaceutical composition according to this invention exhibits a good safety profile. Therefore, a treatment or prophylaxis according to this invention is advantageously possible in those patients for which the mono-therapy with another antidiabetic drug, such as for example metformin, is contraindicated and/or who have an intolerance against such drugs at therapeutic doses. In particular, a treatment or prophylaxis according to this invention may be advantageously possible in those patients showing or having an increased risk for one or more of the following disorders: renal insufficiency or diseases, cardiac diseases, cardiac failure, hepatic diseases, pulmonal diseases, catabolytic states and/or danger of lactate acidosis, or female patients being pregnant or during lactation.

Furthermore, it can be found that the administration of a pharmaceutical composition according to this invention results in no risk or in a low risk of hypoglycemia. Therefore, a treatment or prophylaxis according to this invention is also advantageously possible in those patients showing or having an increased risk for hypoglycemia.

A pharmaceutical composition according to this invention is particularly suitable in the long term treatment or prophylaxis of the diseases and/or conditions as described hereinbefore and hereinafter, in particular in the long term glycemic control in patients with type 2 diabetes mellitus.

The term "long term" as used hereinbefore and hereinafter indicates a treatment of or administration in a patient within a period of time longer than 12 weeks, preferably longer than 25 weeks, even more preferably longer than 1 year.

Therefore, a particularly preferred embodiment of the present invention provides a method for therapy, preferably oral therapy, for improvement, especially long term improvement, of glycemic control in patients with type 2 diabetes mellitus, especially in patients with late stage type 2 diabetes mellitus, in particular in patients additionally diagnosed of overweight, obesity (including class I, class II and/or class III obesity), visceral obesity and/or abdominal obesity.

It will be appreciated that the amount of the pharmaceutical composition according to this invention to be administered to the patient and required for use in treatment or prophylaxis according to the present invention will vary with the route of administration, the nature and severity of the condition for which treatment or prophylaxis is required, the age, weight and condition of the patient, concomitant medication and will be ultimately at the discretion of the attendant physician. In general, however, the SGLT2 inhibitor according to this invention is included in the pharmaceutical composition or dosage form in an amount sufficient that by its administration the glycemic control in the patient to be treated is improved.

In the following preferred ranges of the amount of the SGLT2 inhibitor to be employed in the pharmaceutical composition and the methods and uses according to this invention are described. These ranges refer to the amounts to be administered per day with respect to an adult patient, in particular to a human being, for example of approximately 70 kg body weight, and can be adapted accordingly with regard to an administration 2, 3, 4 or more times daily and with regard to other routes of administration and with regard to the age of the patient. Within the scope of the present invention, the pharmaceutical composition is preferably administered orally. Other forms of administration are possible and described hereinafter. Preferably the one or more dosage forms comprising the SGLT2 inhibitor is oral or usually well known.

In general, the amount of the SGLT2 inhibitor in the pharmaceutical composition and methods according to this invention is preferably the amount usually recommended for a monotherapy using said SGLT2 inhibitor.

The preferred dosage range of the SGLT2 inhibitor is in the range from 0.5 mg to 200 mg, even more preferably from 1 to 100 mg, most preferably from 1 to 50 mg per day. The oral administration is preferred. Therefore, a pharmaceutical composition may comprise the hereinbefore mentioned amounts, in particular from 1 to 50 mg or 1 to 25 mg, even more preferably 5 to 25 mg. Particular dosage strengths (e.g. per tablet or capsule) are for example 1, 2, 2.5, 5, 7.5, 10, 12.5, 15, 20, 25 or 50 mg of the SGLT2 inhibitor, such as a compound of the formula (I), in particular of the compound (1.9) or its crystalline form (I.9X).

The application of the active ingredient may occur up to three times a day, preferably one or two times a day, most preferably once a day.

A pharmaceutical composition which is present as a separate or multiple dosage form, preferably as a kit of parts, is useful in combination therapy to flexibly suit the individual therapeutic needs of the patient.

According to a first embodiment a preferred kit of parts comprises a containment containing a dosage form comprising the SGLT2 inhibitor and at least one pharmaceutically acceptable carrier.

A further aspect of the present invention is a manufacture comprising the pharmaceutical composition being present as separate dosage forms according to the present invention and a label or package insert comprising instructions

that the separate dosage forms are to be administered in combination or alternation.

According to a first embodiment a manufacture comprises (a) a pharmaceutical composition comprising a SGLT2 inhibitor according to the present invention and (b) a label or package insert which comprises instructions that the medicament is to be administered.

The desired dose of the pharmaceutical composition according to this invention may conveniently be presented in a once daily or as divided dose administered at appropriate intervals, for example as two, three or more doses per day.

The pharmaceutical composition may be formulated for oral, rectal, nasal, topical (including buccal and sublingual), transdermal, vaginal or parenteral (including intramuscular, subcutaneous and intravenous) administration in liquid or solid form or in a form suitable for administration by inhalation or insufflation. Oral administration is preferred. The formulations may, where appropriate, be conveniently presented in discrete dosage units and may be prepared by any of the methods well known in the art of pharmacy. All methods include the step of bringing into association the active ingredient with one or more pharmaceutically acceptable carriers, like liquid carriers or finely divided solid carriers or both, and then, if necessary, shaping the product into the desired formulation.

The pharmaceutical composition may be formulated in the form of tablets, granules, fine granules, powders, capsules, caplets, soft capsules, pills, oral solutions, syrups, dry syrups, chewable tablets, troches, effervescent tablets, drops, suspension, fast dissolving tablets, oral fast-dispersing tablets, etc..

The pharmaceutical compositions and dosage forms according to this invention may be packaged using PVC-blisters, PVDC-blisters, PVC/PVDC-blisters or a moisture-proof packaging material such as aluminium foil blister packs, alu/alu blister, transparent or opaque polymer blister with pouch, polypropylene tubes, glass bottles, PP bottles and HOPE bottles optionally containing a child-resistant feature or may be tamper evident. The primary packaging material may comprise a desiccant such as molecular sieve or silica gel to improve chemical stability of the active pharmaceutical ingredient(s). Opaque packaging such as colored blister materials, tubes, brown glass bottles or the like can be used to prolong shelflife of the active pharmaceutical ingredient(s)l by reduction of photodegradation.

The pharmaceutical composition and the dosage forms preferably comprises one or more pharmaceutical acceptable carriers which must be "acceptable" in the sense of being compatible with the other ingredients of the formulation and not deleterious to the recipient thereof. Examples of pharmaceutically acceptable carriers are known to the one skilled in the art.

Pharmaceutical compositions suitable for oral administration may conveniently be presented as discrete units such as capsules, including soft gelatin capsules, cachets or tablets each containing a predetermined amount of the active ingredient; as a powder or granules; as a solution, a suspension or as an emulsion, for example as syrups, elixirs or self-emulsifying delivery systems (SEDDS). The active ingredients may also be presented as a bolus, electuary or paste. Tablets and capsules for oral administration may contain conventional excipients such as binding agents, fillers, lubricants, disintegrants, or wetting agents. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, emulsifying agents, non-aqueous vehicles (which may include edible oils), or preservatives.

The pharmaceutical composition according to the invention may also be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulatory agents such as suspending, stabilizing and/or dispersing agents. Alternatively, the active ingredients may be in powder form, obtained by aseptic isolation of sterile solid or by lyophilisation from solution, for constitution with a suitable vehicle, e.g. sterile, pyrogen-free water, before use.

Pharmaceutical compositions suitable for rectal administration wherein the carrier is a solid are most preferably presented as unit dose suppositories. Suitable carriers include cocoa butter and other materials commonly used in the art, and the suppositories may be conveniently formed by admixture of the active compound(s) with the softened or melted carrier(s) followed by chilling and shaping in moulds.

The pharmaceutical compositions and methods according to this invention show advantageous effects in the treatment and prevention of those diseases and conditions as described hereinbefore. Advantageous effects may be seen for example with respect to efficacy, dosage strength, dosage frequency, pharmacodynamic properties, pharmacokinetic properties, fewer adverse effects, convenience, compliance, etc..

Methods for the manufacture of SGLT2 inhibitors according to this invention and of prodrugs thereof are known to the one skilled in the art. Advantageously, the compounds according to this invention can be prepared using synthetic methods as described in the literature, including patent applications as cited hereinbefore. Preferred methods of manufacture are described in the WO 2006/120208 and WO 2007/031548. With regard to compound (I.9) an advantageous crystalline form is described in the international patent application WO 2006/117359 which hereby is incorporated herein in its entirety.

The active ingredients may be present in the form of a pharmaceutically acceptable salt. Pharmaceutically acceptable salts include, without being restricted thereto, such as salts of inorganic acid like hydrochloric acid, sulfuric acid and phosphoric acid; salts of organic carboxylic acid like oxalic acid, acetic acid, citric acid, malic acid, benzoic acid, maleic acid, fumaric acid, tartaric acid, succinic acid and glutamic acid and salts of organic sulfonic acid like methanesulfonic acid and p-toluenesulfonic acid. The salts can be formed by combining the compound and an acid in the appropriate amount and ratio in a solvent and decomposer. They can be also obtained by the cation or anion exchange from the form of other salts.

The active ingredients or a pharmaceutically acceptable salt thereof may be present in the form of a solvate such as a hydrate or alcohol adduct.

Any of the above mentioned pharmaceutical compositions and methods within the scope of the invention may be tested by animal models known in the art. In the following, *in vivo* experiments are described which are suitable to evaluate pharmacologically relevant properties of pharmaceutical compositions and methods according to this invention.

Pharmaceutical compositions and methods according to this invention can be tested in genetically hyperinsulinemic or diabetic animals like db/db mice, ob/ob mice, Zucker Fatty (fa/fa) rats or Zucker Diabetic Fatty (ZDF) rats. In addition, they can be tested in animals with experimentally induced diabetes like HanWistar or Sprague Dawley rats pretreated with streptozotocin.

The effect on glycemic control according to this invention can be tested after single dosing of the SGLT2 inhibitor in an oral glucose tolerance test in the animal models described hereinbefore. The time course of blood glucose is followed after an oral glucose challenge in overnight fasted animals. The pharmaceutical compositions according to the present invention significantly improve glucose excursion, for example compared to another monotherapy, as measured by reduction of peak glucose concentrations or reduction of glucose AUC. In addition, after multiple dosing of the SGLT2 inhibitor in the animal models described hereinbefore, the effect on glycemic control can be determined by measuring the HbA1c value in blood. The pharmaceutical compositions according to this invention significantly reduce HbA1c, for example compared to another monotherapy or compared to a dual-combination therapy.

The improved independence from insulin of the treatment according to this invention can be shown after single dosing in oral glucose tolerance tests in the animal models described hereinbefore. The time course of plasma insulin is followed after a glucose challenge in overnight fasted animals.

The increase in active GLP-1 levels by treatment according to this invention after single or multiple dosing can be determined by measuring those levels in the plasma of animal models described hereinbefore in either the fasting or postprandial state. Likewise, a reduction in glucagon levels in plasma can be measured under the same conditions.

The effect of a SGLT2 inhibitor according to the present invention on beta-cell regeneration and neogenesis can be determined after multiple dosing in the animal models described hereinbefore by measuring the increase in pancreatic insulin content, or by measuring increased beta-cell mass by morphometric analysis after immunhistochemical staining of pancreatic sections, or by measuring increased glucose-stimulated insulin secretion in isolated pancreatic islets.

## Examples

### Pharmacological Examples

The following examples show the beneficial effect on glycemic control of the pharmaceutical compositions according to the present invention.

**Example 1:**

According to a first example an oral glucose tolerance test is performed in overnight fasted 9-weeks old male Zucker Diabetic Fatty (ZDF) rats (ZDF/Crl-Lepr$^{fa}$). A pre-dose blood sample is obtained by tail bleed. Blood glucose is measured with a glucometer, and the animals are randomized for blood glucose (n = 5 / group). Subsequently, the groups receive a single oral administration of either vehicle alone (0.5% aqueous hydroxyethylcellulose containing 3 mM HCl and 0.015% Polysorbat 80) or vehicle containing the SGLT2 inhibitor. The animals receive an oral glucose load (2 g/kg) 30 min after compound administration. Blood glucose is measured in tail blood 30 min, 60 min, 90 min, 120 min, and 180 min after the glucose challenge. Glucose excursion is quantified by calculating the reactive glucose AUC. The data are presented as mean ± SEM. The two-sided unpaired Student t-test is used for statistical comparison of the control group and the active groups.

A representative experiment is shown in Figures 3A and 3B. Compound (I.9) (1-chloro-4-(β-D-glucopyranos-1-yl)-2-[4-((S)-tetrahydrofuran-3-yloxy)-benzyl]-benzene) was orally administered to ZDF rats at doses of 0.3 mg/kg, 3 mg/kg or 30 mg/kg body weight. The animals then received an oral glucose bolus and the resulting glucose-time profile is shown in Figure 3A. The baseline-corrected area under the glucose-time curves are shown in Figure 3B. Compound

(I.9) reduced glucose excursion by 15% at 0.3 mg/kg (not significant), by 62% at 3 mg/kg ($p < 0.001$) and by 89% at 30 mg/kg ($p < 0.001$).

**Example 2:**

According to a second example an oral glucose tolerance test is performed in overnight fasted male Sprague Dawley rats (Crl:CD(SD)) with a body weight of about 200 g. A pre-dose blood sample is obtained by tail bleed. Blood glucose is measured with a glucometer, and the animals are randomized for blood glucose (n = 5 / group). Subsequently, the groups receive a single oral administration of either vehicle alone (0.5% aqueous hydroxyethylcellulose containing 0.015% Polysorbat 80) or vehicle containing the SGLT2 inhibitor. The animals receive an oral glucose load (2 g/kg) 30 min after compound administration. Blood glucose is measured in tail blood 30 min, 60 min, 90 min, and 120 min after the glucose challenge. Glucose excursion is quantified by calculating the reactive glucose AUC. The data are presented as mean ± S.E.M. Statistical comparisons are conducted by Student's t test.

**Example 3: Treatment of pre-diabetes**

The efficacy of a pharmaceutical composition according to the invention in the treatment of pre-diabetes characterised by pathological fasting glucose and/or impaired glucose tolerance can be tested using clinical studies. In studies over a shorter period (e.g. 2-4 weeks) the success of the treatment is examined by determining the fasting glucose values and/or the glucose values after a meal or after a loading test (oral glucose tolerance test or food tolerance test after a defined meal) after the end of the period of therapy for the study and comparing them with the values before the start of the study and/or with those of a placebo group. In addition, the fructosamine value can be determined before and after therapy and compared with the initial value and/or the placebo value. A significant drop in the fasting or non-fasting glucose levels demonstrates the efficacy of the treatment. In studies over a longer period (12 weeks or more) the success of the treatment is tested by determining the HbA1c value, by comparison with the initial value and/or with the value of the placebo group. A significant change in the HbA1c value compared with the initial value and/or the placebo value demonstrates the efficacy of the pharmaceutical composition according to the invention for treating pre-diabetes.

**Example 4: Preventing manifest type 2 diabetes**

Treating patients with pathological fasting glucose and/or impaired glucose tolerance (pre-diabetes) is also in pursuit of the goal of preventing the transition to manifest type 2 diabetes. The efficacy of a treatment can be investigated in a comparative clinical study in which pre-diabetes patients are treated over a lengthy period (e.g. 1-5 years) with either a pharmaceutical composition according to this invention or with placebo or with a non-drug therapy or other medicaments. During and at the end of the therapy, by determining the fasting glucose and/or a loading test (e.g. oGTT), a check is made to determine how many patients exhibit manifest type 2 diabetes, i.e. a fasting glucose level of >125 mg/dl and/or a 2h value according to oGTT of >199 mg/dl. A significant reduction in the number of patients who exhibit manifest type 2 diabetes when treated with a pharmaceutical composition according to this invention as compared to one of the other forms of treatment, demonstrates the efficacy in preventing a transition from pre-diabetes to manifest diabetes.

**Example 5: Treatment of type 2 diabetes**

Treating patients with type 2 diabetes with the pharmaceutical composition according to the invention, in addition to producing an acute improvement in the glucose metabolic situation, prevents a deterioration in the metabolic situation in the long term. This can be observed is patients are treated for a longer period, e.g. 3 months to 1 year or even 1 to 6 years, with the pharmaceutical composition according to the invention and are compared with patients who have been treated with other antidiabetic medicaments. There is evidence of therapeutic success compared with patients treated with other antidiabetic medicaments if no or only a slight increase in the fasting glucose and/or HbA1c value is observed. Further evidence of therapeutic success is obtained if a significantly smaller percentage of the patients treated with a pharmaceutical composition according to the invention, compared with patients who have been treated with other medicaments, undergo a deterioration in the glucose metabolic position (e.g. an increase in the HbA1c value to >6.5% or >7%) to the point where treatment with an additional oral antidiabetic medicament or with insulin or with an insulin analogue is indicated.

**Example 6: Treatment of insulin resistance**

In clinical studies running for different lengths of time (e.g. 2 weeks to 12 months) the success of the treatment is checked using a hyperinsulinaemic euglycaemic glucose clamp study. A significant rise in the glucose infusion rate

at the end of the study, compared with the initial value or compared with a placebo group, or a group given a different therapy, proves the efficacy of a pharmaceutical composition according to the invention in the treatment of insulin resistance.

**Example 7: Treatment of hyperglycaemia**

In clinical studies running for different lengths of time (e.g. 1 day to 24 months) the success of the treatment in patients with hyperglycaemia is checked by determining the fasting glucose or non-fasting glucose (e.g. after a meal or a loading test with oGTT or a defined meal). A significant fall in these glucose values during or at the end of the study, compared with the initial value or compared with a placebo group, or a group given a different therapy, proves the efficacy of a pharmaceutical composition according to the invention in the treatment of hyperglycaemia.

**Example 8: Prevention of micro- or macrovascular complications**

The treatment of type 2 diabetes or pre-diabetes patients with a pharmaceutical composition according to the invention prevents or reduces or reduces the risk of developing microvascular complications (e.g. diabetic neuropathy, diabetic retinopathy, diabetic nephropathy, diabetic foot, diabetic ulcer) or macrovascular complications (e.g. myocardial infarct, acute coronary syndrome, unstable angina pectoris, stable angina pectoris, stroke, peripheral arterial occlusive disease, cardiomyopathy, heart failure, heart rhythm disorders, vascular restenosis). Type 2 diabetes or patients with pre-diabetes are treated long-term, e.g. for 1-6 years, with a pharmaceutical composition according to the invention and compared with patients who have been treated with other antidiabetic medicaments or with placebo. Evidence of the therapeutic success compared with patients who have been treated with other antidiabetic medicaments or with placebo can be found in the smaller number of single or multiple complications. In the case of macrovascular events, diabetic foot and/or diabetic ulcer, the numbers are counted by anamnesis and various test methods. In the case of diabetic retinopathy the success of the treatment is determined by computer-controlled illumination and evaluation of the background to the eye or other ophthalmic methods. In the case of diabetic neuropathy, in addition to anamnesis and clinical examination, the nerve conduction rate can be measured using a calibrated tuning fork, for example. With regard to diabetic nephropathy the following parameters may be investigated before the start, during and at the end of the study: secretion of albumin, creatinin clearance, serum creatinin values, time taken for the serum creatinin values to double, time taken until dialysis becomes necessary.

**Example 9: Treatment of Metabolic Syndrome**

The efficacy of a pharmaceutical composition according to the invention can be tested in clinical studies with varying run times (e.g. 12 weeks to 6 years) by determining the fasting glucose or non-fasting glucose (e.g. after a meal or a loading test with oGTT or a defined meal) or the HbA1c value. A significant fall in these glucose values or HbA1c values during or at the end of the study, compared with the initial value or compared with a placebo group, or a group given a different therapy, proves the efficacy of an active substance in the treatment of Metabolic Syndrome. Examples of this are a reduction in systolic and/or diastolic blood pressure, a lowering of the plasma triglycerides, a reduction in total or LDL cholesterol, an increase in HDL cholesterol or a reduction in weight, either compared with the starting value at the beginning of the study or in comparison with a group of patients treated with placebo or a different therapy.

**Examples of Formulations**

The following examples of formulations, which may be obtained analogously to methods known in the art, serve to illustrate the present invention more fully without restricting it to the contents of these examples. The term "active substance" denotes a SGLT-2 inhibitor according to this invention, especially a compound of the formula (I), for example a compound of the formula (I.9) or its crystalline form (L9X).

The active pharmaceutical ingredient or active sustance, i.e. the compound (I.9), preferably in the crystalline form (I.9X), is milled with a suitable mill like pin- or jet-mill in order to obtain the desired particle size distribution before manufacturing of the pharmaceutical composition or dosage form.

Examples of typical particle size distribution values X90, X50 and X10 for the preferred active pharmaceutical ingredient according to the invention are shown in the table below.

Typical particle size distribution results

| | Active substance Batch 1 | Active substance Batch 2 |
|---|---|---|
| X10 | 1,8 μm | 1,7 μm |
| X50 | 18,9 μm | 12,1 μm |
| X90 | 45,3 μm | 25,9 μm |

Example 1: Dry ampoule containing 50 mg of active substance per 10 ml

Composition:

| | |
|---|---|
| Active substance | 50.0 mg |
| Mannitol | 50.0 mg |
| water for injections | ad 10.0 ml |

Preparation:

Active substance and mannitol are dissolved in water. After packaging the solution is freeze-dried. To produce the solution ready for use, the product is dissolved in water for injections.

Example 2: Dry ampoule containing 25 mg of active substance per 2 ml

Composition:

| | |
|---|---|
| Active substance | 25.0 mg |
| Mannitol | 100.0 mg |
| water for injections | ad 2.0 ml |

Preparation:

Active substance and mannitol are dissolved in water. After packaging, the solution is freeze-dried.
To produce the solution ready for use, the product is dissolved in water for injections.

Example 3: Tablet containing 50 mg of active substance

Composition:

| | |
|---|---|
| (1) Active substance | 50.0 mg |
| (2) Mannitol | 98.0 mg |
| (3) Maize starch | 50.0 mg |
| (4) Polyvinylpyrrolidone | 15.0 mg |
| (5) Magnesium stearate | 2.0 mg |
| | 215.0 mg |

Preparation:

(1), (2) and (3) are mixed together and granulated with an aqueous solution of (4). (5) is added to the dried granulated material. From this mixture tablets are pressed, biplanar, faceted on both sides and with a dividing notch on

one side.

Diameter of the tablets: 9 mm.

Example 4: Capsules containing 50 mg of active substance

Composition:

| | |
|---|---|
| (1) Active substance | 50.0 mg |
| (2) Dried maize starch | 58.0 mg |
| (3) Mannitol | 50.0 mg |
| (4) Magnesium stearate | 2.0 mg |
| | 160.0 mg |

Preparation:

(1) is triturated with (3). This trituration is added to the mixture of (2) and (4) with vigorous mixing. This powder mixture is packed into size 3 hard gelatin capsules in a capsule filling machine.

Example 5: Tablets containing 2.5mg, 5mg, 10mg, 25mg, 50mg of active substance

| **Active substance** | 2.5 mg mg/per tablet | 5 mg mg/per tablet | 10 mg mg/per tablet | 25 mg mg/per tablet | 50 mg mg/per tablet |
|---|---|---|---|---|---|
| **Wet granulation** | | | | | |
| active substance | 2.5000 | 5.000 | 10.00 | 25.00 | 50.00 |
| Lactose Monohydrate | 40.6250 | 81.250 | 162.50 | 113.00 | 226.00 |
| Microcrystalline Cellulose | 12.5000 | 25.000 | 50.00 | 40.00 | 80.00 |
| Hyd roxypropyl Cellulose | 1.8750 | 3.750 | 7.50 | 6.00 | 12.00 |
| Croscarmellose Sodium | 1.2500 | 2.500 | 5.00 | 4.00 | 8.00 |
| Purified Water | q.s. | q.s. | q.s. | q.s. | q.s. |
| **Dry Adds** | | | | | |
| Microcrystalline Cellulose | 3.1250 | 6.250 | 12.50 | 10.00 | 20.00 |
| Colloidal silicon dioxide | 0.3125 | 0.625 | 1.25 | 1.00 | 2.00 |
| Magnesium stearate | 0.3125 | 0.625 | 1.25 | 1.00 | 2.00 |
| Total core | 62.5000 | 125.000 | 250.00 | 200.00 | 400.00 |
| **Film Coating** | | | | | |
| Film coating pre-mix | 2.5000 | 4.000 | 7.00 | 6.00 | 9.00 |
| Purified Water | q.s. | q.s. | q.s. | q.s. | q.s. |
| **Total** | **65.000** | **129.000** | **257.00** | **206.00** | **409.00** |

Example 6: (a) Manufacturing process for tablets

A tablet according to Example 5 above is for example manufactured as set forth below.

| Step | EQUIPMENT | MATERIALS | OPERATION | IN-PROCESS CONTROLS |
|---|---|---|---|---|
| 1 | Screen, blender and high shear granulator | Active substance → | MIX | |
| | | Hydroxypropyl Cellulose (screened) → | | |
| | | Croscarmellose Sodium → | | |
| | | Part of Microcrystalline Cellulose (PH102) → | | |
| | | Lactose Monohydrate → | | |
| 2 | High shear granulator | Purified Water → | GRANULATE | |
| 3 | Fluid bed drier | | ↓ DISCHARGE ONTO DRYER AND DRY | LOD ≤ 2.0%, preferably ≤ 1.5%, at 100°C |
| 4 | Mill | | ↓ DRY MILL | |
| 5 | Mill, blender | Colloidal Silicon Dioxide + Microcrystalline Cellulose (PH102) → | ↓ MIX | |
| 6 | Mill, blender | Magnesium Stearate → | ↓ MIX | |
| | | | ↓ Final tablet blend | |
| 7 | Tablet press | | ↓ COMPRESS INTO TABLETS | Tablet weight, height, crushing strength, friability, disintegration |
| | | | ↓ Core tablets | |

| Step | EQUIPMENT | MATERIALS | OPERATION | IN-PROCESS CONTROLS |
|---|---|---|---|---|
| | | | ↓ | |
| 8 | Propeller Stirrer<br>Drum coater | Suspend film-coating system in water and mix → | FILM COATING | |
| | | | ↓ | |
| | | | Final film coated tablets | Tablet weight, height, crushing strength, disintegration |

Example 6: (b) Manufacturing process for tablets

A tablet according to Example 5 above is for example manufactured as set forth below.

| Step | EQUIPMENT | MATERIALS | OPERATION | IN-PROCESS CONTROLS |
|---|---|---|---|---|
| 1 | Screen, blender and high shear granulator | Lactose Monohydrate →<br>Active substance →<br>Croscarmellose Sodium →<br>Hydroxypropyl Cellulose →<br>Part of Microcrystalline Cellulose (PH102) → | MIX | |
| 2 | High shear granulator | Purified Water → | Granulate | |
| | | | ↓ | |
| 3 | Fluid bed drier | | DISCHARGE ONTO DRYER AND DRY | LOD ≤ 2.0%, preferably ≤ 1.5%, at 100°C |
| | | | ↓ | |
| 4 | Mill | | DRY MILL | |
| | | | ↓ | |
| 5 | Screen, blender | Colloidal Silicon Dioxide + Microcrystalline Cellulose (PH102) → | MIX | |
| | | | ↓ | |
| 6 | Screen, blender | Magnesium Stearate → | MIX | |
| | | | ↓ | |

| | | | | | |
|---|---|---|---|---|---|
| | | | | Final tablet blend | |
| | | | | ↓ | |
| 7 | Tablet press | | | COMPRESS INTO TABLETS | Appearance, tablet weight, height, crushing strength, friability, disintegration |
| | | | | ↓ | |
| | | | | Core tablets | |
| | | | | ↓ | |
| 8 | Propeller Stirrer Drum coater | Suspend film-coating pre-mix in water and mix | → | FILM COATING | |
| | | | | ↓ | |
| | | | | Final film coated tablets | Appearance, tablet weight, height, crushing strength, disintegration |

Active substance granulate

The active substance, e.g. the compound (I.9), preferably in the crystalline form (I.9X), Lactose Monohydrate, Croscarmellose sodium, Hydroxypropylcellulose and Cellulose microcristalline are screened and subsequently pre-mixed in an appropriate high-shear mixer.

The pre-mix is moistened with purified water and granulated using an appropriate high-shear mixer. The granulate is dried in a fluid bed dryer. Subsequently, the granulate is screened through a suitable sieve.

Final blend

Pre-screened silicia, colloidal anhydrous and cellulose microcristalline are added to the granulate and blended in an appropriate free-fall blender.

Pre-screened magnesium stearate is added to the blend and subsequently final blending is performed in an appropriate free-fall blender.

Tablet cores

The final blend is compressed into tablet cores using a standard rotary tablet press.

Film-coating suspension

An aqueous suspension of opadry yellow 02B38190 (dye suspension) is dispersed within in purified water.

Film-coated tablets

The tablet cores are coated with the film-coating suspension in a drum coater to produce film-coated tablets.

Manufacturing process:

1. Granulate

1.1. Wet granulation

After dispensing, the following raw materials are prescreened using a suitable screening machine to a suitable high-shear mixer/granulator or diffusion blender or diffusion blender and pre-mix until homogeneous:

- ca. 20 - 80 % (for example 50%) of the total quantity of Lactose
- Active substance
- 50 - 90% (for example 80%) of the total quantity of
- Hydroxypropyl cellulose
- Croscarmellose sodium
- the remaining of the total quantity of Lactose
- Microcrystalline cellulose.

Alternatively, the above mentioned excipients are transfered to a suitable high-shear mixer/granulator or diffusion blender without pre-sieving.

Alternatively, the above mentioned excipients are transfered individually to a suitable high-shear mixer/granulator or diffusion blender without pre-sieving and the above mentioned excipients are transfered individually to a suitable high-shear mixer/granulator or diffusion blender with pre-sieving.

For the cases in which blending is performed in a diffusion blender the pre-blended product is transferred to a high-shear mixer/granulator prior to wet granulation.

For pre-screening of excipients a screening mill with a 0.5mm to 1.5mm (for example 0.8mm) sieve at 50 rpm to 2500 rpm (for example 970 rpm) can be used.

Alternatively, a hand sieve with a 0.5mm to 1.5mm (for example 0.8mm) sieve is used.

Then the mixture is wet with water purified in the range of 26 to 35 % (w/w) water purified (for example 28% (w/w) water purified) of the total weight of the pre-mixed excipients.

For pre-mixing in a high-shear mixer/granulator:following process parameters are applicable:

| | |
|---|---|
| Duration: | 3-12.5 min (for example 5min ) |
| Rotor speed setting: | 100-600 rpm (for example 114min ) |
| Chopper speed setting: | 0 to 3000 rpm (for example 1450 rpm) |

Alternatively for pre-mixing in a diffusion blender following process parameters are applicable:

Duration: 5 -30min

Rotation speed: 5- 30 rpm

For wetting in a high-shear mixer/granulator, following process parameters are applicable:

Wetting: Duration: 2 to 5min (for example 2.5min )

Rotor speed setting: 50-600 rpm (for example 114min )
Chopper speed setting: 1500 to 3000 rpm (for example 2900 rpm)

Granulation: Duration: 2 to 5min (for example 2.5 min )

Rotor speed setting: 100-600 rpm (for example 114min )
Chopper speed setting: 1500 to 3000 rpm (for example 2900 rpm)

Water purified is sprayed into the high-shear mixer/granulator using a nozzle with a spray angle of 45-90° (for example 60°) or alternatively water purified is poured into the high-shear mixer/granulator.

1.2. Drying

The wet granulate is dried in a suitable fluid bed dryer. Drying is performed with or alternatively without pre-heating of the fluid bed dryer.

For drying in a fluid bed drier following process parameters are applicable:

Air volume: 100 - 5000 $m^3/h$
Inlet air temperature: 50 - 75 °C (for example 70°C)
Endpoint of process: When product temperature is in the range of 40 to 50°C

The endpoint is monitored by in process control of loss on drying:
Suitable values for loss on drying: 0.5 - 5.0% (for example ≤ 1.5%).

1.3. Dry screening

The dried granulate is screened using a suitable screening mill with a 0.5mm to 2.0mm (for example 1.0mm) sieve at 50 rpm to 2500 rpm (for example 970 rpm) or a hand sieve with a 0.5mm to 1.5mm (for example 0.8mm) sieve is used

2. Preparation of the final mixture

2.1. Main mixing step

In a suitable diffusion blender, the screened, dried granulate is mixed with Colloidal anhydrous silica (pre-screened using a screening mill or a hand sieve machine) and Microcrystalline cellulose (remaining amount) (pre-screened using a screening mill or a hand sieve machine).

For screening of colloidal anhydrous silica and microcrystalline cellulose a screening mill with a 0.5mm to 1.5mm (for example 0.8mm) sieve at 50 rpm to 2500 rpm (for example 970 rpm) can be used. Alternatively, a hand sieve with a 0.5mm to 1.5mm (for example 0.8mm) sieve is used.

For blending a diffusion blender is applicable at following process parameters:

Duration: 5 -30min (for example 15min)
Rotation speed: 5- 30 rpm (for example 10rpm)

Alternatively for blending a high-shear mixer/granulator, following process parameters is applicable:

| | |
|---|---|
| Duration: | 3-30 min |
| Rotor speed setting: | 50-600 rpm |
| Chopper speed setting: | 0 to 3000 rpm |

2.2. Final mixing step

The main blend is placed in a suitable diffusion blender. Magnesium stearate (pre-screened using a hand sieve 0.5mm or alternatively not pre-screened) is added to the main blend.

For final blending a diffusion blender is applicable at following process parameters:

Duration: 5 -30min (for example 10min)
Rotation speed: 5- 30 rpm (for example 10rpm)

3. Tablet cores

On a suitable rotary tablet press the final blend are compressed into tablet cores.

Following process parameters are applicable for tabletting:

Tabletting speed: 20.000 - 300.000 tablets per hour depending on the output of the tabletting machine.
Stirrer blade speed: 10 - 50 rpm (for example 40rpm)
Compression force: 5 - 26 KN (for example 8-20 KN, depending on the tablet size)

4. Film-coating suspension

Water purified is placed in a suitable mixing vessel, OPADRY YELLOW 02B38190 is added and stirred in using a propeller stirrer until complete dissolution

5. Film-coating

In a suitable pan-coater the tablet cores are coated with film-coating suspension A pan-coater of suitable size is used for film-coating of core tablets. Coating is performed in a four step process: pre-heating of tablets, film-coating, drying and cooling.

Following process parameters are applicable for film-coating depending on the equipment size:

| | |
|---|---|
| Drum speed: | 6 - 18 rpm |
| Inlet air flow rate: | 50 - 2000 $m^3/h$ |
| Exhaust air temperature: | 40 - 54°C |
| Spray rate: | 3 - 500 g / min. |

Example 7: Pharmaceutical composition containing other fillers

Copovidone is dissolved in purified water at ambient temperature to produce a granulation liquid. A glucopyranosyl-substituted benzene derivative according to the present invention, mannitol, pregelatinized starch and corn starch are blended in a suitable mixer, to produce a pre-mix. The pre-mix is moistened with the granulation liquid and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm.

Magnesium stearate is passed through a sieve for delumping and added to the granulate. Subsequently the final blend is produced by final blending in a suitable blender for three minutes and compressed into tablet cores.

Hydroxypropyl methylcellulose, polyethylene glycol, talc, titanium dioxide and iron oxide are suspended in purified water in a suitable mixer at ambient temperature to produce a coating suspension. The tablet cores are coated with the coating suspension to a weight gain of about 3 % to produce film-coated tablets. The following formulation variants can be obtained:

| **Ingredient** | **mg/ tablet** | **mg/ tablet** | **mg/ tablet** | **mg/ tablet** | **mg/ tablet** |
|---|---|---|---|---|---|
| Active substance | 2.5 | 5.0 | 10.0 | 25.0 | 50.0 |
| Mannitol | 133.4 | 130.9 | 125.9 | 110.9 | 221.8 |
| Pregelatinised starch | 18.0 | 18.0 | 18.0 | 18.0 | 36.0 |
| Maize starch | 18.0 | 18.0 | 18.0 | 18.0 | 36.0 |
| Copovidone | 5.4 | 5.4 | 5.4 | 5.4 | 10.8 |
| Magnesium stearate | 2.7 | 2.7 | 2.7 | 2.7 | 5.4 |
| Film coat | 5.0 | 5.0 | 5.0 | 5.0 | 10.0 |
| **Total** | **185.0** | **185.0** | **185.0** | **185.0** | **370.0** |

Example 8: Pharmaceutical composition containg other disintegrant

Copovidone is dissolved in purified water at ambient temperature to produce a granulation liquid. An glucopyranosyl-substituted benzene derivative according to the present invention, mannitol, pregelatinized starch and corn starch are blended in a suitable mixer, to produce a pre-mix. The pre-mix is moistened with the granulation liquid and subsequently granulated. The moist granulate is sieved through a suitable sieve. The granulate is dried at about 60 °C inlet air temperature in a fluid bed dryer until a loss on drying value of 1-4 % is obtained. The dried granulate is sieved through a sieve with a mesh size of 1.0 mm.

Crospovidone is added to the dried granulate and mixed for 5 minutes to produce the main blend. Magnesium stearate is passed through a sieve for delumping and added to main blend. Subsequently the final blend is produced

by final blending in a suitable blender for three minutes and compressed into 8 mm round tablet cores with a compression force of 16 kN.

Hydroxypropyl methylcellulose, polyethylene glycol, talc, titanium dioxide and iron oxide are suspended in purified water in a suitable mixer at ambient temperature to produce a coating suspension. The tablet cores are coated with the coating suspension to a weight gain of about 3 % to produce film-coated tablets. The following formulation variants can be obtained:

| Ingredient | mg/ tablet | mg/ tablet | mg/ tablet | mg/ tablet | mg/ tablet |
|---|---|---|---|---|---|
| Active substance | 2.5 | 5.0 | 10.0 | 25.0 | 50.0 |
| Mannitol | 127.5 | 125.0 | 120.0 | 105.0 | 210.0 |
| Microcrystalline Cellulose | 39.0 | 39.0 | 39.0 | 39.0 | 78.0 |
| Crospovidone | 2.0 | 2.0 | 2.0 | 2.0 | 4.0 |
| Copovidone | 5.4 | 5.4 | 5.4 | 5.4 | 10.8 |
| Magnesium stearate | 3.6 | 3.6 | 3.6 | 3.6 | 7.2 |
| Film coat | 5.0 | 5.0 | 5.0 | 5.0 | 10.0 |
| **Total** | **185.0** | **185.0** | **185.0** | **185.0** | **370.0** |

The tablet hardness, the friability, the content uniformity, the disintegration time and the dissolution properties are determined as described hereinbefore.

Example 9: Direct compression formulation

1. Screen the active ingredient, microcrystalline cellulose, croscarmellose.sodium and either hydroxypropyl cellulose or polyethylene glycol powder through a 20 mesh hand screen.
2. Add the above items into the high shear mixer and mix for two minutes.
3. Make a premix (~1/1) of the lactose and colloidal silicon dioxide.
4. Screen the premix through a 20 mesh hand screen and add to the mixer.
5. Screen the remaining lactose through a 20 mesh hand screen and add to the mixer.
6. Mix in components in the mixer for 2 minutes.
7. Screen the magnesium stearate through a 30 mesh hand screen and add to the mixer.
8. Mix for 1 minute 30 seconds to obtain the final blend.
9 Tabletting of the final blend on a suitable tabletting press.
10. Optionally film coating of the tablet cores.

| Ingredient | mg/ tablet | mg/ tablet | mg/ tablet | mg/ tablet | mg/ tablet |
|---|---|---|---|---|---|
| Active substance | 2.5000 | 5.000 | 10.00 | 25.0 | 50.0 |
| Lactose Monohydrate | 43.7500 | 87.500 | 175.00 | 74.0 | 148.0 |
| Microcrystalline Cellulose | 12.5000 | 25.000 | 50.00 | 80.0 | 160.0 |
| Polyethylene glycol | - | - | - | 10.0 | 20.0 |
| Croscarmellose sodium | 1.2500 | 2.500 | 5.00 | 8.0 | 16.0 |
| Hydroxypropyl cellulose | 1.8750 | 3.750 | 7.50 | - | - |
| Colloidal Silicon dioxide | 0.3125 | 0.625 | 1.25 | 1.0 | 2.0 |
| Magnesium stearate | 0.3125 | 0.625 | 1.25 | 2.0 | 4.0 |
| Film coat | 2.5000 | 4.000 | 7.00 | 6.00 | 9.00 |
| Purified water | q.s. | q.s. | q.s. | q.s. | q.s. |

(continued)

| Ingredient | mg/ tablet | mg/ tablet | mg/ tablet | mg/ tablet | mg/ tablet |
|---|---|---|---|---|---|
| **Total** | **65.000** | **129.000** | **257.00** | **206.00** | **409.00** |

Example 10: Tablets containing 0.5mg, 5mg, 25mg, 100mg of active substance

| Active substance | 0.5 mg mg/per tablet | 5 mg mg/per tablet | 25 mg mg/per tablet | 100 mg mg/per tablet |
|---|---|---|---|---|
| **Wet granulation** | | | | |
| active substance | 2.5000 | 5.000 | 25.00 | 100.00 |
| Lactose Monohydrate | 60.00 | 55.00 | 42.00 | 168.00 |
| Microcrystalline Cellulose | 20.00 | 20.00 | 38.00 | 152.00 |
| Hyd roxypropyl Cellulose | 5.00 | 5.00 | 7.50 | 30.00 |
| Croscarmellose Sodium | 4.00 | 4.00 | 6.00 | 24.00 |
| Purified Water | q.s. | q.s. | q.s. | q.s. |
| **Dry Adds** | | | | |
| Microcrystalline Cellulose | 10.00 | 10.00 | 30.00 | 120.00 |
| Colloidal silicon dioxide | - | 0.50 | 0.75 | 3.00 |
| Magnesium stearate | 0.50 | 0.50 | 0.75 | 3.00 |
| **Total** | **100.00** | **100.00** | **150.00** | **600.00** |

The active substance, e.g. the compound (I.9), preferably in the crystalline form (I.9X), hydroxypropyl cellulose, and croscarmellose sodium are mixed in a blender. This premix is mixed with lactose monohydrate and a portion of microcrystalline cellulose. The resulting blend is granulated with purified water. Multiple granulation subparts may be produced for an individual tablet batch, as needed, depending on the batch size and equipment used.

The granulation is discharged onto dryer trays and dried. The granulation is then milled. The remainder of the microcrystalline cellulose is added (as a premix with the colloidal silicon dioxide for all strengths other than the 0.5 mg) to the milled granulation, and mixed. The magnesium stearate is premixed with a portion of the blend, screened into the remainder of the granulation, and mixed.

The final tablet blend is compressed into tablets using a tablet press. The finished tablets are packaged using a suitable container closure system.

Example 11: Tablets containing 1mg, 5mg, 25mg of active substance

| Active substance | 1 mg mg/per tablet | 5 mg mg/per tablet | 25 mg mg/per tablet |
|---|---|---|---|
| **Wet granulation** | | | |
| active substance | 1.00 | 5.00 | 25.00 |
| Lactose Monohydrate | 63.00 | 59.00 | 39.00 |
| Microcrystalline Cellulose | 20.00 | 20.00 | 20.00 |
| Hyd roxypropyl Cellulose | 3.00 | 3.00 | 3.00 |
| Croscarmellose Sodium | 2.00 | 2.00 | 2.00 |

(continued)

| Active substance | 1 mg mg/per tablet | 5 mg mg/per tablet | 25 mg mg/per tablet |
|---|---|---|---|
| **Wet granulation** | | | |
| Purified Water | q.s. | q.s. | q.s. |
| **Dry Adds** | | | |
| Microcrystalline Cellulose | 10.00 | 10.00 | 10.00 |
| Colloidal silicon dioxide | 0.50 | 0.50 | 0.50 |
| Magnesium stearate | 0.50 | 0.50 | 0.50 |
| **Total** | **100.00** | **100.00** | **100.00** |

The active substance, e.g. the compound (I.9), preferably in the crystalline form (L9X), is passed through a screen and added to a blender or a high shear granulator. The hydroxypropyl cellulose and croscarmellose sodium are passed through a screen, added to the drug substance, and mixed. The intra-granular portion of microcrystalline cellulose is passed through a screen into a high shear granulator and mixed with the drug substance premix. Lactose is then added by passing the material through a screen into the granulator and mixing. The resulting blend is granulated with purified water. For larger batches, multiple granulation subparts may be produced for an individual tablet batch, as needed, depending on the batch size and equipment used.

The granulation is discharged onto dryer trays and dried. The granulation is then passed through a mill into a blender. The colloidal silicon dioxide is pre-mixed with a portion of the extra-granular microcrystalline cellulose. This premix is passed through a mill into the blender, followed by the remaining extra-granular microcrystalline cellulose, and mixed with the milled granulation. The magnesium stearate is premixed with a portion of the blend, passed through a mill into the remainder of the granulation, and mixed.

The final tablet blend is compressed into tablets using a tablet press. The finished tablets are packaged using a suitable container closure system.

## Examples of Tests with regard to Properties of Pharmaceutical Compositions and Pharmaceutical Dosage Forms

### 1. Disintegration Test

Disintegration test was performed as described in USP31-NF26 S2, chapter 701 (disintegration). The tables below indicate the average disintegration time (in minutes) for tablets produced at the beginning, middle and end of the production run for the tablets. The active substance in the tablets is the compound (I.9), preferably in the crystalline form (I.9X).

### 1.1. Disintegration of Tablets of Example 10 (section Examples of formulations)

| mg active substance per tablet | Disintegration avg. Minutes | |
|---|---|---|
| 0.5 | Beginning | 1:33 |
| | Middle | 1:23 |
| | End | 1:20 |
| | | |
| 5 | Beginning | 1:38 |
| | Middle | 1:50 |
| | End | 1:09 |
| | | |

(continued)

| mg active substance per tablet | Disintegration avg. Minutes | |
|---|---|---|
| 25 | Beginning | 0:45 |
| | Middle | 0:53 |
| | End | 0:45 |
| | | |
| 100 | Beginning | 1:15 |
| | Middle | 1:15 |
| | End | 1:06 |

1.2. Disintegration of Tablets of Example 11 (section Examples of formulations)

| mg active substance per tablet | Disintegration avg. Minutes | |
|---|---|---|
| 1 | Beginning | 3:21 |
| | Middle | 2:58 |
| | End | 2:45 |
| | | |
| 5 | Beginning | 2:49 |
| | Middle | 2:34 |
| | End | 2:36 |
| | | |
| 5 | Beginning | 2:18 |
| | Middle | 2:16 |
| | End | 1:55 |
| | | |
| 25 | Beginning | 2:11 |
| | Middle | 2:22 |
| | End | 1:55 |
| | | |
| 5 | Beginning | 3:33 |
| | Middle | 4:02 |
| | End | 3:56 |
| | | |
| 5 | Beginning | 2:35 |
| | Middle | 2:30 |
| | End | 2:30 |
| | | |
| 25 | Beginning | 1:29 |
| | Middle | 1:36 |

(continued)

| mg active substance per tablet | Disintegration avg. Minutes | |
|---|---|---|
| | End | 1:48 |
| | | |
| | | |
| 5 | Beginning | 3:18 |
| | Middle | 2:57 |
| | End | 3:01 |
| | | |
| 5 | Beginning | 1:35 |
| | Middle | 2:28 |
| | End | 2:13 |
| | | |
| 5 | Beginning | 2:16 |
| | Middle | 2:07 |
| | End | 2:12 |
| | | |
| 25 | Beginning | 2:03 |
| | Middle | 1:57 |
| | End | 2:00 |

2. Dissolution Test

The standard dissolution test is described in USP31-NF26 S2, chapter 711 (dissolution). The paddle method (Apparatus 2) with an agitation speed of 50 rpm was used. The dissolution media is 900 mL 0.05 M Potassium phosphate or Sodium phosphate buffer pH 6.8 at a temperature of 37°C. Samples are taken after up to 45 minutes. The samples are analyzed via HPLC. The active substance in the tablets is the compound (I.9), preferably in the crystalline form (I.9X).
The same method was used for the example of section 2.3. with the exception that the agitation speed was 75 rpm.

2.1. Dissolution of Tablets of Example 10 (section Examples of formulations)

| mg / tablet | average % dissolved at time point (minutes) | |
|---|---|---|
| 0.5 | 10 min. | 74 |
| | 20 min. | 83 |
| | 30 min. | 87 |
| | 45 min. | 91 |
| | | |
| | | |

(continued)

| mg / tablet | average % dissolved at time point (minutes) | |
|---|---|---|
| 5 | 10 min. | 79 |
| | 20 min. | 85 |
| | 30 min. | 88 |
| | 45 min. | 91 |
| | | |
| 25 | 10 min. | 60 |
| | 20 min. | 73 |
| | 30 min. | 81 |
| | 45 min. | 92 |
| | | |
| 100 | 15 min. | 68 |
| | 30 min. | 76 |
| | 45 min. | 79 |

2.2. Dissolution of Tablets of Example 11 (section Examples of formulations)

| mg / tablet | average % dissolved at time point (minutes) | |
|---|---|---|
| 1 | 15 min. | 80 |
| | 30 min. | 91 |
| | 45 min. | 96 |
| | | |
| 5 | 15 min. | 92 |
| | 30 min. | 102 |
| | 45 min. | 102 |
| | | |
| 5 | 15 min. | 92 |
| | 30 min. | 102 |
| | 45 min. | 106 |
| | | |
| 25 | 15 min. | 66 |
| | 30 min. | 83 |
| | 45 min. | 91 |
| | | |
| 5 | 15 min. | 90 |
| | 30 min. | 100 |
| | 45 min. | 102 |

(continued)

| mg / tablet | average % dissolved at time point (minutes) | |
|---|---|---|
| | | |
| 5 | 15 min. | 91 |
| | 30 min. | 101 |
| | 45 min. | 103 |
| | | |
| 25 | 15 min. | 78 |
| | 30 min. | 92 |
| | 45 min. | 96 |
| | | |
| 5 | 15 min. | 97 |
| | 30 min. | 103 |
| | 45 min. | 102 |
| | | |
| 5 | 15 min. | 91 |
| | 30 min. | 99 |
| | 45 min. | 99 |
| | | |
| 5 | 15 min. | 100 |
| | 30 min. | 101 |
| | 45 min. | 102 |
| | | |
| 25 | 15 min. | 86 |
| | 30 min. | 94 |
| | 45 min. | 97 |

2.3. Dissolution of Tablets of Example 5 (section Examples of formulations)

| mg / tablet | average % dissolved at time point (minutes) | |
|---|---|---|
| 2.5mg | 15min | 100 |
| | 30min | 100 |
| | 45min | 101 |
| | | |
| 5mg | 15min | 98 |
| | 30min | 99 |
| | 45min | 99 |
| | | |

(continued)

| mg / tablet | average % dissolved at time point (minutes) | |
|---|---|---|
| 10mg | 15min | 98 |
| | 30min | 99 |
| | 45min | 99 |
| | | |
| 25mg | 15min | 100 |
| | 30min | 101 |
| | 45min | 101 |
| | | |
| 50mg | 15min | 99 |
| | 30min | 101 |
| | 45min | 101 |

3. Particle Size Distribution Measurement by Laser Diffraction

Particle size distribution measurement is performed for example via light scattering or laser diffraction technique. To determine the particle size the powder is fed into a laser diffraction spectrometer for example by means of a dispersing unit. The test method is described below in detail:

| | |
|---|---|
| Equipment: | Laser Diffraction Spectrometer Sympatec HELOS Particle Sizer. |
| Lens: | R31 (0.5/0.9μm - 175μm) |
| Sample Dispersing Unit: | Dry disperser RODOS/M |

| | |
|---|---|
| Vacuum: | Nilfisk |
| Feeder: | ASPIROS |
| Feed Velocity: | 60.00 mm/s |
| Primary pressure: | 2.00 bar |
| Injector depression: | maximize (mbar)2 |
| Reference Measurement: | 10 seconds |
| Cycle Time: | 100 msec |
| Trigger Conditions: | Start 0.0 seconds after optical concentration ≥ 1% valid always |

Stop after 5.0 seconds optical concentration ≤ 1% or after 30 seconds real time

| | |
|---|---|
| Optical Concentration: | Approximately range 3 - 12 % |
| Evaluation: | HRLD |
| Sample Size: | Approximately 100 mg |
| Number of measurements: | 2 (duplicate) |

The instrument is set up according to the manufacturer's recommendation and using the manufacturer provided software. The sample container is thoroughly mixed and tumbled prior to removing a portion of the sample to ensure that a representative sample is tested. Duplicate samples are prepared by using a spatula to transfer approximately 100 mg of a sample into the ASPIROS glass vials and cap the vials. The capped cials are placed into the feeder.

4. Tablet hardness and friability

Tablet hardness and friability test is performed as described in USP31-NF26 S2, chapter 1217 (tablet breaking force).

5. Pharmacokinetic parameters

The pharmacokinetic parameters of pharmaceutical compositions and pharmaceutical dosage forms are assessed in healthy volunteer and patient populations. In the studies shown below, the participants fasted on the day of sampling, unless as otherwise stated (see for example study .3). The active substance in the studies below is the compound (I.9), preferably in the crystalline form (I.9X), and the dose of active ingredient is indicated in mg. For the quantification of plasma concentrations of the active ingredient, 2.7 mL of blood was collected and transferred into an EDTA (ethylendiaminetetraacetic acid)-anticoagulant blood drawing tube. The EDTA-anticoagulated blood samples were centrifuged immediately after collection. Centrifugation lasted for about 10 minutes (at about 2,000 x gf to 4,000 x gf) at 4-8°C or within 30 minutes while stored on ice. Concentrations of active substance in EDTA human plasma samples were quantitated using a HPLC/MS/MS method. The assay method comprised a solid phase supported liquid-liquid extraction of human plasma coupled with HPLC/MS/MS determination of the extracted samples. The HPLC/MS/MS assay was validated for the range of 1.11 to 1110 nM in human plasma.

Study .1: Single dose study in a healthy volunteer population of N = 72. Healthy volunteers were administered tablets as described in Example 10 (section Examples of formulations).
Study .2: Multiple rising dose trial in a diabetic patient population of N = 48, once daily administration for 8 days. Patients were administered tablets as described in Example 10 (section Examples of formulations).
Study .3: Single dose, cross-over, food effect study in a healthy volunteer population of N = 14. Healthy volunteers were administered tablets as described in Example 10 (section Examples of formulations).
Study .4: 4-week treatment of a diabetic patient population of N = 78, once daily administration for 4 weeks. Patients were administered tablets as described in Example 10 (section Examples of formulations).
Study .5: Single dose study in a healthy volunteer population of N = 48 (Japanese volunteers). Healthy volunteers were administered tablets as described in Example 11 (section Examples of formulations).
$AUC_{0\text{-}inf}$ : area under the concentration-time curve of the analyte in plasma over the time interval from 0 extrapolated to infinity.
Cmax: maximum concentration of the analyte in plasma.
Tmax: time from dosing to maximum concentration.
$AUC_{\tau,ss}$ : area under the concentration-time curve of the analyte in plasma over the time interval from 0 to 24 h at steady-state.
$C_{max,ss}$ : maximum concentration of the analyte in plasma at steady state over a uniform dosing interval.
$t_{max,ss}$ : time from dosing to maximum concentration at steady state.

5.1. Pharmacokinetic parameters, single dose

Table: Pharmacokinetic parameters: area under the plasma concentration-time curve from 0 hours to infinity ($AUC_{0\text{-}inf}$)

| Dose (mg) | Study | $AUC_{0\text{-}inf}$ (nmol*h/L) | | | | | | | |
|---|---|---|---|---|---|---|---|---|---|
| | | Mean | SD | %CV | gMean | %gCV | Min | Median | Max |
| 2.5 | .1 | 396 | 43.4 | 11.0 | 394 | 11.2 | 336 | 398 | 448 |
| | .2 | 476 | 89.4 | 18.8 | 468 | 19.6 | 326 | 501 | 631 |
| 5 | .5 | 1140 | 117 | 10.2 | 1140 | 10.2 | 1000 | 1150 | 1310 |
| 10 | .1 | 1730 | 377 | 21.8 | 1690 | 23.5 | 1170 | 1780 | 2180 |
| | .5 | 2670 | 284 | 10.6 | 2660 | 10.3 | 2340 | 2640 | 3190 |
| | .2 | 1910 | 290 | 15.1 | 1890 | 14.7 | 1600 | 1830 | 2400 |
| | .4 | 1740 | 284 | 16.4 | 1720 | 15.9 | 1390 | 1730 | 2410 |

(continued)

| Dose (mg) | Study | $AUC_{0-inf}$ (nmol*h/L) | | | | | | | |
|---|---|---|---|---|---|---|---|---|---|
| | | Mean | SD | %CV | gMean | %gCV | Min | Median | Max |
| 25 | .1 | 3830 | 825 | 21.5 | 3750 | 23.0 | 2660 | 3980 | 4910 |
| | .5 | 6180 | 825 | 13.4 | 6130 | 13.8 | 5040 | 6340 | 7150 |
| | .2 | 4900 | 1190 | 24.3 | 4780 | 23.7 | 3690 | 4640 | 6920 |
| | .4 | 4340 | 1000 | 23.1 | 4240 | 22.0 | 2840 | 4270 | 7170 |
| 50 | .1 | 8580 | 1680 | 19.6 | 8460 | 18.2 | 7270 | 8290 | 11500 |
| | .3/fasted | 8510 | 2060 | 24.2 | 8310 | 22.1 | 6450 | 7930 | 14100 |
| | .3/fed | 7590 | 1450 | 19.1 | 7460 | 19.3 | 5060 | 7490 | 10800 |

Table: Pharmacokinetic parameters: maximum plasma concentration ($C_{max}$)

| Dose (mg) | Study | $C_{max}$ (nmol/L) | | | | | | | |
|---|---|---|---|---|---|---|---|---|---|
| | | Mean | SD | %CV | gMean | %gCV | Min | Median | Max |
| 2.5 | .1 | 53.2 | 6.23 | 11.7 | 52.9 | 12.3 | 42.8 | 55 | 60.8 |
| | .2 | 62.4 | 12.3 | 19.8 | 61.3 | 20.5 | 43.3 | 62.6 | 81.2 |
| 5 | .5 | 166 | 44.2 | 26.6 | 161 | 26.4 | 123 | 153 | 230 |
| 10 | .1 | 226 | 46.0 | 20.4 | 221 | 23.6 | 143 | 239 | 268 |
| | .5 | 379 | 73.5 | 19.4 | 372 | 22.6 | 242 | 398 | 454 |
| | .2 | 245 | 51.5 | 21.0 | 240 | 21.2 | 163 | 233 | 344 |
| | .4 | 309 | 140 | 45.2 | 291 | 33.4 | 205 | 271 | 796 |
| 25 | .1 | 505 | 130 | 25.9 | 490 | 27.7 | 334 | 520 | 678 |
| | .5 | 661 | 68.8 | 10.4 | 658 | 9.93 | 605 | 643 | 790 |
| | .2 | 606 | 147 | 24.2 | 592 | 23.7 | 420 | 569 | 905 |
| | .4 | 722 | 144 | 20.0 | 709 | 19.9 | 496 | 697 | 1030 |
| 50 | .1 | 1110 | 274 | 24.6 | 1080 | 26.9 | 722 | 1100 | 1450 |
| | .3/fasted | 1180 | 340 | 28.9 | 1140 | 26.1 | 878 | 1010 | 2020 |
| | .3/fed | 824 | 167 | 20.3 | 806 | 23.3 | 436 | 830 | 1070 |

Table: Pharmacokinetic parameters: time to reach maximum plasma concentration ($t_{max}$)

| Dose (mg) | Study | $t_{max}$ (h) | | | | | | | |
|---|---|---|---|---|---|---|---|---|---|
| | | Mean | SD | %CV | gMean | %gCV | Min | Median | Max |
| 2.5 | .1 | 1.83 | 0.684 | 37.4 | 1.72 | 38.9 | 0.983 | 1.75 | 2.98 |
| | .2 | 1.41 | 0.278 | 19.7 | 1.37 | 27.5 | 0.667 | 1.50 | 1.50 |
| 5 | .5 | 1.63 | 0.586 | 36.1 | 1.51 | 46.4 | 0.750 | 2.00 | 2.00 |
| 10 | .1 | 1.42 | 0.387 | 27.2 | 1.38 | 28.1 | 0.983 | 1.50 | 2.03 |
| | .5 | 1.67 | 0.753 | 45.2 | 1.54 | 44.1 | 1.00 | 1.50 | 3.00 |
| | .2 | 1.50 | 0.254 | 17.0 | 1.48 | 18.2 | 0.983 | 1.50 | 2.00 |
| | .4 | 1.50 | 0.447 | 29.8 | 1.44 | 30.2 | 1.00 | 1.50 | 2.50 |
| 25 | .1 | 2.19 | 0.747 | 34.1 | 2.06 | 41.8 | 1.00 | 2.05 | 3.02 |
| | .5 | 2.33 | 1.03 | 44.3 | 2.14 | 49.6 | 1.00 | 2.00 | 4.00 |
| | .2 | 1.72 | 0.872 | 50.7 | 1.60 | 38.5 | 0.983 | 1.50 | 4.00 |
| | .4 | 1.39 | 0.399 | 28.7 | 1.33 | 30.6 | 0.750 | 1.50 | 2.00 |

(continued)

| Dose (mg) | Study | $t_{max}$ (h) | | | | | | | |
|---|---|---|---|---|---|---|---|---|---|
| | | Mean | SD | %CV | gMean | %gCV | Min | Median | Max |
| 50 | .1 | 1.75 | 0.832 | 47.5 | 1.59 | 54.3 | 0.750 | 1.50 | 3.00 |
| | .3/fasted | 1.53 | 1.00 | 65.8 | 1.29 | 62.2 | 0.750 | 1.02 | 4.07 |
| | .3/fed | 2.46 | 1.18 | 48.0 | 2.18 | 57.4 | 1.00 | 2.48 | 4.00 |

5.2. Pharmacokinetic parameters, steady state

Table: Pharmacokinetic parameters: area under the plasma concentration-time curve over a dosing interval at steady-state ($AUC_{\tau,ss}$)

| Dose (mg) | Study | $AUC_{\tau,ss}$ (nmol*h/L) | | | | | | | |
|---|---|---|---|---|---|---|---|---|---|
| | | Mean | SD | %CV | gMean | %gCV | Min | Median | Max |
| 2.5 | .2 | 471 | 108 | 23 | 460 | 24.3 | 283 | 458 | 677 |
| 10 | .2 | 2030 | 362 | 17.8 | 2000 | 17.4 | 1640 | 1940 | 2580 |
| | .4 | 1870 | 298 | 15.9 | 1850 | 15.8 | 1350 | 1840 | 2600 |
| 25 | .2 | 4990 | 1080 | 21.5 | 4890 | 21.5 | 3440 | 4560 | 6650 |
| | .4 | 4740 | 1000 | 21.2 | 4640 | 20.8 | 2790 | 4480 | 7640 |

Table: Pharmacokinetic parameters: maximum plasma concentration at steasy-state ($C_{max,ss}$)

| Dose (mg) | Study | $C_{max,ss}$ (nmol/L) | | | | | | | |
|---|---|---|---|---|---|---|---|---|---|
| | | Mean | SD | %CV | gMean | %gCV | Min | Median | Max |
| 2.5 | .2 | 68.5 | 16.8 | 24.5 | 66.6 | 26.7 | 40.3 | 72 | 96.3 |
| 10 | .2 | 283 | 90.1 | 31.9 | 272 | 30.1 | 172 | 279 | 479 |
| | .4 | 259 | 64.3 | 24.8 | 252 | 25.7 | 166 | 251 | 367 |
| 25 | .2 | 630 | 106 | 16.8 | 622 | 17.4 | 443 | 603 | 793 |
| | .4 | 687 | 126 | 18.4 | 676 | 18.7 | 481 | 671 | 907 |

Table: Pharmacokinetic parameters: time to reach maximum plasma concentration at steady-state ($t_{max,ss}$)

| Dose (mg) | Study | $t_{max,ss}$ (h) | | | | | | | |
|---|---|---|---|---|---|---|---|---|---|
| | | Mean | SD | %CV | gMean | %gCV | Min | Median | Max |
| 2.5 | .2 | 1.33 | 0.362 | 27.3 | 1.28 | 27.4 | 0.983 | 1.50 | 2.00 |
| 10 | .2 | 1.43 | 0.327 | 22.8 | 1.40 | 24.3 | 0.983 | 1.50 | 2.00 |
| | .4 | 1.72 | 0.731 | 42.5 | 1.61 | 36.7 | 0.983 | 1.50 | 4.00 |
| 25 | .2 | 2.26 | 1.21 | 53.5 | 1.97 | 62.6 | 0.667 | 2.00 | 4.20 |
| | .4 | 1.55 | 0.771 | 49.9 | 1.40 | 46.3 | 0.750 | 1.50 | 3.02 |

5.3. Pharmacokinetic parameters, single dose, dose-normalized

Table: Pharmacokinetic parameters: dose-normalized area under the plasma concentration-time curve from 0 hours to infinity ($AUC_{0\text{-inf, norm}}$) and dose-normalized maximum plasma concentration ($C_{max,\ norm}$)

| Dose (mg) | Study | $AUC_{0\text{-inf,norm}}$ (nmol*h/L/mg) | | | | $C_{max,norm}$ (nmol/L/mg) | | | |
|---|---|---|---|---|---|---|---|---|---|
| | | gMean | Min | Median | Max | gMean | Min | Median | Max |
| 2.5 | .1 | 158 | 134 | 159 | 179 | 21 | 17 | 22 | 24 |
| | .2 | 187 | 130 | 200 | 252 | 25 | 17 | 25 | 32 |
| 5 | .5 | 228 | 200 | 230 | 262 | 32 | 25 | 31 | 46 |
| 10 | .1 | 169 | 117 | 178 | 218 | 22 | 14 | 24 | 27 |
| | .5 | 266 | 234 | 264 | 319 | 37 | 24 | 40 | 45 |
| | .2 | 189 | 160 | 183 | 240 | 24 | 16 | 23 | 34 |
| | .4 | 172 | 139 | 173 | 241 | 29 | 21 | 27 | 80 |
| 25 | .1 | 150 | 106 | 159 | 196 | 20 | 13 | 21 | 27 |
| | .5 | 245 | 202 | 254 | 286 | 26 | 24 | 26 | 32 |
| | .2 | 191 | 148 | 186 | 277 | 24 | 17 | 23 | 36 |
| | .4 | 170 | 114 | 171 | 287 | 28 | 20 | 28 | 41 |
| 50 | .1 | 169 | 145 | 166 | 230 | 22 | 14 | 22 | 29 |
| | .3/fasted | 166 | 129 | 159 | 282 | 23 | 18 | 20 | 40 |
| | .3/fed | 149 | 101 | 150 | 216 | 16 | 9 | 17 | 21 |

5.4. Pharmacokinetic parameters, steady state

Table: Pharmacokinetic parameters: dose-normalized area under the plasma concentration-time curve over a dosing interval at steady-state ($AUC_{\tau,ss,\ norm}$) and dose-normalized maximum plasma concentrations at steady-state ($C_{max,ss,\ norm}$)

| Dose (mg) | Study | $AUC_{\tau,ss,norm}$ (nmol*h/L/mg) | | | | $C_{max,ss,norm}$ (nmol/L/mg) | | | |
|---|---|---|---|---|---|---|---|---|---|
| | | gMean | Min | Median | Max | gMean | Min | Median | Max |
| 2.5 | .2 | 184 | 113 | 183 | 271 | 27 | 16 | 29 | 39 |
| 10 | .2 | 200 | 164 | 194 | 258 | 27 | 17 | 28 | 48 |
| | .4 | 185 | 135 | 184 | 260 | 25 | 17 | 25 | 37 |
| 25 | .2 | 196 | 138 | 182 | 266 | 25 | 18 | 24 | 32 |
| | .4 | 186 | 112 | 179 | 306 | 27 | 19 | 27 | 36 |

## Claims

**1.** A pharmaceutical composition comprising a compound of the formula (I.9),

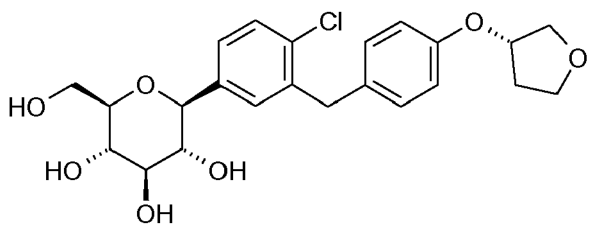

(I.9)

which when administered to a fasting human:

a) at a dose of 2.5 mg exhibits:

i. a $C_{max}$ of 40.3 to 96.3 nmol/L; and
ii. a AUC of 283 to 677 nmol*h/L; or

b) at a dose of 5.0 mg exhibits:

i. a $C_{max}$ of 123 to 230 nmol/L; and
ii. a AUC of 1,000 to 1,310 nmol*h/L; or

c) at a dose of 10.0 mg exhibits:

i. a $C_{max}$ of 143 to 796 nmol/L; and
ii. a AUC of 1,170 to 3,190 nmol*h/L; or

d) at a dose of 25.0 mg exhibits:

i. a $C_{max}$ of 334 to 1,030 nmol/L; and
ii. a AUC of 2,660 to 7,640 nmol*h/L; or

e) at a dose of 50.0 mg exhibits:

i. a $C_{max}$ of 722 to 2,020 nmol/L; and
ii. a AUCof 6,450 to 14,100 nmol*h/L.

**2.** A pharmaceutical composition comprising a compound of the formula (I.9),

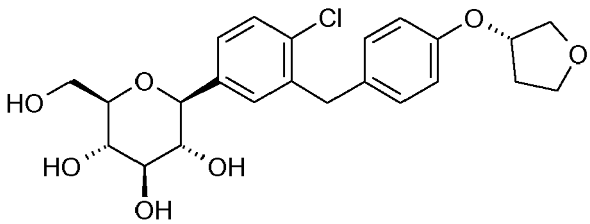

(I.9)

which when administered to a fasting human exhibits:

i. a dose-normalized $C_{max, norm}$ of 13 to 80 nmol/L/mg; and
ii. a dose-normalized $AUC_{0-inf, norm}$ of 106 to 306 nmol*h/L/mg.

**3.** The pharmaceutical composition according to claim 1 or 2, wherein the particle size distribution in said composition is X90 < 200 μm.

**4.** The pharmaceutical composition according to any one of claims 1 to 3, wherein said compound of the formula (I.9) represents 25% or less of the weight of said composition.

**5.** A pharmaceutical composition comprising a compound of the formula (I.9),

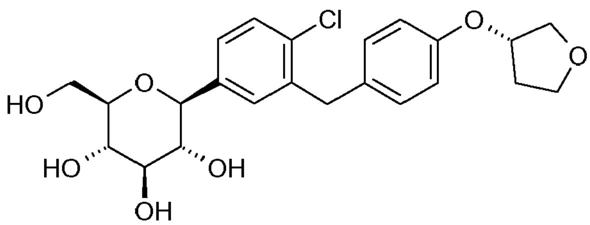

(I.9)

wherein the particle size distribution in said composition is X90 < 200 μm, wherein said compound of the formula (I.9) represents 25% or less of the weight of said composition.

**6.** The pharmaceutical composition according to any one of claims 1 to 5, wherein said composition comprises crystalline form (L9X) of said compound of the formula (I.9).

**7.** The pharmaceutical composition according to any one of claims 1 to 6, wherein said composition comprises a disintegrant and a binder, wherein the ratio of said disintegrant to said binder is between 1.5 : 3.5 and 1 : 1 (weight/weight).

**8.** The pharmaceutical composition according to any one of claims 1 to 7, wherein at least 99% of the particles of said binder (by weight) are 250 μm or smaller.

**9.** The pharmaceutical composition according to any one of claims 1 to 8, wherein said composition is obtained by high shear wet granulation, wherein said composition further comprising a diluent, wherein 5 - 20% (by weight) of said diluent is added to said composition as a dry add after said wet granulation.

**10.** The pharmaceutical composition according to any one of claims 1 to 9, wherein said composition comprises:

| | Amount (% by weight) |
|---|---|
| the compound of the formula (I.9) | 0.5 - 25 |
| one or more diluents | 65 - 93 |
| one or more binders | 1 - 5 |
| one or more disintegrants | 1 - 4 |
| optionally one or more additional additives | ad 100 % |

**11.** The composition according to any one of claims 1 to 10, further comprising one or more lubricants.

**12.** The pharmaceutical composition according to any one of claims 1 to 11, further comprising one or more glidants.

**13.** The pharmaceutical composition according to any one of claims 1 to 12, further comprising one or more film coats.

**14.** A pharmaceutical dosage form comprising a pharmaceutical composition according to any one of claims 1 to 13.

**15.** The pharmaceutical dosage form according to claim 14, wherein said dosage form is a tablet.

**16.** A method of treating a metabolic disorder, in particular for improving glycemic control in a patient, comprising administering to a patient a composition according to any one of claims 1 to 13, or a pharmaceutical dosage form according to claim 14 or 15.

**17.** The method according to claim 16, wherein said metabolic disorder is selected from the group consisting of type 1 diabetes mellitus, type 2 diabetes mellitus, impaired glucose tolerance (IGT), impaired fasting blood glucose (IFG), hyperglycemia, postprandial hyperglycemia, overweight, obesity and metabolic syndrome.

**18.** A wet granulation process for making a pharmaceutical dosage form comprising a compound of the formula (I.9) and one or more excipients,

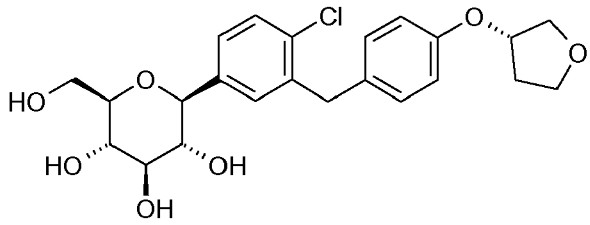

(I.9)

wherein said process comprises the steps of:

(1) Premixing said compound of the formula (I.9) and the main portion of the excipients including a binder in a mixer to obtain a pre-mixture;
(2) granulating the pre-mixture of step (1) by adding a granulation liquid, preferably water;
(3) drying the granules of step (2) in a fluidized bed dryer or a drying oven;
(4) optionally dry sieving of the dried granules of step (3);
(5) mixing the dried granules of step (4) with the remaining excipients in a mixer to obtain the final mixture;
(6) tableting the final mixture of step (5) by compressing it on a suitable tablet press to produce tablets cores;
(7) optionally film-coating of the tablet cores of step (6) with a film coat.

**19.** A pharmaceutical composition obtainable by the process of claim 18.

**20.** A direct compression process for making a pharmaceutical composition comprising a compound of the formula (I.9) and one or more excipients,

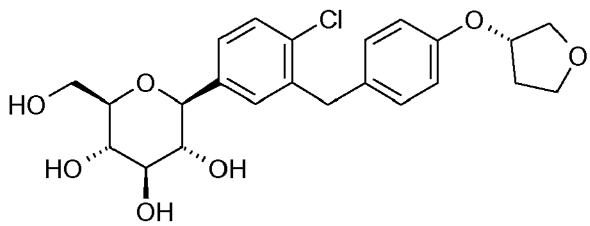

(I.9)

wherein said process comprises the steps of:

(1) Premixing said compound of the formula (I.9) and the main portion of the excipients in a mixer to obtain a pre-mixture;
(2) optionally dry screening the pre-mixture through a screen in order to segregate cohesive particles and to improve content uniformity;
(3) mixing the pre-mixture of step (1) or (2) in a mixer, optionally by adding remaining excipients to the mixture and continuing mixing;
(4) tableting the final mixture of step (3) by compressing it on a suitable tablet press to produce the tablet cores;
(5) optionally film-coating of the tablet cores of step (4) with a film coat.

**21.** A pharmaceutical composition obtainable by the process of claim 20.

**22.** A dry granulation process for making a pharmaceutical composition comprising a compound of the formula (I.9) and one or more excipients,

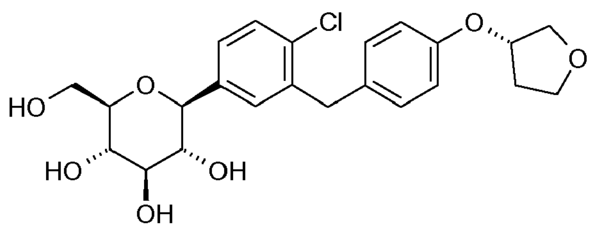

(I.9)

wherein said process comprises the steps of:

(1) mixing said compound of the formula (I.9) with either all or a portion of the excipients in a mixer;
(2) compaction of the mixture of step (1) on a suitable roller compactor;
(3) reducing the ribbons obtained during step (2) to granules by suitable milling or sieving steps;
(4) optionally mixing the granules of step (3) with the remaining excipients in a mixer to obtain the final mixture;
(5) tabletting the granules of step (3) or the final mixture of step (4) by compressing it on a suitable tablet press to produce the tablet cores;
(6) optionally film-coating of the tablet cores of step (5) with a fim coat.

**23.** A pharmaceutical composition obtainable by the process of claim 22.

Figure 1: X-ray powder diffraction pattern of the crystalline form

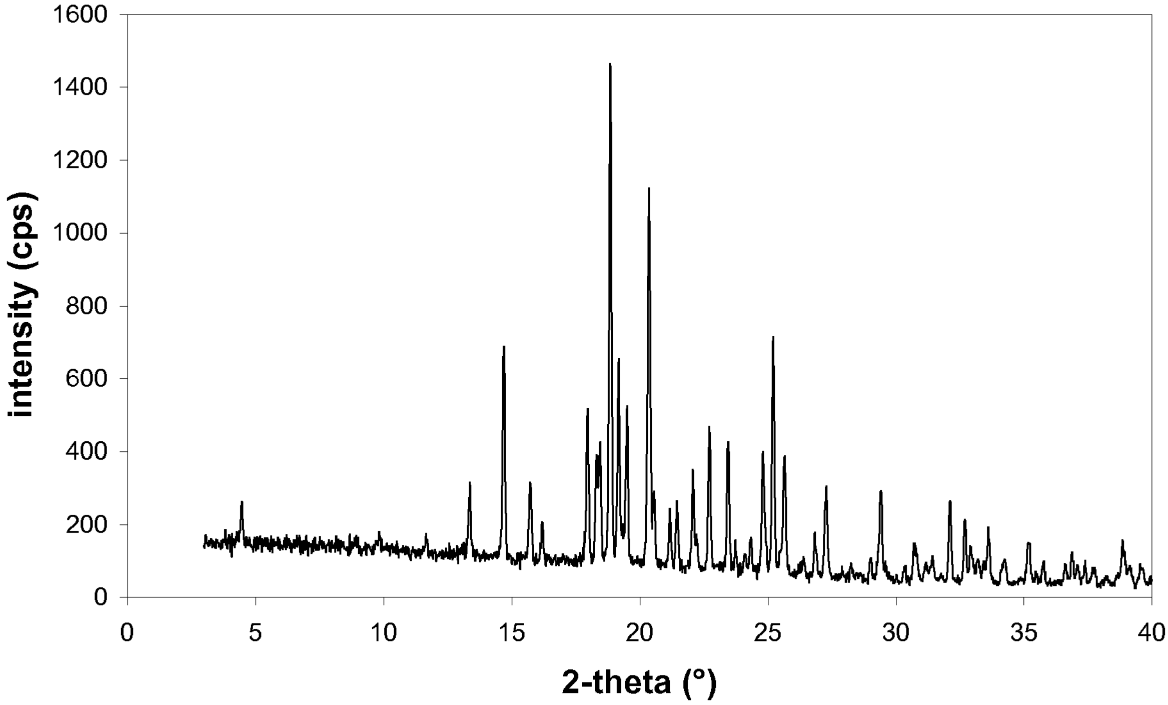


Figure 2: DSC and TG diagram of the crystalline form

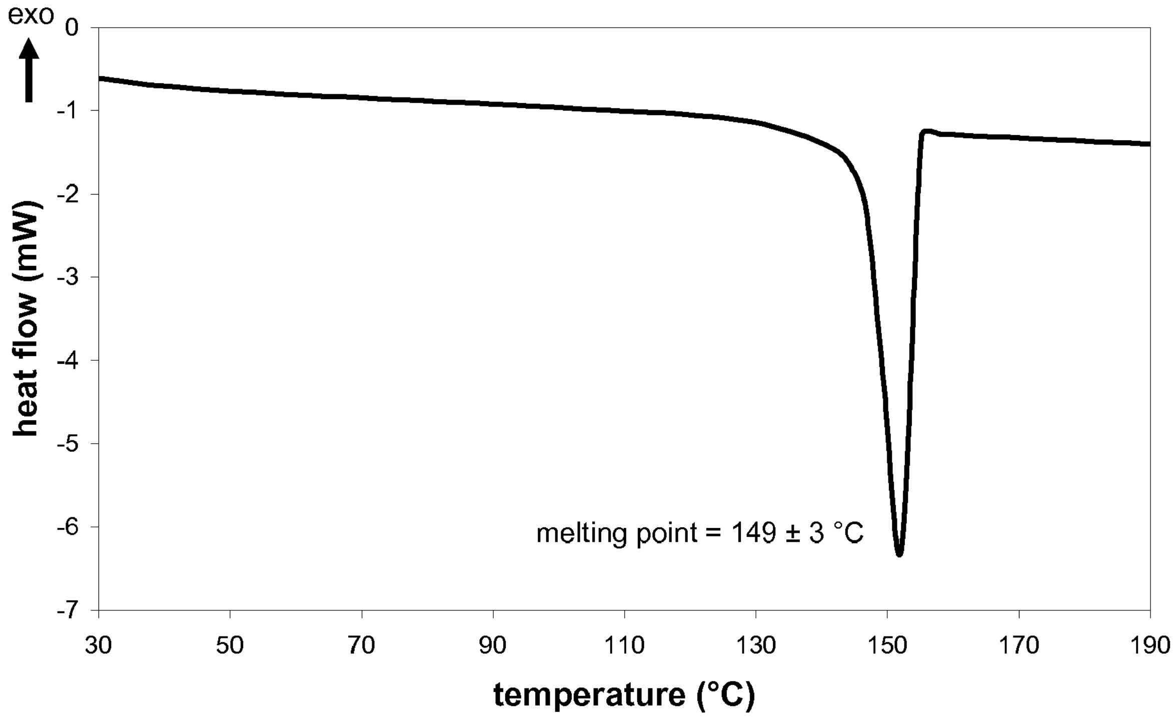

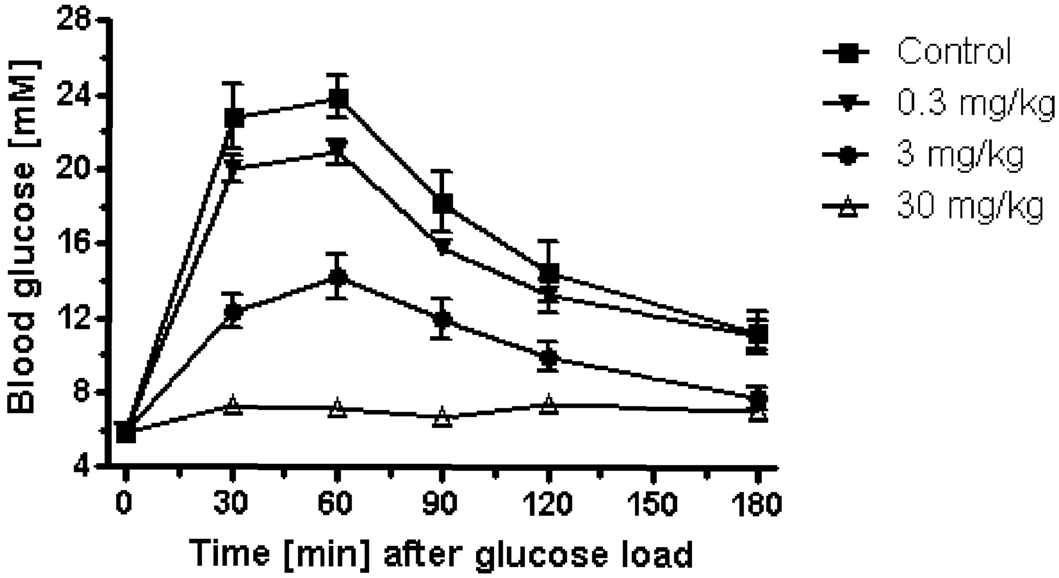
Blood glucose [mM]
28
24
20
16
12
8
4
0
30
60
90
120
150
180
Time [min] after glucose load
Control
0.3 mg/kg
3 mg/kg
30 mg/kg

Fig. 3A

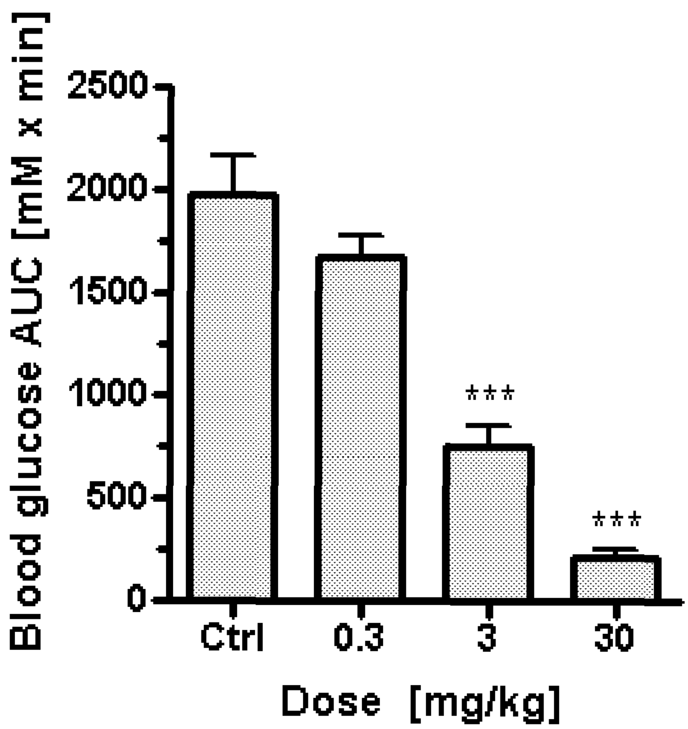
Blood glucose AUC [mM x min]
2500
2000
1500
1000
500
0
***
***
Ctrl
0.3
3
30
Dose [mg/kg]

Fig. 3B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0127128 A
- WO 03099836 A
- WO 2005092877 A
- WO 2006034489 A
- WO 2006064033 A
- WO 2006117359 A
- WO 2006117360 A
- WO 2007025943 A
- WO 2007028814 A
- WO 2007031548 A
- WO 2007093610 A
- WO 2007128749 A
- WO 2008049923 A
- WO 2008055870 A
- WO 2008055940 A
- WO 2006120208 A
- WO 2008020011 A


### Non-patent literature cited in the description


- **FORD ES et al.** *JAMA,* 2002, vol. 287, 356-9
- **KATSUKI A et al.** *Diabetes Care,* 2001, vol. 24, 362-5
- **MATTHEWS et al.** *Diabetologia,* 1985, vol. 28, 412-19
- **FORST et al.** *Diabetes,* 2003, vol. 52 (1), A459
- **GALVIN P et al.** *Diabet Med,* 1992, vol. 9, 921-8
- **STUMVOLL et al.** *Eur J Clin Invest,* 2001, vol. 31, 380-81
- **J. B. MEIGS et al.** *Diabetes,* 2003, vol. 52, 1475-1484
- The Prevention or Delay of Type 2 Diabetes. American Diabetes Association and the National Institute of Diabetes and Digestive and Kidney Diseases, 2002, vol. 25, 742-749
- **LAAKSONEN DE et al.** *Am J Epidemiol,* 2002, vol. 156, 1070-7
- Executive Summary of the Third Report of the National Cholesterol Education Program (NCEP) Expert Panel on Detection, Evaluation, and Treatment of High Blood Cholesterol in Adults (Adult Treatment Panel III). *JAMA: Journal of the American Medical Association,* 2001, vol. 285, 2486-2497
- **LAAKSONEN DE et al.** *Am J Epidemiol.,* 2002, vol. 156, 1070-7
- Labor und Diagnose. TH-Books Verlagsgesellschaft mbH, 2000
- USP 31-NF 26 through Second Supplement. *United States Pharmacopeial Convention*
- European Pharmacopoeia 6.3. European Directorate for the Quality of Medicines and Health Care, 2000